# EUROPEAN PATENT SPECIFICATION

(11) **EP 4 413 140 B1**
(45) Date of publication and mention of the grant of the patent: **13.08.2025**
(21) Application number: 22793205.0
(22) Date of filing: 07.10.2022
(51) Int. Cl.: C12N 15/62, C12N 15/11, C12N 9/22, C12N 15/10

(54) **SYNTHETIC GENOME EDITING SYSTEM**
SYNTHETISCHES GENOMEDITIERSYSTEM
SYSTÈME D'ÉDITION DE GÉNOME SYNTHÉTIQUE

(30) Priority: 08.10.2021 GB 202114453; 01.07.2022 GB 202209735
(43) Date of publication of application: 14.08.2024
(73) Proprietor: Pencil Biosciences Limited, Macclesfield, Cheshire SK10 4TG (GB)
(72) Inventor: IBRAHIM, Mohamed Rajik Mohamed, Macclesfield Cheshire SK10 4TG (GB); KANWAR, Nisha, Macclesfield Cheshire SK10 4TG (GB); RAITSKIN, Oleg, Macclesfield Cheshire SK10 4TG (GB)
(74) Representative: Titmus, Craig Edward
(86) International application number: PCT/GB2022/052555
(87) International publication number: WO 2023/057777

(56) References cited:
- WO-A1-2020/142676
- WO-A1-2021/084533
- MEHMET FATIH BOLUKBASI ET AL: "DNA-binding-domain fusions enhance the targeting range and precision of Cas9", NATURE METHODS (AUTHOR MANUSCRIPT), vol. 12, no. 12, 19 October 2015 (2015-10-19), New York, pages 1150 - 1156, XP055548728, ISSN: 1548-7091, DOI: 10.1038/nmeth.3624
- BOLUKBASI MEHMET FATIH ET AL: "DNA-binding-domain fusions enhance the targeting range and precision of Cas9", NATURE METHODS, vol. 12, no. 12, 19 October 2015 (2015-10-19), New York, pages 1150 - 1156, XP093012024, ISSN: 1548-7091, Retrieved from the Internet <URL:http://www.nature.com/articles/nmeth.3624> DOI: 10.1038/nmeth.3624
- RAUCH SIMONE ET AL: "Programmable RNA-Guided RNA Effector Proteins Built from Human Parts", CELL, vol. 178, no. 1, 27 June 2019 (2019-06-27), pages 122, XP085721270, ISSN: 0092-8674, DOI: 10.1016/J.CELL.2019.05.049
- COFSKY JOSHUA C ET AL: "CRISPR-Cas12a exploits R-loop asymmetry to form double-strand breaks", ELIFE, vol. 9, 10 June 2020 (2020-06-10), XP093011978, Retrieved from the Internet <URL:https://cdn.elifesciences.org/articles/55143/elife-55143-v2.xml> DOI: 10.7554/eLife.55143
- WÖLCKE JULIAN ET AL: "A DNA-BINDING PEPTIDE FROM A PHAGE DISPLAY LIBRARY", NUCLEOSIDES, NUCLEOTIDES & NUCLEIC ACIDS, vol. 20, no. 4-7, 31 March 2001 (2001-03-31), US, pages 1239 - 1241, XP093012180, ISSN: 1525-7770, DOI: 10.1081/NCN-100002526

## Description

### Field of the invention

The present invention provides a synthetic, modular system for DNA modification as may be employed for genome editing comprising a targeting nucleic acid possessing both DNA targeting ability and ability to bind a recognition module of a modular polypeptide where the modular polypeptide also includes as a separate module an effector component. The effector component may be a protein component for modifying structure or regulation of a gene, either alone or for example after dimerization. Desirably it may be an artificial nickase comprising a multimer of linked, self-assembling short peptides as also now taught herein. By including in the modular polypeptide component additionally a short peptide DNA-binding component, devoid of enzyme activity and which recognises a pre-determined sequence in the target, a totally synthetic gene modifying tool has been attained which is far smaller than a CRISPR-Cas9 single guide RNA (sgRNA) gene modifying system thereby easing delivery to cells. The polypeptide component may be delivered with the targeting nucleic acid(s) in a single AAV vector.

### Background of the invention

RNA-guided CRISPR-Cas9 systems and other RNA-guided CRISPR-Cas systems have revolutionised the field of gene editing since the initial studies showing the ability of a sgRNA to direct CRISPR-Cas9 double-strand DNA breaks upon protospacer adjacent motif (PAM) recognition in the target DNA. However, several limitations remain to be addressed in relation to use of such RNA-guided DNA targeting systems for all desired purposes, especially for therapeutic genome modification. One such recognised disadvantage is size which restricts efficient AAV packaging. The *Streptococcus pyogenes* Cas9 (SpCas9) with sgRNA (the most widely used Cas9 version) is 4.1 kbp (1368 aa) in size and more recently identified smaller cas enzymes are still nucleases of substantial size and less than optimal for desired vector delivery. The overall size of AAV only allows up to 5kb of heterologous sequence to be packaged.

In addition, immunogenicity is a consideration for *in vivo* therapeutic application in humans. *Streptococcus pyogenes* Cas9 reactive T cells have been reported in the adult human population.

Published International Application WO2013/088446 of Targetgene Biotechnologies Ltd. proposes an alternative DNA editing system employing a Fok1 nuclease domain linked via a linking domain with a specificity conferring nucleic acid (SCNA). The SCNA comprises a nucleotide sequence complementary to a sequence of the target nucleic acid and a recognition component capable of specifically linking the SCNA to the linking domain, e.g. the recognition component of the SCNA may be one of a specific binding partner pair or a naturally-occurring RNA aptamer which recognises its cognate polypeptide binding component. A pairing of such artificial nucleoprotein complexes on the target DNA is required to enable dimerization of Fok1 nuclease domains. Furthermore, the Fok1 nuclease thus provided must be capable of effective interaction with the DNA to make a double stranded DNA break at the target site. However, WO2013/088446 provides only prophetic exemplification for any therapeutic utility and doubts remain over the efficiency of such a system.

WO 2021/084533 mentions protospacer adjacent motif ("PAM")-reduced and PAM-abolished Cas derivatives compositions and uses thereof in genetic modulation. Bolukbasi et al.,

(2015) Nature Methods 17:12, 1150-1156 mentions DNA-binding-domain fusions which enhance the targeting range and precision of Cas9.

Rauch et al., (2019) Cell 174:122-134 and WO 2020/142676 A1 disclose engineered modular RNA-guided RNA effector proteins built from human protein parts that might overcome the size and immunogenicity limits of CRISPR-Cas systems.

### Summary of the invention

The inventors in this instance have set out to provide a totally synthetic modular system for gene modification as an alternative to use of a CRISPR-Cas/sgRNA system which in the nuclease mode provides good efficiency for DNA target double-strand or single-strand cutting at a predetermined target site and can be designed to provide a gene editing system of far smaller size. The small size achievable with use of an artificial nickase as now taught offers the opportunity to choose a wide variety of delivery means including single AAV vector delivery with one, or more than one, targeting RNA.

The invention is defined according to the claims. Thus, the invention provides a nucleoprotein complex for use in modifying a target nucleic acid sequence comprising (A) a targeting nucleic acid and (B) a modular polypeptide component,
wherein said targeting nucleic acid comprises:
   (i) a targeting nucleic acid element (TE) complementary to a region of the target;
   (ii) a recognition element which specifically interacts with a nucleic acid recognition module of said modular polypeptide component and
   (iii) a connecting sequence which links said targeting element and said recognition element and said modular polypeptide component comprises as linked, separate functional modules:
      (a) said nucleic acid recognition module which is devoid of enzymic activity;
      (b) a DNA-binding domain (DBD) for assisting in melting and/or unwinding of a double helix of the target, wherein the DBD recognises a predetermined sequence in the target, module (a) and said DBD not being naturally found in linkage,
      (c) an effector component for use in modifying the target whereby site-specific modification directed by said TE and DBD will occur,
wherein said DBD is a polypeptide sequence of no more than 70-75 amino acid residues;
wherein the length of the modular polypeptide component is no more than 300 amino acids in length;
and wherein module (b) is joined to module (a) and module (c) respectively; optionally wherein modules (a), (b) and (c) are joined by a first and/or a second linker.

In one embodiment, said targeting nucleic acid is an RNA.

In one embodiment, the length of the modular peptide component is no more than 220-250 amino acids in length.

In one embodiment, said recognition element of the targeting nucleic acid is an RNA scaffold (RS) which binds an RNA scaffold binding domain (RSBD) providing said module (a) of the modular polypeptide component, for example said RS is the 19nt Box B RNA motif and said RSBD is the Lambda N22 peptide as set forth in SEQ. ID. No 2.

In one embodiment, the nucleoprotein complex comprises
as said component (A) a wholly nucleic acid component (NAC) and (B) a modular polypeptide component, wherein the NAC comprises:
(i) a targeting element (TE) complementary to a region of the target;
(ii) an RNA scaffold (RS) which specifically binds to an RNA scaffold binding domain (RSBD) of said modular polypeptide component and
(iii) a connecting sequence which links said TE and the RS
and said modular polypeptide component comprises as linked, separate functional modules:
(a) said RSBD;
(b) said DNA-binding domain linked to (a) which recognises a pre-determined sequence in the target, module (a) and said DBD not being naturally found in linkage;
(c) an effector component linked to (b) for use in modifying the target, whereby site specific modification directed by said TE and said DBD will occur and
(d) optionally, or if required, a first linker and/or a second linker linking said DBD to said effector component and the RSBD respectively .

In one embodiment, said targeting nucleic acid or said NAC provides two or more RNA scaffolds, either the same or different.

In one embodiment:
said DBD is no more than a 30 mer, no more than a 25 mer, no more than a 20mer, preferably no more than a 15mer; optionally wherein said DNA-binding domain binds a predetermined sequence of 3-6 nucleotides; optionally wherein said DBD is a peptide of no more than 15 amino acids, e.g. the 15 mer of sequence SLETWLKHREKDGGS (SEQ. ID. No. 4) which binds the dsDNA sequence represented by the 5' to 3' sequence 5'GAGGTC3' . In one embodiment, said DBD is capable of binding to more than one predetermined sequence . In one embodiment, said modular polypeptide component additionally provides a nuclear localization signal (NLS) and /or organelle localization signal, wherein said NLS and /or organelle localization signal is provided at the N- or C-terminus optionally connected to an additional sequence to aid detection.

In one embodiment, said effector component is selected from (i) an endonuclease for producing double-stranded breaks or a Fok 1 nuclease domain (ii) a nickase (iii) a transcription activator (iv) a transcriptional repressor (v) an epigenetic modulator enzyme (vii) a recombinase (viii) a transposase (ix) an integrase and (x) a nucleobase modifying enzyme construct.

In one embodiment, said effector component is an artificial nuclease or nickase comprising a multimer of linked, self-assembling peptides forming a beta sheet structure wherein:
(i) the self-assembling peptides of said multimer each present an alternating pattern of hydrophobic and hydrophilic residues with, all or at least a proportion, of the hydrophilic residues of each such peptide representing a catalytic triad of amino acids whereby said multimer is capable of cleaving a single-strand or both strands of a dsDNA;
(ii) the self-assembling peptides are joined by linkers to limit the multimer number, e.g. to 4-15, preferably 5-10 and
(iii) N- terminal and C-terminal flanking sequences are provided linked to the multimer to prevent or limit aggregation between multimers of the nuclease protein with increased positive charge compared to the peptide units of the multimer due to inclusion of one or more positively charged residues, e.g. by inclusion of one or more lysine or arginine residues .

In one embodiment,
(i) the modular polypeptide component is no more than 220-300 amino acids excluding any cleavable tag or localization signal or any other sequence other than components (a) - (d);
(ii) said peptides forming said multimer are identical 7mers or comprise two or more non-identical 7mers wherein the alternating hydrophobic residues are leucine and/ or isoleucine starting with the N-terminal residue;
(iii) said peptides forming said multimer are identical peptides all continuously linked in the N-terminal to C-terminal direction so as to provide an anti-parallel beta sheet structure;
(iv) the linkers joining the peptides of said multimer are amino acid linkers capable of providing a beta turn or beta turn like loops, e.g. consisting of a combination of amino acids selected from glycine (G), serine (S), asparagine (N), glutamine (Q), threonine (T), glutamic acid (E) and aspartic acid (D); and/or
(v) the C-terminus of said C-terminal flanking peptide provides a flexible tail sequence at its C-terminus to aid solubility.

In one embodiment,
said peptides of said multimer are 7 mers of sequence IEIDIHI;
optionally wherein an artificial nickase is provided comprising the sequence 
IEKDIHINDGGIEIDIHIDSGGIEIDIHISSGSIEIDIHINNGNIEIDIHIGSDGIEIDIHIGNSGIEIDIHI
DNGGIEIDIHIDSDGIEIDIHISSSGIEIDIHIGSEGIEIDIHIGDSGIEIDKHINQGS (SEQ. ID. No.
6)
or variant thereof with one or more variant beta turns and /or variation of the NQGS tail sequence;
optionally wherein the modular polypeptide component comprises the sequence of SEQ. ID. NO. 1 consisting of the following components fused in the N- terminal to C-terminal direction:
   (i) the Lambda N22 protein, MDAQTRRRERRAEKQAQWKAAN (SEQ. ID. No. 2)
   (ii) the linker GGGGSGGGGSGGGGSGGGGSGGGGSGGGGSGGGGS (SEQ. ID. No. 3)
   (iii) the DBD SLETWLKHREKDGGS (SEQ. ID. No. 4) which binds the dsDNA sequence represented by the 5' to 3' sequence 5'GAGGTC3'
   (iv) the linker GGGGSGGGGSGGGGGS (SEQ. ID. No. 5) and
   (v) the nickase module: 
IEKDIHINDGGIEIDIHIDSGGIEIDIHISSGSIEIDIHINNGNIEIDIHIGSDGIEIDIHIGNSGIEIDIHI
DNGGIEIDIHIDSDGIEIDIHISSSGIEIDIHIGSEGIEIDIHIGDSGIEIDKHINQGS (SEQ. ID. No.
6)
or the same modular polypeptide sequence with one or both of said linkers substituted by an alternative linker.

In one embodiment, the modular polypeptide component comprises the following components fused in the N-terminal to C-terminal direction:
(i) the Lambda N22 protein, MDAQTRRRERRAEKQAQWKAAN (SEQ. ID. No. 2)
(ii) the linker GGGGSGGGGSGGGGSGGGGSGGGGSGGGGSGGGGS (SEQ. ID. No. 3)
(iii) the DBD SLETWLKHREKDGGS (SEQ. ID. No. 4) which binds the dsDNA sequence represented by the 5' to 3' sequence 5'GAGGTC3'
(iv) the linker GGGGSGGGGSGGGGGS (SEQ. ID. No. 5) and
(v) an alternative effector component other than said artificial nickase module, e.g. a transcription activator such as VP64
or the same modular polypeptide sequence with one or both of said linkers substituted by an alternative linker.

The invention also provides a nucleic acid or combination of nucleic acids for provision of one or more nucleoprotein complexes of the invention in a host cell;
optionally wherein the nucleic acid is a vector capable of expressing both the polypeptide component of a nucleoprotein complex of the invention providing an artificial nickase and at least one targeting nucleic acid, e.g. targeting RNA.

The invention also provides a method for modifying one or more target nucleic acid sequences employing one or more nucleoprotein complexes of the invention or one or more nucleic acids for provision thereof in a host cell with the proviso that the method is not a method of modifying the germ line identity of a human being or method of treatment practised on the human or animal body.

The invention also provides a combination of (i) at least one modular polypeptide component of a nucleoprotein complex of the invention, or a polynucleotide encoding the same and (ii) one or more targeting nucleic acids which can link with said polypeptide component(s), or
one or more polynucleotides encoding the same, for use in a method of modifying a target nucleic acid, for example for use in such a method for therapeutic treatment.

In one aspect, the present invention provides a nucleoprotein complex for use in modifying a target nucleic acid, e.g. target DNA, comprising (A) a targeting nucleic acid and (B) a modular polypeptide component,
wherein said targeting nucleic acid comprises:
   (i) a targeting nucleic acid element (also referred to herein as a targeting element, TE) complementary to a region of the target;
   (ii) a recognition element (e.g. RNA scaffold, RS) which specifically interacts with a nucleic acid recognition module (e.g. RNA scaffold binding domain, RSBD) of said modular polypeptide component and
   (iii) a connecting sequence which links said targeting element and said recognition element and said modular polypeptide component comprises as linked, separate functional modules:
      (a) said nucleic acid recognition module which is devoid of enzymic activity;
      (b) a DNA-binding domain (DBD) for assisting in melting and/or unwinding of a double helix of the target, wherein the DBD recognises a predetermined sequence in the target, module (a) and said DBD not being naturally found in linkage, and
      (c) an effector component for use in modifying the target, whereby site-specific modification directed by said targeting element of the targeting nucleic acid and said DBD will occur,
wherein said DBD is a polypeptide sequence of no more than 70-75 amino acid residues, e.g. no more than a 30 mer, no more than a 25 mer, no more than a 20mer, preferably no more than a 15mer;

wherein the length of the modular peptide component is no more than 300 amino acids in length;
and wherein module (b) is joined to module (a) and module (c) respectively; optionally wherein modules (a), (b) and (c) are joined by a first and/or a second linker.

Without being bound by theory, the intended purpose of the DBD is to destabilize the structure of a targeted dsDNA upon binding to the predetermined sequence (PDS) in a manner so as to facilitate accessibility of the targeting nucleic acid sequence to the dsDNA structure for hybridization. Such functional ability may be assessed as exemplified in Example 1, e.g. using a dsDNA including the chosen PDS and T7 Endonuclease 1 (T7E1) to evaluate DNA structure. T7E1 is well-known as a structure selective enzyme that will detect structural deformities in dsDNA. Other methods of detecting the desired structural deformation upon binding of the DBD polypeptide to a PDS will be recognised.

The DBD may have binding affinity for a single predetermined sequence but may have ability to bind more than one predetermined sequence. The requirement is that the DBD in concert with the targeting element of the targeting nucleic acid directs site-specific modification. However, the DBD is viewed as more than just a secondary tool for positioning at the required target site; it is viewed as having a structural role in facilitating hybridization of the targeting element and thereby facilitating the overall desired target modification. It will thus be recognised that provision of a short DBD polypeptide sequence in this instance is not comparable with use of for example a zinc finger protein or TALE array to merely position an effector or portion of an effector at a specific DNA sequence.

The modular polypeptide component comprises:
(a) a nucleic acid recognition module (e.g. RSBD);
(b) a DNA-binding domain (DBD) linked directly to (a) which recognises a pre-determined sequence in the target, module (a) and said DBD not being naturally found in linkage;
(c) an effector component linked directly to (b) for use in modifying the target, whereby site specific modification directed by the targeting element of the targeting nucleic acid and said DBD will occur and
(d) optionally, or if required, a first linker and/ or a second linker providing direct linkage of said DBD to said effector component and said nucleic acid recognition module respectively.

The modules of the modular polypeptide component will generally be linearly linked. Thus, modules (a), (b) and (c) may, for example, be linearly joined with (a) at the N-terminal and the effector component at the C-terminal or vice versa. In this way, interaction of the targeting nucleic acid with the recognition module of the modular polypeptide component is distanced from the effector component.

The targeting nucleic acid may be an RNA, preferably wholly an RNA. It will be appreciated that the nucleic acid targeting element is comparable in functional purpose to a CRISPR-Cas9 gRNA. As indicated above, conveniently and preferably, this targeting element may be joined, via a connector, with an RNA motif providing an RNA scaffold (possibly alternatively referred to as an RNA aptamer) which binds a cognate protein or peptide module in the modular polypeptide. The RNA scaffold recognizing module of the modular polypeptide component in this case may be referred to as the RNA scaffold binding domain (RSBD). In this way, the nucleoprotein complex may advantageously comprise (i) a nucleic acid component (NAC) capable of expression as an RNA from an encoding nucleic acid sequence and (ii) a modular polypeptide component, also expressible from a single encoding nucleic acid sequence.

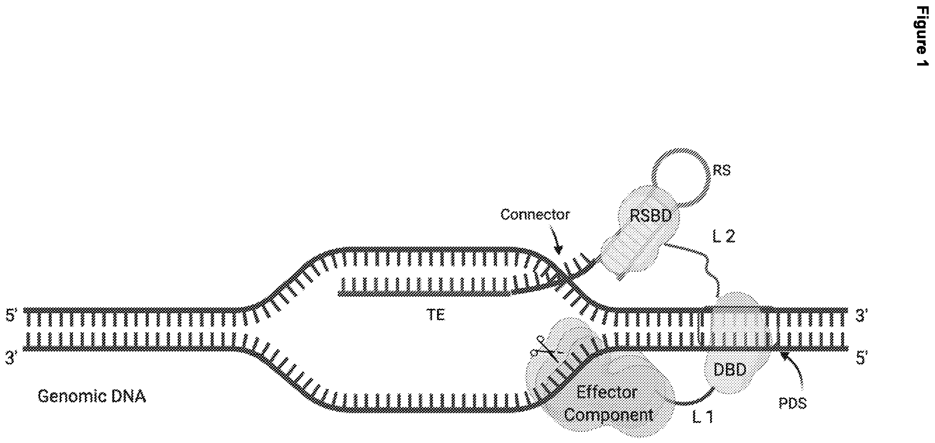

Thus, in a preferred embodiment, a nucleoprotein complex of the invention for use in modifying a target nucleic acid, e.g. target DNA, comprises (A) a wholly nucleic acid component (NAC) and (B) a modular polypeptide component,
wherein the NAC comprises:
(i) a targeting element (TE) complementary to a region of the target;
(ii) an RNA scaffold (RS) which specifically binds to an RNA scaffold binding domain (RSBD) of said modular polypeptide component and
(iii) a connecting sequence which links said TE and the RS
and said modular polypeptide component comprises as linked, separate functional modules:
(a) said RSBD;
(b) a DNA-binding domain (DBD) linked to (a) which recognises a pre-determined sequence in the target, module (a) and said DBD not being naturally found in linkage;
(c) an effector component linked to (b) for use in modifying the target, whereby site specific modification directed by said targeting element of the NAC and said DBD will occur and
(d) optionally, or if required, a first linker and/ or a second linker linking said DBD to said effector component and the RSBD respectively,
wherein said DBD is a polypeptide sequence of no more than 70-75 amino acid residues, e.g. no more than a 30 mer, no more than a 25 mer, no more than a 20mer, preferably no more than a 15mer as discussed above. See Figure 1.

It will be understood that the separate functional modules of the modular polypeptide component can be advantageously independently substituted and encoded by a single nucleic acid such that they are joined in defined linear succession.

The target may be any target nucleic acid, RNA or DNA, including a viral nucleic acid. The target may be a single-stranded DNA or more commonly a double-stranded DNA (dsDNA). A nucleoprotein complex of the invention as indicated above may be particularly favoured as an alternative to a CRISPR-Cas system for delivery to cells to achieve genomic DNA modification, especially in eukaryotic cells including plant cells and human cells.

Without being bound by theory, as noted above, it is considered that the DBD by its specific recognition of a predetermined sequence in a double-stranded DNA assists in melting and/or unwinding of the double helix and thereby assists in targeting by the target nucleic acid and operation of the effector component at the desired site.

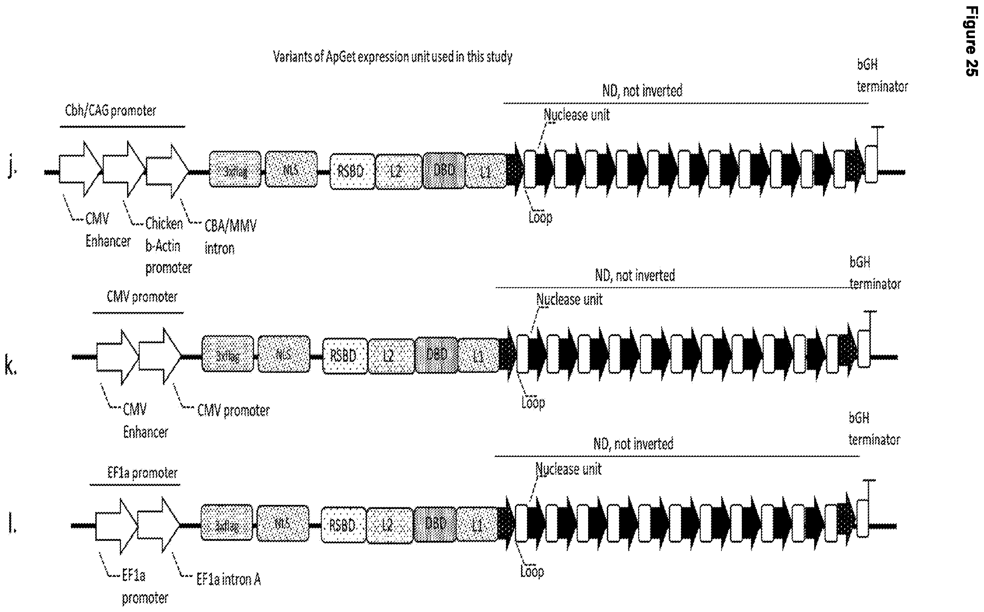

A nuclear localization signal (NLS) and/or organelle localization signal may also be provided as part of the modular polypeptide component for efficient transportation into the nucleus of a eukaryotic cell or efficient transportation into a desired cellular organelle e.g. a mitochondrial or chloroplast localization signal. Such a signal sequence will generally be provided at the N- or C-terminus. It may be connected to one or more additional sequences, e.g. a detection tag to aid detection such as an epitope tag, provided the required function of the modular polypeptide component is maintained; see Figure 25.

The effector component will not specifically bind to said predetermined sequence and commonly will be devoid of target binding ability. However, it will be recognised that it is merely essential that the effector component does not prevent site-specific targeting of the desired modification by the nucleic acid targeting element and DBD. This may not preclude the effector component having some target, e.g. DNA, recognition capacity.

As indicated above, modification of the target may be structural or chemical modification, e.g. a change of nucleotide sequence, or change of regulation, e.g. transcription activation.

The effector component may be any type of effector known for modifying DNA including, for example, a Fok1 nuclease domain (i.e. a Fok1 nuclease minus its DNA binding domain) which can form a functional nuclease when dimerization occurs, e.g. through appropriate targeting of two nucleoprotein complexes of the invention to a target DNA region. However, as indicated above, the effector component may preferably comprise an artificial nuclease formed from linked, self-assembling short peptides as further discussed below. Such an artificial nuclease may act as an artificial nickase in that it cuts just one strand of a double-stranded DNA at a targeted site. The effector component may be a fusion protein in which a nickase is linked to or replaced by another functional component, e.g. a base editor or reverse transcriptase.

As noted above, the DBD will be a short peptide, generally no more than a 20 mer, e.g. no more than a 16 mer-18 mer, preferably no more than a 15 mer, selected for binding affinity to a pre-determined sequence in the target of, for example 2-7, preferably 3-6 nucleotides, e.g. the 6 nucleotide sequence 5'GAGGTC3' in a dsDNA target as exemplified herein. The DBD is expected to aid scanning of a genome and establish initial contact next to the desired modification site, e.g. cleavage site. In addition, it is further highlighted that binding of the DBD to the target DNA is expected to trigger structural changes and subsequent melting at the adjacent sites. Such unwinding should help the TE to interrogate the adjacent sequences for complementarity and, where the TE is an RNA sequence, form an RNA-DNA complex (R-loop). Hence, the DBD component of a genome-editing tool of the invention is seen as an important module for improving genome modification efficiency which can be provided without compromising the wish for a short overall polypeptide component length, preferably compatible with expression from an AAV vector also expressing at least one targeting RNA.

The modular polypeptide component of a genome editing tool of the invention may thus comprise entirely synthetic short peptides with linkers and providing nickase or nuclease activity by means of an artificial nuclease at a target DNA site. Such a genome-editing tool employing a modular polypeptide component has been termed ApGet, standing for 'Artificial peptidic genome editing tool'. Such a polypeptide component may be no more than 220 amino acids (minus any cleavable tag, localization signal or any other sequence other than components (a)-(d)) as exemplified by the ApGet polypeptide of SEQ. ID. No. 1: 
[MDAQTRRRERRAEKQAQWKAANGGGGSGGGGSGGGGSGGGGSGGGGSGGGGSGGGGSSLETWLKHREKD
GGSGGGGSGGGGSGGGGGSIEKDIHINDGGIEIDIHIDSGGIEIDIHISSGSIEIDIHINNGNIEIDIHIGSDGIEIDIHI
GNSGIEIDIHIDNGGIEIDIHIDSDGIEIDIHISSSGIEIDIHIGSEGIEIDIHIGDSGIEIDKHINQGS]
 consisting of the following components fused in the N- terminal to C-terminal direction:
- an RSBD of 22 amino acids corresponding to the Lambda N22 protein,
   MDAQTRRRERRAEKQAQWKAAN (SEQ. ID. No. 2)
- the linker GGGGSGGGGSGGGGSGGGGSGGGGSGGGGSGGGGS (SEQ. ID. No. 3), i.e. (GGGGS)₇
- the DBD SLETWLKHREKDGGS (SEQ. ID. No. 4) which binds the dsDNA sequence represented by the 5' to 3' sequence 5'GAGGTC3'
- the linker GGGGSGGGGSGGGGGS (SEQ. ID. No. 5) and
- the nuclease module 
IEKDIHINDGGIEIDIHIDSGGIEIDIHISSGSIEIDIHINNGNIEIDIHIGSDGIEIDIHIGNSGIEIDIHID
NGGIEIDIHIDSDGIEIDIHISSSGIEIDIHIGSEGIEIDIHIGDSGIEIDKHINQGS (SEQ. ID. No. 6),
 minus any additional N- or C-terminal sequence which may be provided as indicated above, e.g. to provide a short NLS and possibly a detection tag.

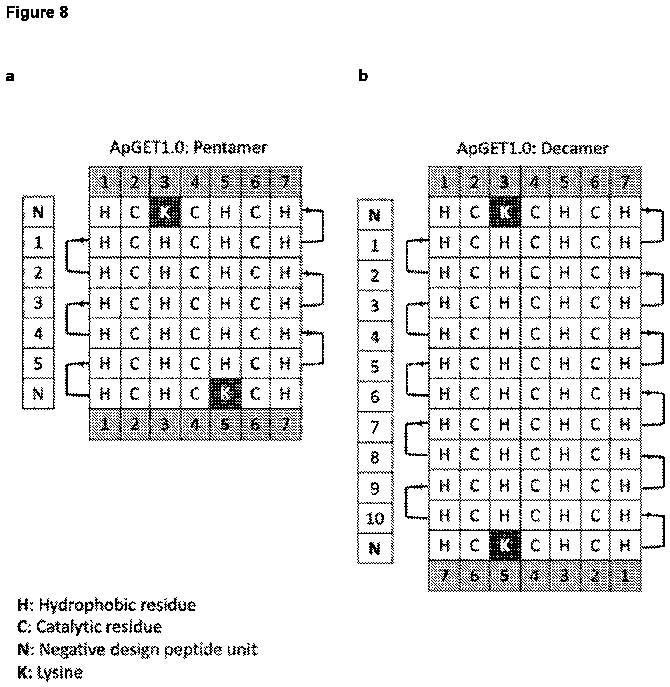

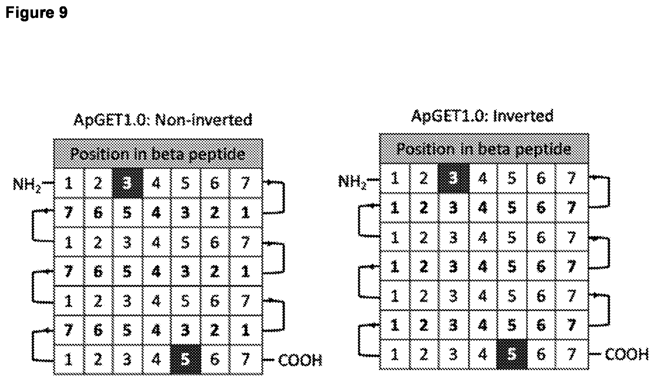

The nuclease (shown in bold in SEQ. ID. No.1 above) has ten identical 7 mer peptide units (IEIDIHI; SEQ.ID. No. 7) all linked in the same N to C-terminal direction (referred to herein as an example of a non-inverted decamer) with 4 mer linkers providing beta-turns and additional N- and C-terminal flanking sequences. This permits folding whereby the decamer of identical peptide units can produce a secondary structure resembling an anti-parallel beta sheet in which consecutive monomer units will appear orientated in opposite directions; see Figure 8b and Figure 9. The modular polypeptide component of SEQ. D. No. 1, as part of a DNA-editing tool of the invention, has been shown to be capable of cutting a single-strand of a dsDNA at a targeted site. Thus the artificial nuclease module can be viewed as providing the function of a nickase and may thus be referred to as an artificial nickase. It is to be expected that smaller such genome-editing tools can be achieved with, for example, provision of a nuclease with a lower multiplicity of self-assembling peptides and/or variance of other components. This includes any different peptide sequence of same pattern and also combination of different peptide sequences.

The artificial nuclease of SEQ. ID. No. 1 may be substituted by another effector component, e.g. a transcriptional activator such as VP64 or another effector component as discussed below. Moreover it will be recognised that one or both of the flexible linkers may be substituted by an alternative linker. Thus for example, linker 1 between the artificial nuclease (or a substitute effector component) and DBD may be substituted by the linker specified as linker L1a (SEQ. ID. No. 69). Linker 2 between the RSBD and DBD may be substituted, e.g. by a shorter linker as exemplified by linker 2b (SEQ. ID. No. 70). The linkers in a modular polypeptide component may be varied and optimised for any pair of DBD binding and target cutting sites. Such variation will be recognised to be a matter of length requirement calculation based on known target sequence information and appropriate testing. Such linkers may also be designed to increase the protein stability and or solubility.

While the Lambda N22 protein was chosen as the RSBD for the illustrative ApGet as set out as SEQ. ID. No. 1 above on the basis that this is only a short 22 amino acid sequence with well-known ability to recognise the 19 nucleotide box B lambda phage sequence (Baron-Benhamou et al.(2004) Methods Mol. Biol. 257, 135-54), it will be appreciated that this may additionally or alternatively be substituted by another RSBD, including any variant thereof which retains the desired binding affinity for an RNA scaffold, e.g. the same box B sequence. It will additionally be appreciated that an RS can be used in tandem to facilitate the binding of multiple RSBDs. A Lambda N22 protein RSBD paired with a Box B sequence provided as the RS of a targeting nucleic acid may be substituted by any of a number of other well-known RNA aptamer- polypeptide binding domain pairings as discussed further herein below.

The small size of an ApGet's polypeptide component is expected to improve the DNA scanning and recognition efficiency of the system compared with known gene-editing systems for producing targeted DNA strand breaks and employing larger molecular weight proteins such as ZFNs, TALENs, meganucleases and CRISPR-Cas entities. Such larger prior art systems will have lower diffusion rates.

The exemplification herein provided furthermore indicates that an ApGet minus any nuclease or other effector component, i.e. just comprising the nucleic acid recognition module, e.g. an. RSBD, linked to a DBD and coupled with an appropriate targeting nucleic acid may have utility in inhibiting transcription at a target site. Such a system is referred to herein as an ApGet-i system. In some instances, it may be chosen to express an ApGet-i polypeptide with addition of a final C-terminal linker sequence, e.g. the linker of SEQ. ID. No. 5 above. However, for transcription inhibition an ApGet-i polypeptide minus such a linker will be preferred.

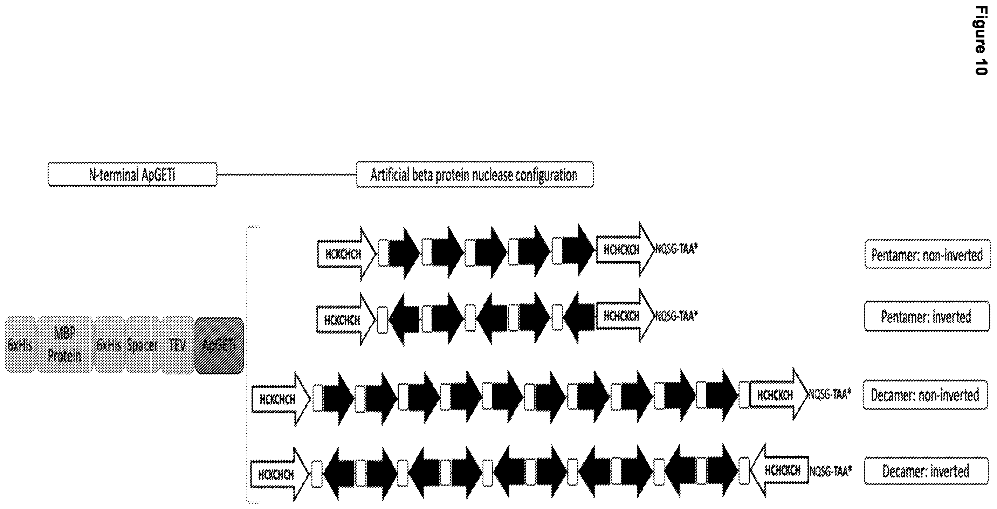

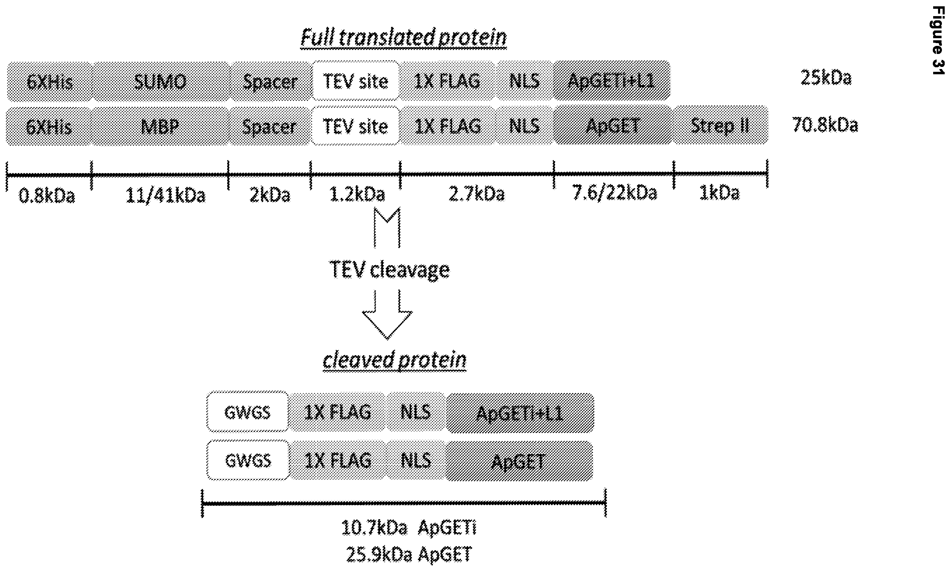

It will be recognised that a modular polypeptide component for a nucleoprotein complex of the invention may be initially expressed as part of a longer polypeptide with an N-terminal and /or C-terminal extension to aid for example solubility in an expression system, e.g. a host cell such as *E. coli* or an *in vitro* expression system and /or purification and /or detection. Such an extension may include a protease cleavage site, e.g. a TEV protease cleavage site whereby protease cleavage removes unwanted sequence in the final modular polypeptide for target modification; see Figures 10 and 31.

In some instances, it may be chosen to express the modular polypeptide component with inclusion of a protein transduction domain (PTD), also sometimes referred to as a cell-penetrating peptide (CPP) or domain to facilitate delivery across a membrane, e.g. into a target cell. Again such a domain may be provided with a linker providing a protease cleavage site. Such a PTD may be positioned at the N-terminus or C-terminus. In general, PTDs can be classified into 3 types: cationic peptides of for example 6-12 amino acids in length, comprised predominately of arginine, ornithine and /or lysine residues, hydrophobic peptides such as leader sequences of secreted growth factors and cytokines and cell specific peptides. Exemplary PTDs include but are not limited to that of the HIV TAT protein, a polyarginine peptide sequence, a VP22 domain (Zender et al. (2002) Cancer Gene Ther. 9,, 486-96), a PDX1 protein transduction domain (Noguchi et al. (2003) Diabetes 52, 1732-1737), a truncated human calcitonin peptide (Trehin et al. (2004) Pharma. Res. 21 1248-1256) and a polylysine sequence (Wender et al. (2000) Proc. Natl. Acad. Sci. USA 97, 13003-13008). An activatable CPP (ACPP) may be provided comprising a polycationic CPP (e.g. Arg9 or R9) connected via a cleavable linker to a matching polyanion (e.g. Glu9 or E9) which reduces the net charge to nearly zero and thereby inhibits adhesion and uptake into cells (Aguilera et al. (2009) Integr. Biol. (Camb) 1, 371-381) Upon cleavage of the linker, the polyanion is released thus locally unmasking the polyarginine and activating its inherent adhesiveness to facilitate membrane transport.

A nucleoprotein complex of the invention may be delivered to host cells either as an active complex, e.g. by electroporation, or with one or both of the polypeptide component and targeting nucleic acid expressed by a polynucleotide.

Thus, in a further aspect, there is provided a nucleic acid or combination of nucleic acids for provision of one or more nucleoprotein complexes of the invention in a host cell. As indicated above, the nucleic acid may preferably be a vector, e.g. an AAV vector, capable of expressing both the modular polypeptide component, e.g. including an artificial nickase described herein, and the targeting nucleic acid. More than one targeting nucleic acid may be provided to a host cell to target more than one site. For example, a pair of nucleoprotein complexes of the invention including an artificial nickase may be provided by vector delivery to a cell, possibly by means of a single vector, together with a sequence template for homologous recombination.

Applications of a nucleoprotein complex or complexes of the invention extend to the full panoply of applications for DNA modification for which a CRISPR-Cas/sgRNA may be employed. Thus, as a further aspect of the invention, there is provided a method for modifying one or more target nucleic acid sequences employing one or more nucleoprotein complexes of the invention or one or more nucleic acids for provision thereof in a host cell with the proviso that such methods as claimed do not extend to methods of modifying the germ line identity of a human being or methods of medical treatment practised on a human or animal body as such. As indicated above, desirably, a single modular polypeptide component for provision of the one or more nucleoprotein complexes may be provided in cells by expression from a vector. The same vector may preferably also express the required one or more targeting nucleic acids.

As a still further aspect of the invention there is provided a combination of (i) at least one modular polypeptide component for a genome modification tool of the invention, or a polynucleotide encoding the same and (ii) one or more targeting nucleic acids which can link with said polypeptide component (s), or one or more polynucleotides encoding the same, for use in a method of the invention as discussed above or for use in therapeutic treatment. As indicated above both the polypeptide component and the one or more targeting nucleic acids may preferably be expressed from a single vector.

Also described herein is an artificial nuclease that is suitable for use in an ApGet nucleoprotein complex. Use of self-assembling peptide clusters incorporating catalytic residues have been previously used to generate artificial enzymes acting as esterases or providing a number of other enzymatic reactions. However, the inventors in this instance have for the first time attained an artificial nuclease which can provide nickase function in a dsDNA using linked, self-assembling short peptides with an approach designed to achieve the required function while minimising size and thereby providing an artificial enzyme well-suited for providing the effector module in a genome editing tool. Linking individual peptide units in this way also helps to control the extent of self-assembly.

Such artificial nucleases of the disclosure and genome modifying nucleoprotein complexes of the invention will be discussed in more detail below with reference to the following figures.

### Brief description of the figures

**Figure 1****:** Schematic diagram of a genome editing tool of the invention consisting of a nucleic acid component (NAC) and modular polypeptide component. The NAC is a targeting RNA in which TE is the targeting element providing a sequence complementary to a strand region of the target. RS is an RNA scaffold part of the NAC which binds to an RNA scaffold binding domain (RSBD) whereby the NAC interacts with the modular polypeptide component. The TE and RS form a single nucleic acid with a connecting sequence joining the two functional binding sequences such that the required binding can occur. DBD is the DNA binding domain of the modular polypeptide component which recognises a predetermined sequence (PDS) of the target DNA, L1 is a linker linking the DBD to the effector protein for genome modification, e.g. a nuclease (an endonuclease for double-strand breaks or a nickase for single-strand breaks), a transcription activator or repressor or a methylase, and L2 is a linker linking the DBD and RSBD. The target sequences can either be immediately adjacent to the PDS, or spaced from the PDS

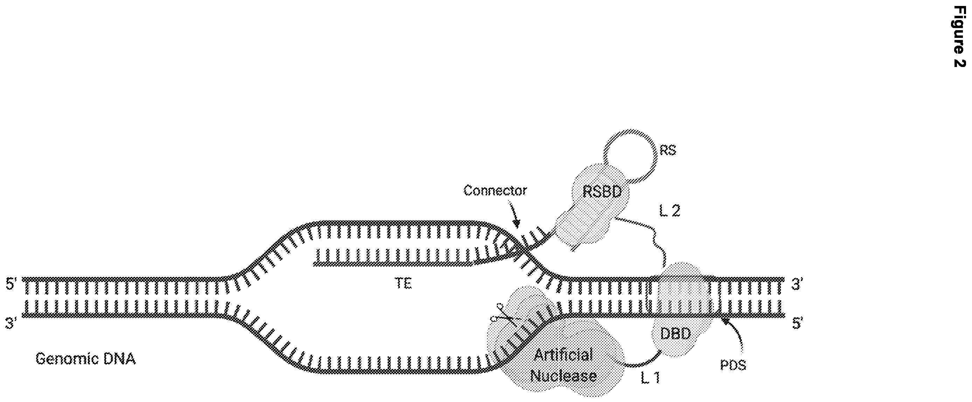

**Figure 2****:** Schematic diagram more specifically of the ApGet system where the modular polypeptide component includes a fused artificial nuclease. TE is the targeting element of the targeting RNA which hybridises to a sequence at the target DNA region for nickase action. The recognition element of the targeting RNA is joined to the TE by a connecting sequence and provides an RNA scaffold (RS) which binds to an RNA scaffold binding domain (RSBD). DBD is the DNA binding domain which binds to the predetermined sequence (PDS) on one strand of the target dsDNA. L1 and L2 are peptide linkers. Binding of the DBD to the PDS facilitates hybridization of the TE to its complementary sequence.

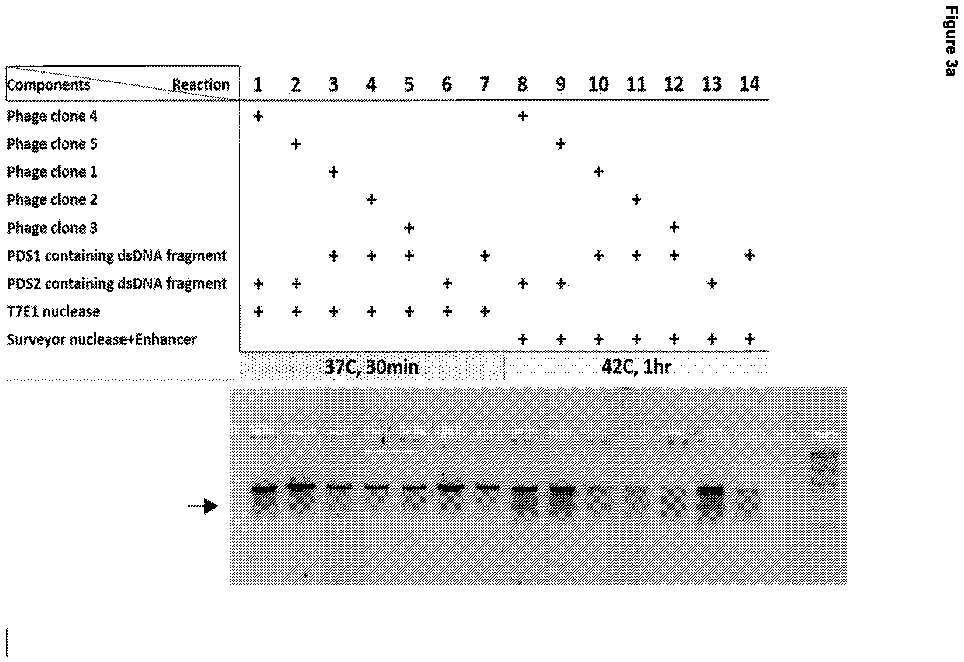

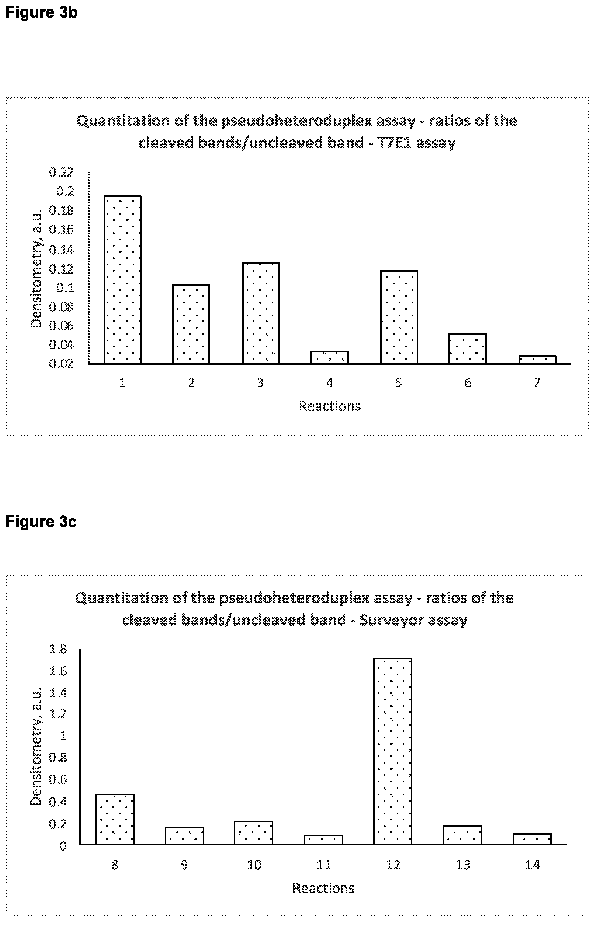

**Figures 3a****-c:** Evidence of the specific binding and unwinding of dsDNA targets by selected phage clones presenting 12 mer polypeptides. The dsDNA targets presented two PDS sequences: 5'-GAGGTC-3' (PDS1) and 5'- ACGGGT -3' (PDS2). The selected phage clones were incubated with dsDNA containing either PDS1 (reactions 3, 4, 5, 10, 11 and 12) or PDS2 (reactions 1, 2, 8 and 9) sequences and with either T7E1 nuclease (reactions 1-5) or Surveyor nuclease (reactions 8-12). Mock reactions are the incubations of the dsDNA without presence of the selected phage clones, with either T7E1 (reactions 6 and 7) or Surveyor (reactions 13 and 14). Upon binding of the phage clones to the dsDNA partial unwinding may occur, which generates a structure similar to heteroduplex DNA, providing a suitable substrate for endonucleolytic cleavage by T7E1 and Surveyor nucleases and thereby resulting in a faster migrating DNA band (see arrow in Figure 3a). Quantification of the T7E1 and Surveyor mediated cleavage was performed by densitometry analysis using ImageJ software. The results are shown in Figures 3b and 3c respectively.

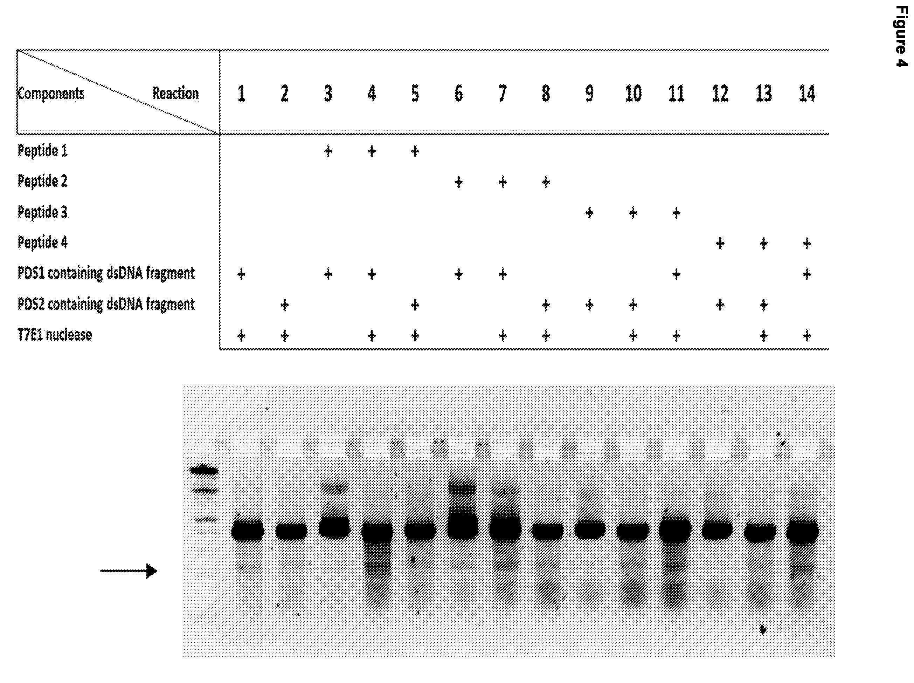

**Figure 4****:** Cleavage assay results using T7E1 enzyme for binding of the chemically synthesized peptides 1, 2, 3 and 4 (Table 2 in Example 1) to the two 6 nt sequences as above designated PDS1 and PDS2 in a dsDNA target and unwinding of the dsDNA target. The arrow shows the band which intensifies upon T7E1 endonucleolytic cleavage of the dsDNA target. These results led to selection of the Peptide 1 sequence (SEQ. ID. No: 4, also shown in Table 2 as SEQ. ID. No. 37, and incorporating a GGS linker sequence) as a DNA-binding domain for the 6 nt PDS 5'-GAGGTC-3'.

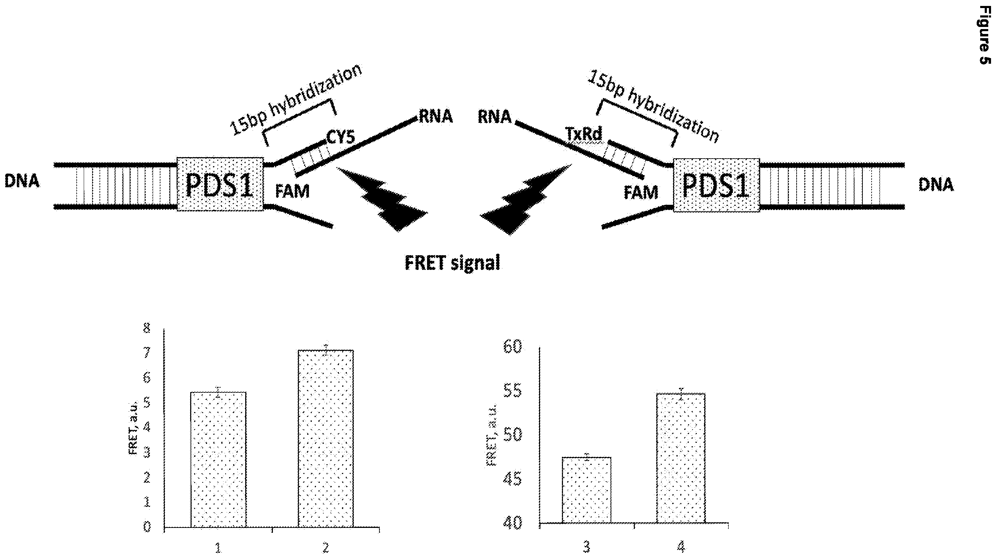

**Figure 5****:** Schematic diagram illustrating a FRET experiment to assess specific binding and unwinding of a dsDNA target by peptide binding to a PDS with RNA hybridization. Results are also shown for binding of Peptide 1 (SEQ. ID. No. 37 as shown in Table 2) to PDS1 having the 5' to 3' sequence GAGGTC. The FRET signal was determined upon incubation of the PDS1 containing dsDNA targets with 5' FAM-RNA with (2, 4) or without peptide (1, 3). Binding of Peptide 1 is shown to facilitate hybridization of the RNA to sequences adjacent to PDS1 as evident from the increase of FRET signal.

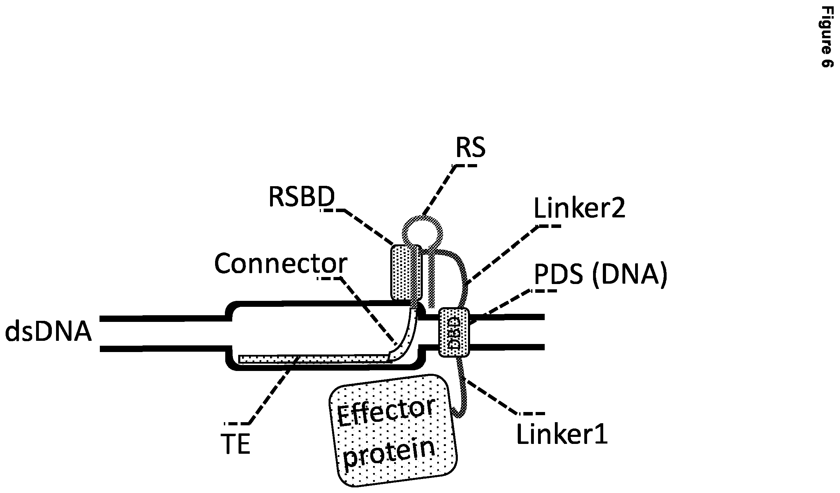

**Figure 6****:** Schematic diagram of a genome-editing tool of the invention showing possible alternative strand hybridization of a targeting element (TE) of a targeting RNA. RS is the RNA scaffold for tethering the targeting RNA to the modular polypeptide component via the RNA scaffold binding domain (RSBD). DBD is the DNA-binding domain which binds a short predetermined sequence (PDS) to facilitate heteroduplex formation. The DBD is linked via linker to a C-terminal effector protein for nucleic acid modification.

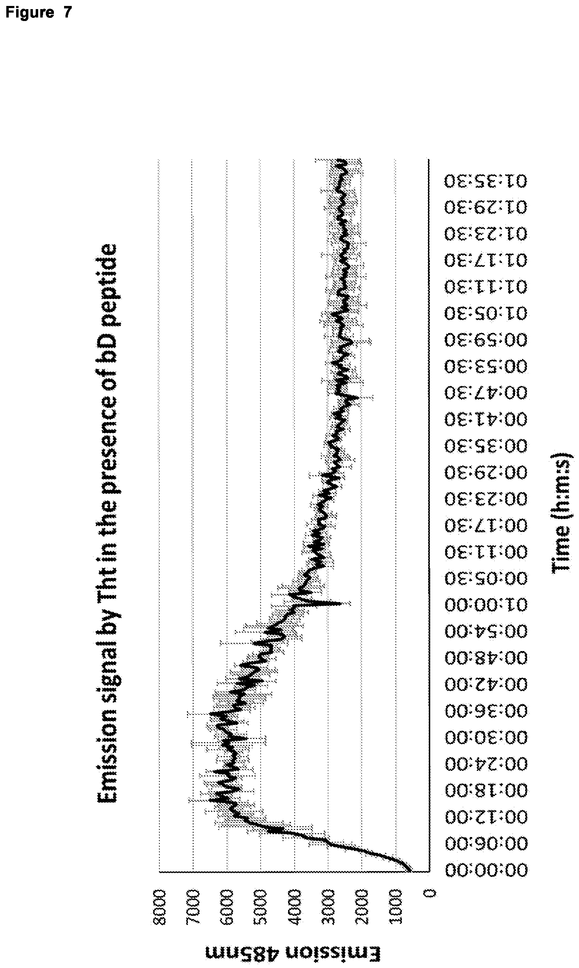

**Figure 7****:** Emission signal of Thioflavin T (Tht) in the presence of the 7 amino acid peptide LELDLHL (bD peptide; SEQ. ID. no. 8) indicative of production of amyloid β-sheet structure.
**Figure 8****:** Designs for an artificial nuclease (a) employing a pentamer or (b) a decamer of linked identical beta-sheet forming peptides with flanking peptide units with increased positive charge through substitution of a hydrophobic residue by a positively charged residue such as lysine or arginine. H = hydrophobic reside. C = catalytic amino acid. N= negative design peptide unit (which limits or avoids inter-molecule polymerization), in this case C-terminal and N- terminal peptide units with increased positive charge. K= lysine residue substituted for a hydrophobic residue of the beta-sheet forming peptide. The C-terminal flanking peptide may also have a final flexible soluble tail, e.g. the 4mer NQRS. Each of the preceding peptide units is linked by a linker, e.g. of 4 amino acid residues providing a beta turn.
**Figure 9****:** "Non-inverted and inverted" artificial nuclease designs. "Non-inverted" means that the individual beta strand units of the nuclease share the same primary sequence in the N-to C-terminal direction whereas in the "inverted" design the primary sequence of consecutive beta stand units is reversed. Boxes 1-7 signify the amino acid sequence of the beta peptide in the N-C terminal direction.
**Figure 10****:** Diagram illustrating the assembly of pentamer and decamer beta protein nucleases in a complete modular polypeptide component of an ApGet. The beta peptide units (black) are assembled using either five or ten beta peptide units. The units are connected using a series of beta turns like connecting loops. Each design contains additional flanking beta peptide units that incorporate a charged residue (lysine, K) to limit inter-molecule polymerization, i.e. negative design (flanking arrows). The orientation of beta peptide sequence is modified to mimic either inverted or non-inverted in an attempt to mimic either a parallel or antiparallel beta sheet fold (see again boxes 1-7 in Figure 9 signifying the amino acid sequence of the beta peptide in the N-C terminal direction). The N-terminus of each artificial beta protein nuclease design is fused with linker 1 as in the ApGet-1.0 protein onto the C-terminal end of the ApGet-i protein providing the remainder of the required modules for a complete synthetic genome editing system. The ApGet-i can contain several purification/solubility tags to enhance protein expression and solubility. Shown is such a tag providing a maltose-binding protein fused to a 6x His tag at both the N-terminus and C-terminus. The C-terminal His tag is separated from the N-terminal of the ApGet by a spacer unit and TEV cleavage site whereby the whole multi-element tag can be removed.

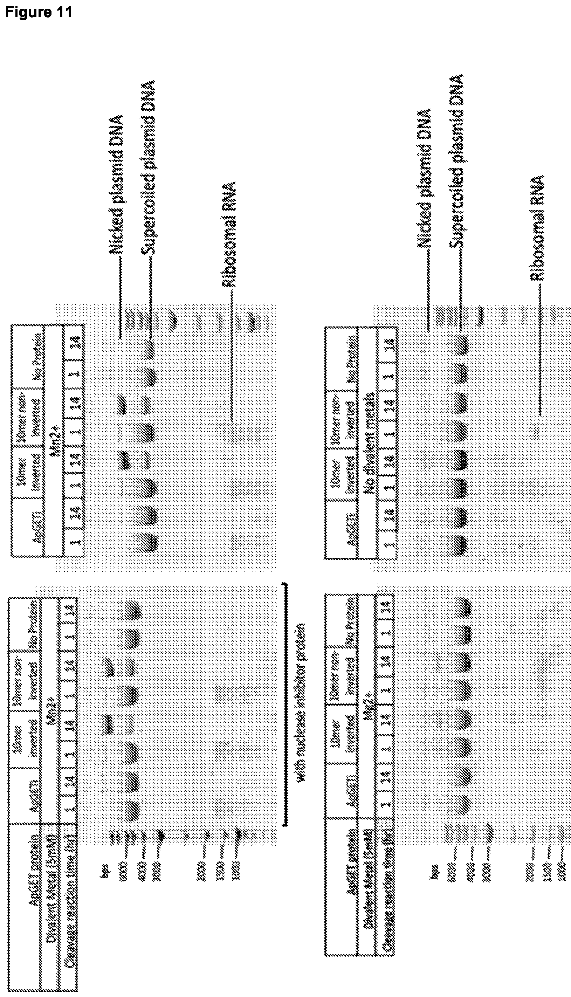

**Figure 11****:** Gel analysis of plasmid DNA cleavage by *in vitro* transcription and translation (IVTT)-expressed ApGet1.0 protein with 10mer of the IbD peptide. ApGet-i (expressed with additional inclusion of the DBD-enzyme linker of ApGet1.0 at the C-terminus) and a no protein control were carried out for each buffer condition to control for activity enabled by the buffer composition or by the contaminating protein components of the IVTT mix. Plasmid DNA was mixed with identical quantities of total IVTT protein mix (as per Abs 280nm) for ApGet1.0 10mer non-inverted, inverted, ApGeti and a water control in reaction buffer containing either no metal or 5mM Mn²⁺ or Mg²⁺ A secondary reaction comparing the cleavage of the IVTT proteins in the presence of Mn²⁺ and nuclease inhibitor protein was also carried out.

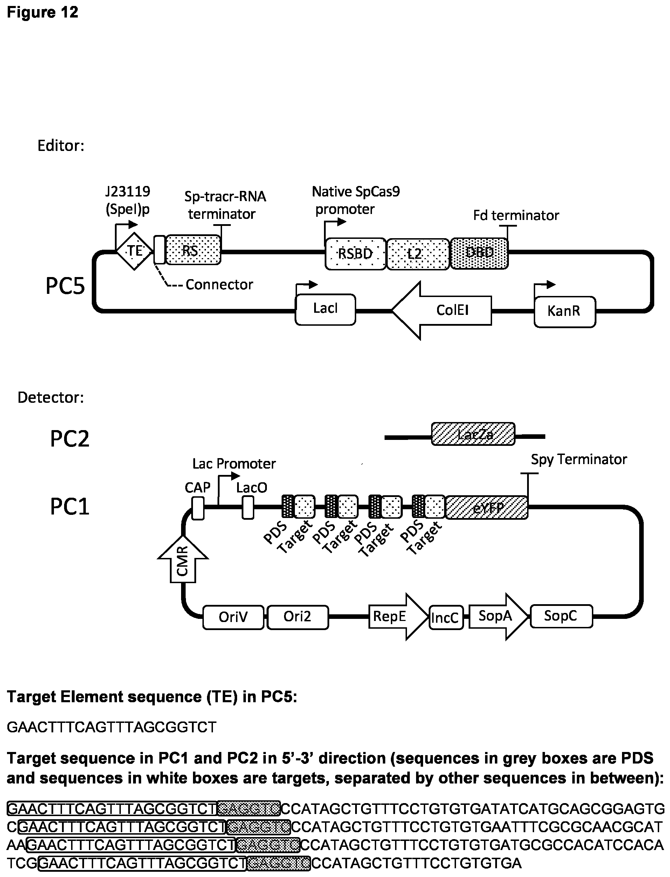

**Figure 12****:** Diagrams of the plasmids used in testing recruitment of an ApGet-i by a targeting nucleic acid to PDS-target sequence sites in *E*. *coli* cells.

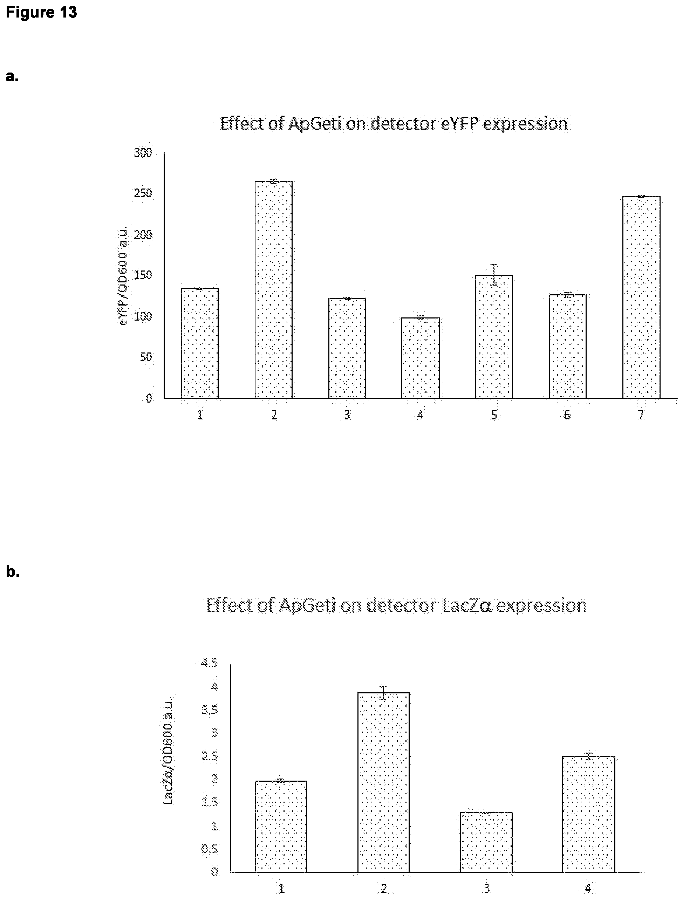

**Figure 13****:** Results of testing ApGet-i for reducing transcriptional activity on (a) detector for eYFP expression and (b) detector for LacZα expression in EPI300 *E.coli* cells. Tables 5 and 6 in the exemplification summarise the construct testing. The values presented are average values of the eYFP/OD600 or LacZα/OD600 of the experiments done in triplicate with the standard deviation indicated.

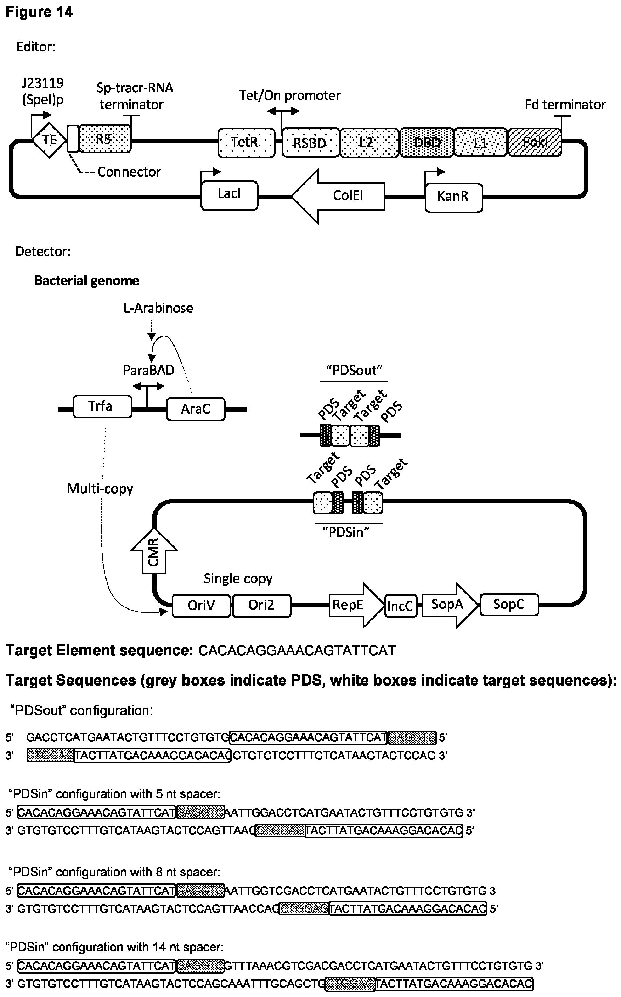

**Figure 14****:** Diagram of the 'Editor' plasmid and 'Detector' plasmid used in testing a complete ApGet system in *E. coli* cells. The ApGet modular polypeptide component was the amino acid sequence of SEQ. ID. No.1 but with a Fok1 nuclease domain instead of the artificial nuclease.

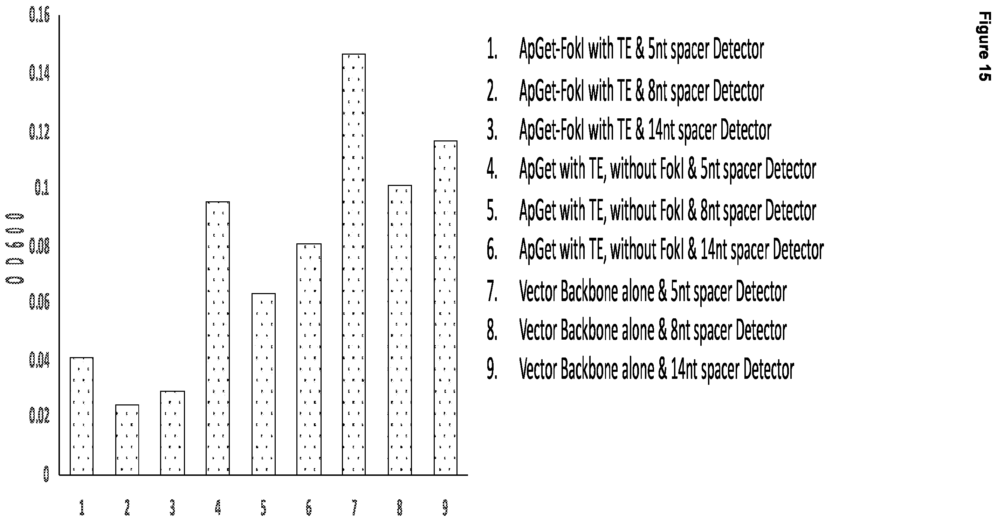

**Figure 15****:** Results of ApGet-Fokl editing obtained using 'Editor' and 'Detector' plasmids as illustrated in Figure 14. Editing by the ApGet- Fok1 nuclease construct is evident from the loss of the target-containing detector plasmid resulting in reduced bacterial growth on the selective media (see bars 1-3). The results shown are from co-transformation of EPI300 *E*.
*coli* cells with an ApGet-Fokl expressing editor plasmid with detector plasmids containing "PDSin" configurations of the targets and different length spacers between the target sequences. Detection of the editing activity was by measuring OD600 a day after induction of ApGet with Anhydrotetracycline (ATC). Control experiments included co-transformation of either ApGet-i expressing editor (ApGet construct, lacking a nuclease domain) or non-ApGet expressing plasmid and detector plasmids into EPI300 cells. The bars correspond to expression of the ApGet-Fok1 construct and detector targets as follows:
   1. ApGet-Fok1 with TE and 5nt spacer detector
   2. ApGet-Fok1 with TE and 8nt spacer detector
   3. ApGet-Fok1 with TE and 14nt spacer
   4. ApGet with TE, without Fok1 and 5nt spacer detector
   5. ApGet with TE, without Fok1 and 8nt spacer detector
   6. ApGet with TE, without Fok1 and 14nt spacer detector
   7. Vector backbone alone and 5nt spacer detector
   8. Vector backbone alone and 8nt spacer detector
   9. Vector backbone alone and 14nt spacer detector.
**Figure 16****:** Schematic representation of 'Editor' plasmids used in testing the ApGet system with artificial nuclease in bacterial cells.

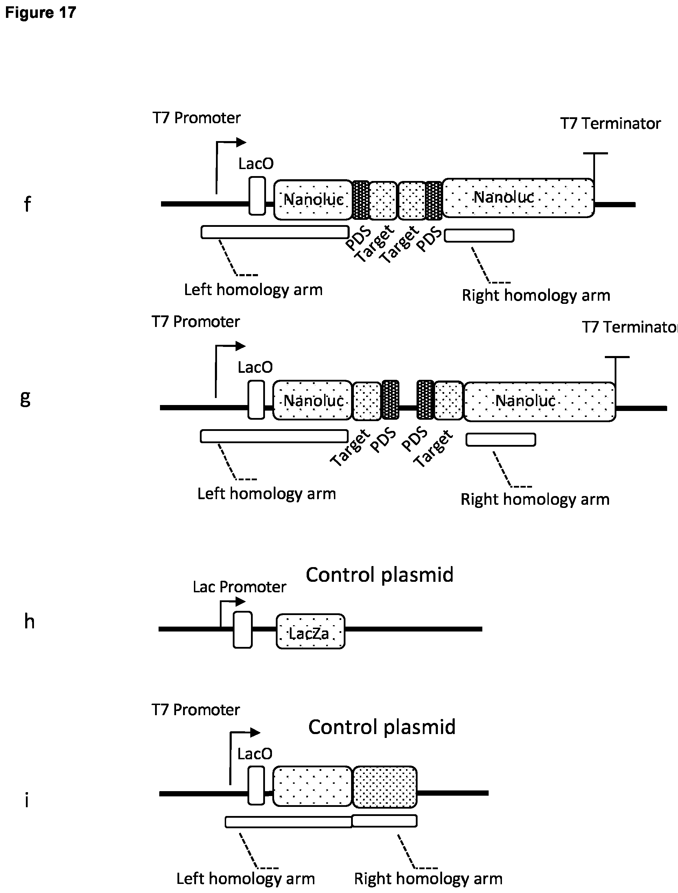

**Figure 17****:** Schematic representation of 'Detector' plasmids used in testing ApGet systems possessing an artificial nuclease in bacterial cells. Detectors were designed with either 'PDS-out' configuration (construct f) or 'PDS- in' configuration (construct g) for the pair of PDS-target sequence elements. Target and PDS sequences of PDSin and PDSout detectors are located exactly between the homology arms, as in Figure 14, and are identical to those in Figure 14. Successful targeting is shown by observation of nanoluciferase activity in the presence of a suitable HDR template. The Homology-directed repair (HDR) template for the nanoluciferase was constructed on the same plasmids that express ApGet as a separate unit.

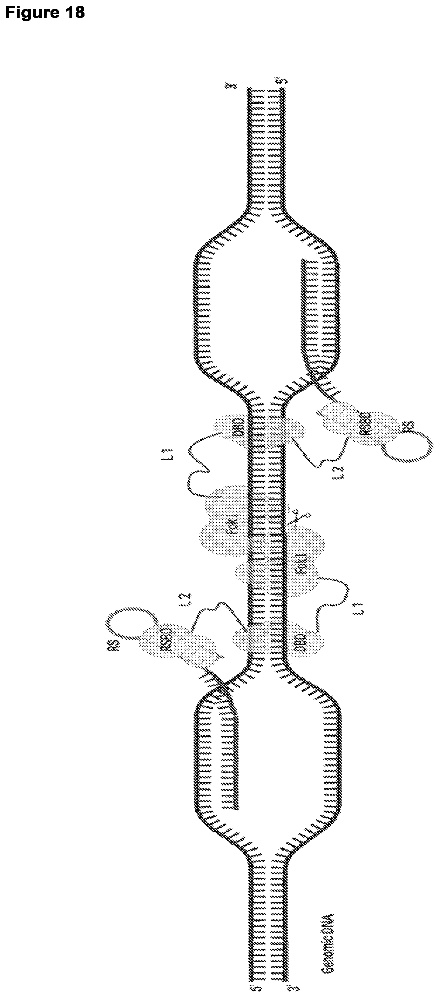

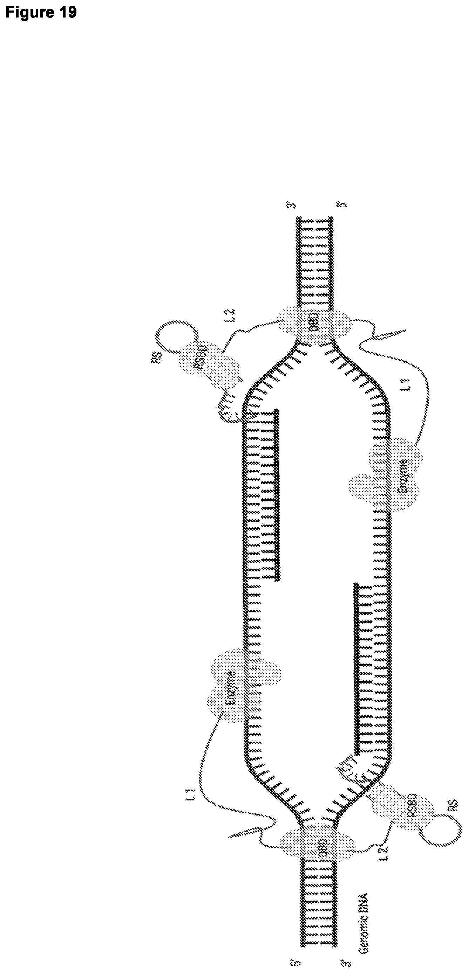

**Figures 18** **and** **19** are schematic diagrams showing possible alternative heteroduplex interactions when a pair of genome editing tools of the invention are employed to modify, e.g. cut, both strands of a target genomic DNA at chosen sites. In **Figure 18****,** each polypeptide component is shown as including a Fok1 nuclease domain. Two such nuclease domains are shown as being caused to dimerise to provide a functional endonuclease by use of two targeting RNAs which target different sequences on opposite strands of the target DNA. The two DNA-binding domains are shown as each binding to a PDS in a fully complementary region of the target DNA in which the Fok1 endonuclease cuts. The PDS sequences are both internal to the pair of target sequences. This is referred to as employing PDS-in positions. In **Figure 19****,** each polypeptide component is shown as including a fused enzyme which may be for example a fused artificial nickase as herein described. In this case, each DBD is shown as binding to a PDS outside a single heteroduplex region formed by the targeting sequences of two different targeting RNAs hybridising with a different target strand. Each of the pair of enzyme domains acts on a different strand within the heteroduplex region. Since the pair of PDS sequences are outside the pair of target sequences, this scheme is referred to as employing PDS-out positions.

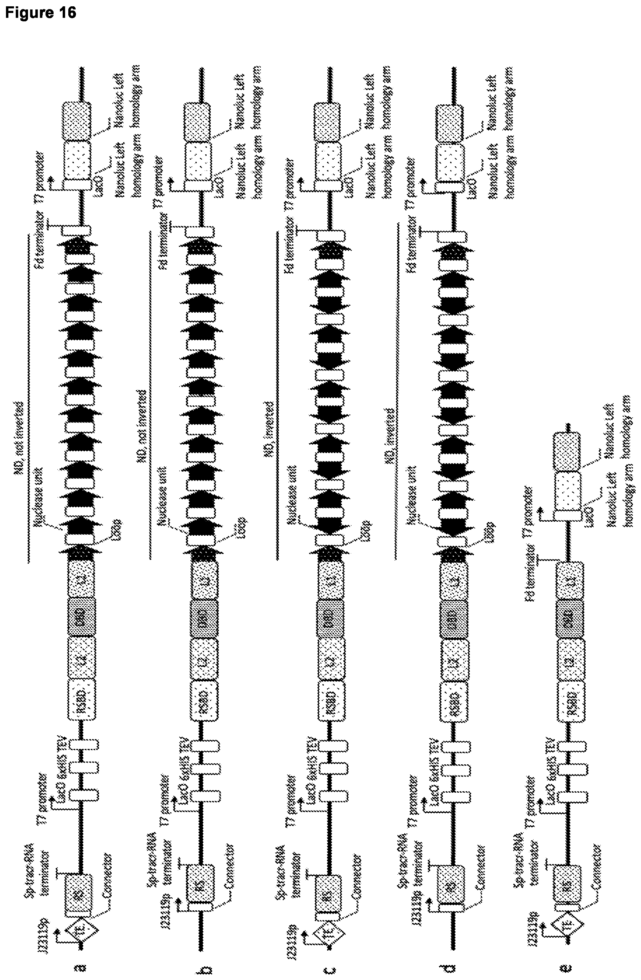

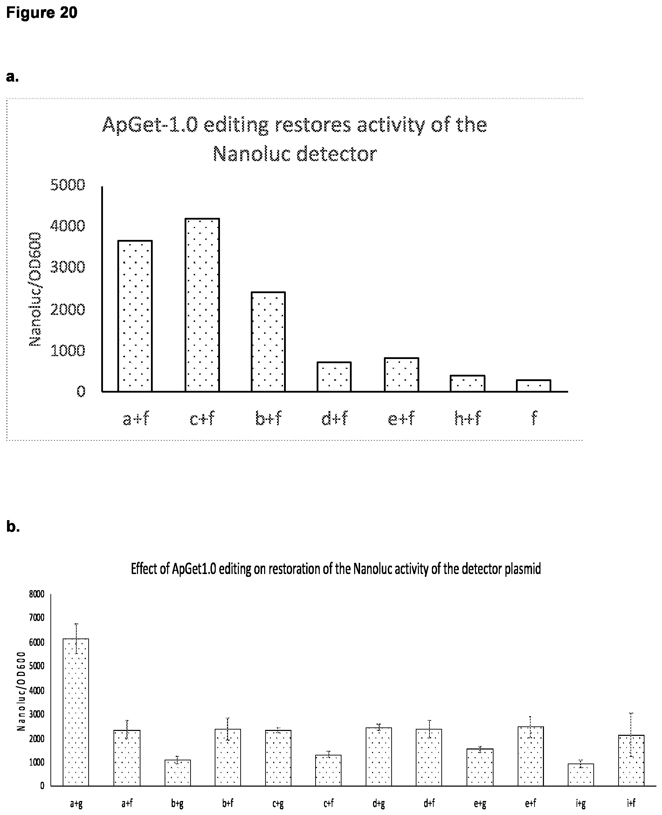

**Figures 20a** **and b:** Results of tests of ApGet editing to restore functional nanoluciferase by triggering homology directed repair of a disrupted nanoluciferase open reading frame. Various Editor plasmids including an expression cassette for various configurations of ApGet1.0 (Figure 16) and with an expression cassette for nanoluciferase HDR template were co-transformed with the detector plasmids expressing disrupted open reading frame of the nanoluciferase with the targets in "PDSin" and "PDSout" configurations (Figure 17). Colonies of co-transformants of ApGet1.0 with detectors were grown in liquid medium with IPTG or with and without IPTG for same construct and corresponding antibiotics and Nanoluc/OD600 signal was analysed next day. Error bars are standard deviation of three experiments.
**Figures 21** **and** **22** illustrate the ApGet-i expressing Editor plasmid and Detector plasmids respectively used in evaluating the flexibility of the DBD of SEQ. ID. No. 4 for binding to variants of PDS1 (5'-GAGGTC-3').
**Figures 23a** **and b** show the control plasmid used with the detector plasmids of Figure 22 and the results for the binding ability of the tested DBD for various 6mer PDSs.
**Figure 24****:** Schematic representation of the ApGet expressing plasmid construct designed for transient expression of ApGet variants in mammalian cell lines.
**Figure 25****:** Schematic representations of the variant ApGet expression units incorporated into the plasmid construct as shown in Figure 24 together with a high copy number bacterial origin of replication and antibiotic resistance gene. Each expression unit provided at the N-terminus of the ApGet a detection tag (FLAG^{®} epitope tag) preceded by a nuclear localization signal linked to the RSBD.
**Figure 26****:** Layout of the ApGet targeting region in exon 4 of the PD-L1 gene. TE binding regions designated as PD-L1 target sites are flanked by PDS sites. The direction of the TE binding region shows forward and reverse complementary strand binding sites for the ApGet.
**Figure 27****:** Reduction of PD-L1 expression in cell cultures transfected with ApGet. Quantitation of the PD-L1 expression in cell samples from each image (ImageJ). Error bar is standard deviation of signal strength of individual cells in the sample.
**Figure 28** provides (a) a representation of the genome target region and donor template structure as discussed in Example 6(ii) to investigate ApGet-mediated repair of a target region in the PD-L1 gene and (b) a table setting out the editing components employed and sample results for PCR confirmation of an HDR event.
**Figure 29** shows the genomic reference sequence with the layout of primers that was used to evaluate targeting of the TSKU gene in HEK293 cells with ApGet expressing constructs as reported in Example 6(iii).
**Figure 30****:** TIDER analysis from the studies reported in Example 6(iii) using Sangar sequencing which compared the levels of substitution from the wild-type sequence between the untreated samples and ApGet transfected samples.
**Figure 31****:** Configurations of recombinant ApGet proteins purified from an *E. coli* expression system. The configurations detail the various purification, solubility and cleavage tags as discussed in Example 7.
**Figure 32****:** Illustration of the expression cassettes employed for expressing an ApGeti-VP64 construct as discussed in Example 8 for transcriptional activation of the ASCL-1 gene in HEK293T cells.
**Figure 33****:** Results of transcriptional activation of the ASCL-1 gene in HEK293T cells using ApGeti-VP64 constructs expressed in conjunction with 4 NACs targeting different promoter locations and comparison with a dCas9-VP64 fusion construct.

### Detailed description

A targeting nucleic acid for provision of a genome editing tool of the invention will generally be an RNA. The targeting element (TE) is complementary to a sequence on the target DNA. By "complementary" will be understood that the TE hybridises to the target sequence to fulfil its targeting purpose; generally it will be fully complementary to the target sequence.

Generally, the TE will be 15-25 nucleotides, for example, 15-20 or 21 nucleotides, preferably 18-20 or 21 nucleotides. Longer or shorter TEs may however be feasible in some circumstances, e.g. a TE of about 10 to 35 nucleotides. However, in order to target a specific sequence in a genome with a single-stranded nucleic acid, e.g. single-stranded RNA, it is necessary that the selected target sequence is in an unwound and therefore accessible state. The inventors in this instance hypothesized that this could be assisted by providing a small peptide that can bind a proximal short predetermined dsDNA sequence (PDS), e.g. of 3-6 nucleotides. Without wishing to be bound by theory, it was reasoned that high-affinity binding interaction between a peptide (the DNA-binding domain or DBD) and a PDS may destabilise Watson-Crick base pairing in the adjacent regions of the dsDNA helix which in turn will facilitate the hybridisation of the TE to its complementary target region. Where the TE is an RNA sequence, there will thereby be formation of an R loop - a region of otherwise double-stranded DNA where a sequence of single-stranded DNA is displaced while its complement forms an RNA/DNA hybrid helix with an invading RNA strand. As indicated above, a DNA-binding domain of this type is considered an essential element of a genome-editing tool of the invention which aids in optimising efficiency of the required genome modification.

Provision of such a DBD to a predetermined sequence can be achieved by various methods. Such methods may comprise use of computational methods for ligand design. However, a preferred method as exemplified herein is use of a phage display peptide library to select displayed peptides with binding affinity for the chosen target dsDNA sequence. Use of such a bio-panning method is illustrated herein by selection of DBD candidates using a commercially available M13 phage display library which contains around 1 x 10⁹ unique 12 amino acid length peptides fused to the N-terminus of the phage minor coat protein III by a GGS linker. See Example 1. By this means a preferred 15mer peptide was selected with an incorporated C-terminal GGS (see SEQ. ID. No. 4 above), which binds to the 6 mer dsDNA sequence 5'GAGGTC3' representing a possible choice as the PDS. This represents just one DBD-PDS combination which may be employed. It will be recognised that many alternative DBD peptides may be found which bind other desired short dsDNA sequences.

As indicated above, a DBD may for example be a polypeptide sequence of no more than 70-75 amino acid residues, e.g. no more than a 30 mer, no more than a 25 mer, no more than a 20mer, preferably no more than a 15mer. In keeping with the desirability to minimise size of the total polynucleotide component for a genome editing tool of the invention, provision of a DBD of no more than 15 amino acids, e.g. about 12-15 amino acids is however favoured (including possibly a GGS element at the C-terminus). Furthermore, it will be recognised that the DBD may not necessarily require recognition of 6bp. It could be less than 6bp. For example, 5'NNGG3' or 5'GG3'.

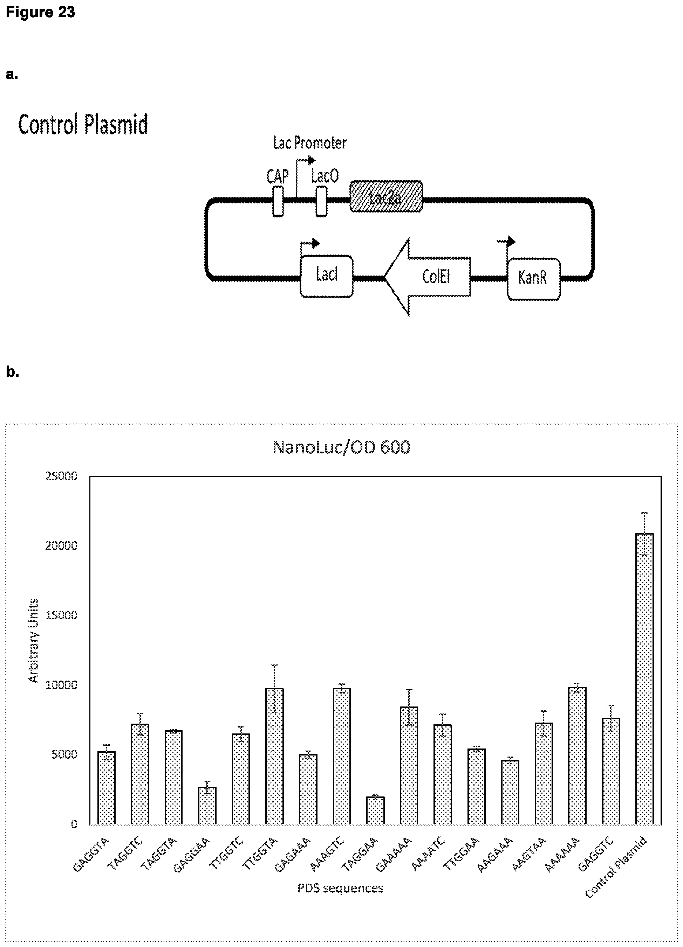

As indicated above, the chosen DBD may have binding affinity for more than one predetermined sequence, e.g. more than one 6 mer dsDNA sequence. This may be favoured to provide flexibility of use at different DNA sites. Thus the exemplified DBD of SEQ. ID no. 4 has been shown to be effective with the same targeting nucleic acid when presented with a number of 6mer dsDNA sequences other than 5'GAGGTC3' as a PDS; see Example 5 and Figure 23b. It may be particularly favoured to also pair, for example, with a PDS of sequence 5 'TTGGTA3', 5'AAAAAA3' or 5'AAAGTC3'.

The DBD may for example be chosen to target parts of the genome that a CRISPR-Cas system, e.g. a Cas9 CRISPR system cannot access due to PAM restriction. It may for example be specifically designed to bind to AT rich regions of the genome rather than regions providing a Cas9 PAM, i.e.5'-NGG-3', e.g. in a CGG region.

The targeting nucleic acid component must be bound by a module of the modular polypeptide component. Preferably this will be an end module. This interaction may be by various means known for linking a nucleic acid with a recognition domain in a polypeptide structure. For example, the targeting nucleic acid component may present as its recognition element for interaction with the modular polypeptide component a non-nucleic acid label which is a member of a specific binding pair. This will bind with its binding partner presented by the modular polypeptide component. By way of example, the specific binding pair may be biotin-streptavidin or the nucleic acid may be coupled to an antigenic epitope in which case the recognition domain of the modular polypeptide may comprise a ScFV for binding the epitope. However, more preferably where the targeting nucleic acid component is an RNA, as indicated above, the provided recognition element will be an RNA motif (alternatively referred to as an RNA scaffold) which binds a module of the polypeptide component referred to herein as an RNA scaffold binding domain (RSBD). Many such naturally occurring RNA scaffold-RSBD interactions are known, often referred to in the literature as RNA aptamer-binding domain interactions, for tethering an RNA sequence to a protein domain. Examples of such binding complexes include (i) the 19 nt Box B RNA motif and its cognate 22 amino acid RNA-binding domain of the lambda phage anti-terminator N protein (the lambda N22 peptide; see SEQ. ID. No. 2 above) (ii) a MS2 phage operator stem-loop and an MS2 coat protein (MCP) binding domain sequence therefor (iii) a PP7 phage operator stem-loop sequence and a PP7 phage coat protein (PCP) binding domain sequence therefor (iv) a phage Com RNA scaffold sequence and a phage Com binding polypeptide therefor (v) a telomerase RNA Ku binding motif and a Ku protein or RNA-binding section thereof and (vi) a telomerase RNA Sm7 binding motif and a Sm7 protein or RNA binding section thereof. However, a non-natural RNA aptamer or scaffold may be preferred as the recognition element of the targeting RNA which binds to a synthetic binding domain of the modular polypeptide component. Thus, any of the above noted naturally-occurring RNA scaffold-RSBD pairs may be substituted by a variant in which either or both members of the pair is a mutant sequence provided the required binding affinity is maintained. A phage display peptide library may again be employed to attain a peptide-RNA scaffold binding pair or alternative panning of a peptide library for identification of appropriate peptide binding activity for an RNA scaffold, e.g. an RNA structure comprising one or more than one hairpins. The length of the RNA scaffold should preferably be between about 15 and 25 nucleotides, e.g. 19-20 nucleotides as exemplified by the lambda Box B sequence. The RNA scaffold-RSBD pair will have multiple contact points to provide high affinity interaction and with a view to reducing off-target non-specific interaction of the scaffold sequence with any host genomic sequences.

It may be chosen for a targeting nucleic acid or an RNA scaffold element thereof and /or connector sequence to incorporate one or more modified nucleotides and/or one or more modified internucleotide linkages. Modified nucleotides for this purpose may include, for example, 2'-O-methyl analogs, 2'-fluoro analogs or 2' deoxy analogs. The use of locked nucleic acid (LNA) monomers may be contemplated in which the 2' hydroxyl group is linked to the 4' carbon atom of the sugar ring forming a 2'C, 4'-C-oxymethylene linkage and thereby providing a bicyclic sugar moiety . Modified bases such as 2-aminopurine, 5-bromouridine, 5-methylcytidine and 5-methoxyuridine may be employed. One or more internucleotide linkages may for example be a phosphorothioate linkage. Such methods for modifying RNA oligonucleotides to aid stability and required activity in a cellular environment are well-known and any such methods may find use in designing a targeting nucleic acid for use in accordance with the invention. It will be appreciated that such RNA oligonucleotide modification may be contemplated where chemical synthesis of the targeting nucleic acid can be carried out for RNP delivery of a nucleoprotein complex.

By way of specific examples of modified wild-type phage aptamers which retain binding affinity for their cognate binding protein and may be employed in a targeting nucleic acid,
reference may be made to the MS2 stem-loop variants taught as RNA stem-loop motifs in WO2022/011232 (Horizon Discovery Limited and Dharmacon, Inc; published 13 January 2022), for example the MS2 motif variant in which A is changed to 2'-deoxy- 2-aminopurine or 2'-ribose-2- aminopurine at position 10 from the 5'-terminus of the stem-loop (the F-5 variant substitution a shown in Figure 2B of the same published WO application).

In some instances, it may be desired to provide two or more RNA scaffolds on a targeting RNA (either the same or different) to enhance the efficiency of the system or to facilitate the interaction with more than one protein via a RSBD, e.g. two different RNA scaffolds in tandem separated by a connector sequence. In this way, a single targeting RNA may interact with more than one modular polypeptide component for a nucleoprotein complex of the invention, which may be the same or different. Such a targeting RNA with more the one RNA scaffold may also be used to recruit another effector component, e.g. a base editor, joined to an appropriate RSBD.

For the purpose of initial proof of concept studies reported herein, as noted above, a Box B RNA motif was chosen as the targeting RNA scaffold with the modular polypeptide component providing its cognate binding peptide, Lambda N22 (MDAQTRRRERRAEKQAQWKAAN SEQ. ID. No:2). (See Baron- Benhamou et al. (2004) Methods Mol. Biol. 257, 135-154, 'Using the lambdaN peptide to tether proteins to RNAs'.)

Whatever the chosen recognition element of the targeting nucleic acid, a short connector sequence, e.g. of 2, 3, 4, 5 or 6 nucleotides, will generally, if not always, be provided between the targeting element and the recognition element to facilitate correct binding by both to their complementary sequence and binding domain respectively. The RNA scaffold can be positioned in the 5' or 3' end of the nucleic acid component. Thus, in the example given below an RNA nucleic acid component was fused to the 5' terminal of the following sequence in which the short connector AATTT is fused to the BoxB RNA scaffold sequence shown in bold :

### AATTTGGGCCCTGAAGAAGGGCCC (SEQ. ID. No. 9)

It will be appreciated that the connector in this sequence may be substituted by an alternative sequence provided it permits correct functioning of both the joined targeting element (TE) and RNA scaffold (RS). Also either the TE or RS may be the 5'-terminal sequence.

The TE in a targeting nucleic acid for a gene-editing tool of the invention may be designed to bind to either strand of a dsDNA. See Figure 6.

It will be appreciated that the complete modular polypeptide component must also be designed such that each module has the ability to perform its desired purpose and hence one or two inter-module linkers may be required. Thus a linker may be provided between the selected DBD and one or both of the effector component and recognition domain for tethering the targeting nucleic acid - one or both of the distinct linker peptides shown as linkers 1 and 2 in any of Figures 1, 2 and 6. Suitable linkers for this purpose are well-known which do not provide secondary structure or unwanted domain interaction; see for example Chen et al. (2013) Adv. Drug Deliv. Rev. 65, 1357-1369, 'Fusion Protein Linkers: Property, Design and Functionality'. In general flexible linkers are preferred, for example, composed of a stretch of glycine and serine residues or an XTEN peptide linker (Komor et al, "Programmable Editing of a Target Base in Genomic DNA without Double-Stranded DNA Cleavage." Nature 533 (7603) p.420-424). Suitable linkers may be 2 to 15 or more amino acids in length, e.g. (Gly)n or (GGGGS)n. See also linker 1a as employed in the studies of Example 8. In some instances, a flexible linker far longer than 15 amino acids may be preferred, e.g. up to 35 amino acids as exemplified by the linker in the ApGet of SEQ. ID. No. 1 linking the RSBD and DBD - (GGGGS)₇. See also the shortened version of this linker employed in Example 8 (GGGGs)₆. The length of any such linker may preferably be minimised with a view to the desire for the complete length of the modular polypeptide component to be as low as possible, e.g. preferably no more than 220-250 amino acids, for example when including an artificial nuclease as described herein. However, longer modular polypeptide components may be required in some instances, e.g. 300 amino acids in length or longer, for example. if using a different effector component.

The effector component as hereinbefore indicated may be any of a diverse range of moieties for use in modifying DNA (either modifying structure or regulation) including (i) an endonuclease for producing double-stranded breaks, e.g. a Fok 1 nuclease domain (ii) a nickase (iii) a transcription activator such as VP64 (iv) a transcriptional repressor (v) an epigenetic modulator enzyme (vii) a recombinase (viii) a transposase (ix) an integrase and (x) a nucleobase modifying enzyme construct, including for example a nickase such as an artificial nickase as herein described with a base editor.

As indicated above, while it may be preferred that the effector component has no DNA binding ability, it is not excluded that the effector component has some DNA binding ability provided this does not prevent site-specific modification directed by the targeting element and DBD. It is known for example to fuse a Cas9 protein with a transposase or HIV integrase for site specific insertion of an exogeneous nucleic acid into a genome. A transposase or integrase may be similarly employed as the effector component in a modular polypeptide component for genome editing as now described with site-specific insertion of a co-supplied exogeneous nucleic acid being directed by hybridisation of the targeting element to its complementary sequence facilitated by the binding of the DBD.

However, as noted above, especially preferred is provision of an artificial nickase as now further described below. The effector component may be a fusion construct for example in which a nickase, including possibly an artificial nuclease as herein described, is provided together with a base editor or reverse transcriptase.

### Artificial nuclease

There is now provided an artificial nuclease comprising a multimer of linked, self-assembling peptides forming a beta sheet structure wherein:
(i) the self-assembling peptides of said multimer each present an alternating pattern of hydrophobic and hydrophilic residues with all or at least a proportion, of the hydrophilic residues of each such peptide representing a catalytic triad of amino acids or more than three catalytic amino acids whereby said multimer is capable of cleaving a dsDNA either on both strands or on a single-strand, i.e. exhibits nickase activity;
(ii) the self-assembling peptides are joined by linkers to limit the multimer number, e.g. to no more than 20, e.g. 4-15 or 16, preferably to no more than 10, more preferably 5-10 and
(iii) N-terminal and C-terminal flanking sequences are provided linked to the multimer to prevent or limit aggregation between multimers of the nuclease protein with increased positive charge compared to the peptide units of the multimer due to inclusion of one or more positively charged residues, e.g. by inclusion of one or more lysine or arginine residues. Other means of capping the multimer at the N- and /or C-terminal may be employed to aid performance, e.g. by aiding solubility and /or stability.

By beta sheet structure will be understood as desirably an amyloid-like beta sheet structure. The self-assembling peptides may, for example, be chosen and linked with a view to attaining an anti-parallel beta sheet. The linkers between each such peptides can have the same or different lengths. Thus the linker length may be 2 to 25 amino acids, e.g. a 4, 5, 6, 7, 8, 9, 10, 11 or 12 mer linker may be chosen. Short linkers suitable for providing beta turns or beta turn-like loops, e.g. 4 mers, may be designed based on knowledge of the amino acid composition of such turns in native proteins as further discussed and exemplified below.

Appropriate self-assembling peptides (alternatively referred to as folding peptides as in a single folding structure) will form a structure which can be identified by thioflavin T (Tht) binding. Fluorescence emission assay of Thioflavin T binding is commonly employed to detect amyloid fibrils. Upon binding to amyloid fibrils, ThT gives a strong fluorescence signal at approximately 482-485 nm when excited at 450 nm (Xue et al. (2017) Royal . Soc. Open Sci. 4: 160696, 'Thioflavin T as an amyloid dye: fibril quantification, optimal concentration and effect on aggregation).

The C-terminal flanking sequence may preferably also provide a flexible, tail sequence at its C-terminus to aid solubility, e.g. the 4 mer NQGS [SEQ. ID. No. 10] or a longer sequence.

For production as a separate enzyme entity or as part of a fusion construct, such a polypeptide comprising an artificial nuclease may also desirably be produced with an N-terminal tag to aid purification and /or solubility, e.g. a His tag, e.g. a hexa-His tag, to aid purification or a tag comprising one or more His tags and/ or a sequence to aid solubility such as maltose binding protein (MBP). Preferably such a tag will be a cleavable tag, for example, chosen from SUMO or TEV tags. Thus, a suitable tag may comprise 6x His linked to MBP with a further C-terminal 6x His tag separated by a short spacer from a TEV protease cleavage site; see Figure 10.

For inclusion in a synthetic genome-editing tool of the invention as the effector component, an artificial nickase as disclosed herein is preferred. Described herein are polypeptides comprising or consisting of an artificial nuclease as now taught.

It will be appreciated that the self-assembling peptides of said multimer may be identical as in the artificial nuclease of SEQ. ID. No.6 as discussed above, although it may be chosen to use two or more non-identical peptides of the same length, e.g. 7 mers. Preferably, the alternating hydrophobic residues of the self-assembling peptides may be leucine and /or isoleucine, most preferably isoleucine, starting with the N-terminal residue. The hydrophobic amino acid residues may, however, also be selected from any hydrophobic amino acid residues including for example valine. The length may be, for example, a 6 mer to 15 mer, preferably a 7 mer to 11 mer, e.g. a 7 mer or 9 mer, although a 7 mer is preferred, preferably a 7mer with alternating isoleucine residues starting with the N-terminal residue.

It will be appreciated that the peptides of the multimer may be continuously linked in the N-terminal to C-terminal direction or alternate peptides may be reversed throughout or in part and for convenience will preferably be identical. Thus, a multimer formed of 7mer self-assembling peptides may be represented as:
(i) non-inverted multimer: (H-C₁-H-C₂-H-C₃-H-(X)ₙ-H-C₁-H-C₂-H-C₃-H)ₙ
   or
(ii) inverted multimer: (H-C₁-H-C₂-H-C₃-H-(X)ₙ-H-C₃-H-C₂-H-C₁-H)ₙ

where H = a hydrophobic residue, preferably leucine or isoleucine, most preferably isoleucine,
C₁, C₂ and C₃ are each a different catalytic amino acid and
X represents an amino acid linker which may be varied, e.g. any of a 4, 5, 6, 7, 8, 9, 10, 11 or 12 mer, preferably a 4 mer, designed with the aim of providing a beta turn or beta turn like loop, e.g. NDGG, DSSG, SSGS, NNGN, GSDG, GNSG, DNGG, DSDG, SSSG, GSEG, GDSG (SEQ. ID. Nos. 11-21) and other linkers consisting of a combination of amino acids selected from glycine (G), the polar amino acids serine (S), asparagine (N), glutamine (Q) and threonine (T) and the charged amino acids glutamic acid (E) and aspartic acid (D). Such linkers may be employed with any suitable self-assembling peptides to control the multimer size and with a view to aiding stabilization and solubility of the desired beta structure design. Possible options will be readily discernible by those skilled in the field of protein structure analysis and design having regard to the exemplification provided.

Preferably, all the self-assembling peptides will be linked in the N- to C-terminal direction. In this way an assembly of consecutive monomer units may be provided in an anti-parallel beta sheet structure as further discussed below. See Figures 8 and 9.

C₁, C₂, and C₃ may correspond to a trio of different amino acids of a naturally-occurring nuclease known to be capable of nicking a single-strand of a dsDNA, e.g. a known catalytic triad of such a nuclease. Preferably, the chosen catalytic amino acids may correspond to the catalytic amino acids of a RuvC nuclease domain as present in an endonuclease, for example, a Cas9 nuclease, i.e. glutamate (E), aspartate (D) and histidine (H).

A RuvC nuclease domain as present in a Cas9 nuclease has been reported to have 4 catalytic amino acids - His983, Asp986, Asp10 and Glu762. Mutation of any of these catalytic amino acids has been shown to result in loss of function (Nishimasu et al. (2014) Cell 156, 935-949). However, it has been shown that in the context of a multimer as above it is possible for simplicity to employ identical peptide units with alternating hydrophobic residues and, for example, just a single His, Asp and Glu whereby the peptide units maintain propensity to form a stable β-sheet like supramolecular structure while all required catalytic amino acid residues are brought into proximity for a functioning nickase.

Thus by way of example, a preferred 7 amino acid peptide for linkage as above is IEIDIHI. This peptide could also be made of different length of amino acids, anywhere between 5 to 11. Such a peptide may be optionally fused in the multimer structure to a linker sequence providing an aspartate (D residue) as indicated above. D residues may be incorporated into the beta turn designs with a view to potentially increasing the solubility of the complete nuclease.

As noted above, a multimer of identical self-assembling peptides will be flanked by N-terminal and C-terminal sequences with increased positive charge to reduce propensity for aggregation. This may be by substitution of at least one hydrophobic residue in peptides otherwise identical to the self-assembling peptide for the multimer by a positively charged residue, e.g. preferably substitution of a hydrophobic residue by a lysine. For example where the multimer is formed of 7 mer units of alternating hydrophobic and catalytic residues starting with leucine or isoleucine, preferably for example IEIDIHI 7 mer units, such a C-terminal and N-terminal unit may have an identical sequence except for a positively charged amino acid substitution for the isoleucine at position 3 and/or 5 from the N-terminus, e.g. a lysine (K) residue. Thus the N-terminal flanking sequence may be IEKDIHI (SEQ. ID. No. 22) or IEIDKHI (SEQ. ID. No. 23) fused to a 4 mer linking sequence for linkage to the first 7 mer peptide unit of the multimer of identical peptide units. The C-terminal flanking sequence may be selected from the same sequences with an additional C-terminal tail sequence. Thus, the N-terminal flanking sequence may be IEKDIHI and the C-terminal flanking sequence may be IEIDKHI or vice versa. One or both lysine residues may be an alternative positively charged residue e.g. arginine. In this way there will be repulsion of nuclease assemblies when for example such flanking units are linked to a multimer of 10 identical peptides. As indicated above, the C-terminal tail sequence may be chosen to aid solubility, e.g. NQGS.

By way of exemplification of such a functional nickase formed entirely from linked synthetic peptides the amino acid sequence of SEQ. ID. No. 6 is provided which can alternatively be represented as individual peptide units all in the N- to C-terminal direction as follows: 
I E K D I H I N D G G
I E I D I H I D S G G
I E I D I H I S S G S
I E I D I H I N N G N
I E I D I H I G S D G
I E I D I H I G N S G
I E I D I H I D N G G
I E I D I H I D S D G
I E I D I H I S S S G
I E I D I H I G S E G
I E I D I H I G D S G
I E I D K H I N Q G S
(SEQ. ID. Nos. 24-35)
 where the ten identical self-assembling 7mer peptides (peptide IbD with hydrophobic isoleucine residues) are shown in bold.

Since these peptides are all linked in the N-terminal to C-terminal direction by a 4 mer linker aimed at providing a beta turn, it will be appreciated the consecutive peptide monomers will be orientated in opposite directions in an anti-parallel beta sheet structure as follows:

I E I D I H I
I H I D I E I
I E I D I H I (SEQ. ID. No. 36).

It will be appreciated that one or more of the beta turns may be varied and /or the C-terminal NQGS sequence with maintenance of the desired nuclease activity.

### Substitution of the artificial nuclease of SEQ. ID. No. 1

It will be recognised that the artificial nuclease as provided in SEQ. ID. No.1 may be substituted by any alternative effector component desired for modification of a target dsDNA site, either with maintenance or change of the linker to the DBD. By way of exemplification, such a functional synthetic genome editing tool as now taught has the artificial nuclease of SEQ. ID. No.1 substituted by a Fok 1 nuclease domain (referred to herein as an ApGet-Fok1 construct). It will be appreciated that any ApGet-i comprising a nucleic acid recognition module linked to a DBD may be additionally linked to a Fok1 nuclease domain. Such a modular polypeptide construct may for example be used with a pair of targeting nucleic acids with differing TEs to provide a functional Fok1 nuclease capable of cutting dsDNA at a target site. One configuration for this is illustrated in Figure 18. See also the exemplification below employing the 'Editor' plasmid illustrated in Figure 14 for expression of an ApGet-Fok1 construct to produce a double strand break in a detector plasmid. It will be recognised that the DBDs and targeting elements may be varied to produce a double-strand break at different dsDNA target sites. However, with a view to minimising size for expression of a complete modular polypeptide component of a gene-editing tool as now taught, it is preferred to substitute the Fok1 nuclease domain by an artificial nickase described herein.

By way of further exemplification, the artificial nuclease as provided in SEQ. ID. No. 1 may be substituted by a transcription activator, e.g. VP 64, and the resulting modular polypeptide combined with one or more targeting nucleic acids designed to target a promoter location and thereby activate or enhance transcription of the one or more coding sequences in operational linkage with the promoter; see Example 8. Such effector component substitution may be accompanied by change of one or more of the RSBD, the DBD, the linker between the RSBD and DBD (Linker 2, L2,as shown in Figure 1) and the linker between the effector component and DBD (linker L1, L1, as shown in Figure 1) provided required function of the RSBD, DBD and effector component is maintained. Thus, for example, as illustrated by Example 8, the artificial nuclease of SEQ. ID. NO. 1 may be substituted by the VP64 polypeptide with shortening of linker 2 (see linker 2b of SEQ. ID. No. 70) or change of linker 1 (see linker 1a of SEQ. ID. No. 69).

### Further aspects

It will be appreciated that an artificial nickase described herein has use beyond inclusion in a complete synthetic gene-editing tool as discussed above. It may be employed as part of a fusion enzyme in fusion with another entity for DNA modification, e.g. a dCas9, possibly also linked to a further component for DNA modification such as a base editor or reverse transcriptase. It may be expressed from a polynucleotide, e. g. expression vector either as part of a fusion protein, e.g. a modular polypeptide component of a DNA modifying tool for use with a targeting nucleic acid, or as a separate protein.

Polynucleotides capable of expressing a polypeptide of the invention, e.g. comprising an artificial nuclease or nickase described herein and host cells transformed by such polynucleotides are also described herein.

Also provided is a polynucleotide encoding an artificial nuclease or nickase protein described herein with an N-terminal tag sequence to aid purification and solubility as discussed above, e.g. such a tag sequence which is a cleavable tag sequence providing both a His tag and tag to aid solubility, e.g. Maltose Binding Protein (MBP) sequence. Such a polynucleotide may be an expression cassette for production of the nuclease or nickase protein in an *in vitro* transcription and translation system or an expression vector for expression of such a nuclease protein in a host cell, e.g. a bacterial cell such as an *E.coli* cell or yeast cell. A host cell thus transformed represents a further aspect of the disclosure.

Additionally provided is a method of producing a polypeptide comprising an artificial nuclease or nickase described herein which comprises:
(i) culturing a host cell as above or use of an *in vitro* transcription-translation system whereby said polypeptide is produced with an N-terminal tag sequence to aid purification and solubility, desirably with a cleavable tag such as a cleavable His-MBP-His fusion tag;
(ii) isolating the polypeptide using the N- terminal tag and where said N-terminal tag is cleavable,
(iii) cleaving the tag from the polypeptide and
(iv) separating the polypeptide from the tag.

As indicated above, an artificial nuclease described herein, is however especially preferred to be provided as part of a modular polypeptide of an artificial gene editing system of the invention. In this case, the artificial nuclease or nickase will generally be joined to the DBD by a linker sequence such that the DBD can bind to the selected PDS and the nuclease is able to act at the required target site. For expression of such a modular polypeptide, an expression cassette, e.g. vector, will be provided which encodes the whole modular polypeptide. An N-terminal tag may be provided in some instances, e.g. a cleavable His tag (see Figure 16).

Preferred methods are herein now taught for recombinant production of either an ApGet-i or full ApGet modular polypeptide including an artificial nuclease described herein. Thus such a modular polypeptide component, or any modular polypeptide component for use in a nucleoprotein complex of the invention for target sequence modification, may be initially expressed, e.g. in a host cell such as *E. coli,* with a multiple component tag sequence to assist all, or any of, solubility, purification and detection including a protease cleavage site whereby prior to use unwanted tag sequence can be removed. Such a multiple component tag sequence may be linked to an NLS whereby upon protease cleavage an NLS is retained at the N- or C- terminus possibly together with additional sequence which does not interfere with required target modification, e.g. an end sequence including a detection sequence. Such a multiple component sequence tag may desirably provide all of (i) at least one his tag, e.g. at least one hexa-his tag, (ii) a polypeptide sequence to aid solubility, e.g. a MBP or small ubiquitin-like modifier (SUMO) (iii) a protease cleavage site and (iv) a detection sequence, e.g. an epitope tag. By way of example of such a preferred multiple component tag, it has been found beneficial to express an ApGet or ApGet-i in *E. coli* with an N-terminal extension providing in the N- to C-terminal direction all of: (i) a hexa-his tag (ii) a solubility tag of either MBP or SUMO (iii) a linker providing a spacer (iv) a TEV protease cleavage site and (v) an epitope tag such as the FLAG^{®} epitope joined to an NLS. Upon protease cleavage, the final modular polypeptide for target modification will retain both the NLS and epitope tag with a short N-terminus leader sequence, e.g. a 4mer N-terminus leader sequence such as GWGS (See Figure 31). The linker between the solubility tag and TEV protease cleavage site may desirably be an asparagine rich linker rather than a linker adding to requirement for glycine and serine. For expression of ApGet it has been found additionally beneficial to incorporate a Strep-tag^{®} II at the C-terminus to aid separation from prematurely terminated product. It will be appreciated however that a variety of other N- and or C-terminal tags may be employed in initially expressing a modular polypeptide component
to aid solubility and/or purification and/or detection and/or membrane penetration. For example as indicated above, the modular polypeptide component may in some instances be expressed with inclusion of a cell penetrating peptide sequence.

### Use of synthetic gene modifying systems of the invention

As indicated above, a nucleoprotein complex of the invention may be delivered to host cells either as an active complex, e.g. by electroporation, or with one or both of the polypeptide component and targeting nucleic acid component expressed by a polynucleotide.

### Delivery of ApGet components into cells

The required nucleic acid components can be delivered to cells by any of a variety of suitable methods without limitation. Many such methods are known. Thus, direct introduction of synthetic RNA molecules to the cell of interest may be by electroporation, nucleofection, transfection, via nanoparticles, via viral mediated RNA delivery, via non-viral mediated delivery, via extracellular vesicles (for example exosome and microvesicles), via eukaryotic cell transfer (for example by recombinant yeast ) and other methods that can package the nucleic acid molecules and provide delivery to the target viable cell. Other methods for the introduction of RNA molecules include non-integrative transient transfer of DNA polynucleotides whereby the relevant sequence is transcribed intracellularly. This includes, without limitation, by employing DNA-only vehicles (for example, plasmids, MiniCircles, MiniVectors, MiniStrings, protelomerase generated DNA molecules (for example Doggybones), artificial chromosome (for example HAC), cosmids) or use of vehicles such as nanoparticles, extracellular vesicles (for example exosomes and microvesicles), via eukaryotic cell transfer (for example by recombinant yeast), transient viral transfer by AAV, non-integrating viral particles (for example lentivirus and retrovirus based systems), cell penetrating peptides and other technology that can mediate the introduction of DNA into a cell without direct integration into the genomic landscape. Another method for the introduction of the RNA components include the use of integrative gene transfer technology for stable introduction of the machinery for RNA transcription into the genome of the target cells. This can be controlled via constitutive or promoter inducible systems to attenuate RNA expression. Such technology for stable gene transfer includes, but not limited to, integrating viral particles (for example lentivirus, adenovirus and retrovirus based systems), transposase mediate transfer (for example Sleeping Beauty and Piggybac), and other technology that encourages integration of the target DNA into a cell of interest.

Delivery of the protein or peptidergic components, such as the artificial nickase, of the system, can be carried out by the same technology but in some situations, there are advantages to mediate the delivery via different methods. Such applicable methods, and not limited to, are listed below. Firstly, the direct introduction of protein molecules to the cell of interest by electroporation, nucleofection, transfection, via nanoparticles, via viral mediated packaged delivery, extracellular vesicles (for example exosome and microvesicles), via eukaryotic cell transfer (for example by recombinant yeast), and other methods that can package the macromolecules for delivery to the target viable cell without integration into genomic landscape. Other methods for the introduction of protein molecules include non-integrative transient transfer of DNA polynucleotides that include the relevant sequence for transcription and translation to provide the required intracellular protein molecule. Again, this includes, without limitation, possible use of DNA-only vehicles (for example, plasmids, MiniCircles, MiniVectors, MiniStrings, protelomerase generated DNA molecules (for example Doggybones), artificial chromosome (for example HAC), cosmids), or DNA- carrying vehicles such as nanoparticles, extracellular vesicles (for example exosome and microvesicles), via eukaryotic cell transfer (for example by recombinant yeast), transient viral transfer by AAV, non-integrating viral particles (for example lentivirus and retrovirus based systems), and other technology that can mediate the introduction of DNA into a cell without direct integration into the genomic landscape. Another method for the introduction of the protein component(s) includes the use of integrative gene transfer technology for stable introduction of the machinery for transcription and translation into the genome of the target cells. Many such methods are well-known in the gene modification field. Control can again be via constitutive or inducible promoter systems. The design may be so that the system can be removed after the utility has been met (for example, introducing a Cre-Lox recombination system). Such technology for stable gene transfer includes, but not limited to, integrating viral particles (for example lentivirus, adenovirus and retrovirus based systems), transposase mediate transfer (for example Sleeping Beauty and Piggybac), and other technology that encourages integration of the target DNA into a cell of interest.

### Expression systems

As indicated above, it may be desirable to express one or more of any protein component and/or RNA component from an encoding polynucleotide. This can be performed in a variety of ways.

For example, one or more intermediate vectors may be employed for introduction into prokaryotic or eukaryotic cells and suitable for replication and/or transcription to express the required component(s). Expression vectors may be employed for administration to the chosen host cell, for example a plant cell, an animal cell, e.g. a bird, mammalian cell or a human cell, a fungal cell, a bacterial cell, or a protozoan cell. Accordingly, the present invention provides nucleic acids that encode any of the RNA components or proteins
as mentioned above. Preferably, the nucleic acids are isolated and/or purified.

Examples of expression constructs of use in application of a nucleoprotein complex of the invention include a vector, such as a plasmid or viral vector, into which a nucleic acid sequence of the invention has been inserted, in a forward or reverse orientation. In a preferred embodiment, the construct further includes regulatory sequences, including a promoter, operably linked to the sequence. Large numbers of suitable vectors and promoters are known to those of skill in the art, and commercially available.

Examples of expression vectors of use in application of nucleoprotein complexes of the invention include chromosomal, non-chromosomal and synthetic DNA sequences, bacterial plasmids, phage DNA, baculovirus, yeast plasmids, vectors derived from combinations of plasmids and phage DNA, viral DNA such as vaccinia, adenovirus, fowl pox virus, and pseudorabies.

The vector may include appropriate sequences for amplifying expression. In addition, the expression vector preferably contains one or more selectable marker genes to provide a phenotypic trait for selection of transformed host cells such as dihydrofolate reductase or neomycin resistance for eukaryotic cell cultures, or such as tetracycline or ampicillin resistance in *E*. *coli.*

A vector or vectors for use in application of a nucleoprotein complex of the invention, e.g. to express the modular polypeptide component(s) and/ or one or more targeting RNAs intracellularly or production of a protein component of the invention can be designed with appropriate control sequences for such expression or production in the chosen host cell. Examples of suitable expression hosts include bacterial cells (e.g., *E*. *coli, Streptomyces, Salmonella typhimurium),* fungal cells (yeast), insect cells (e.g., *Drosophila* and *Spodoptera frugiperda* (Sf9)), animal cells (e.g., CHO, COS, and HEK 293), adenoviruses, and plant cells. The selection of an appropriate host for production of protein
is within the scope of those skilled in the art.

### Applications

Thus, in a further aspect, there is provided a nucleic acid or combination of nucleic acids for provision of one or more nucleoprotein complexes of the invention in a host cell. However, direct delivery of an active complex of the invention to host cells (a protein component plus targeting RNA) is not excluded. Such host cells may be prokaryotic or eukaryotic cells. They may for example be any of bacterial cells, yeast cells, insect cells, and fungal cells and eukaryotic cells including plant cells and human and non-human animal cells, such as, for example, hamster (e.g. CHO cells), monkey (e.g. vero cells), rat, mice, bird or chicken cells. Suitable host cells will be understood to include *ex-vivo* cells e.g. *ex- vivo* stem cells, induced pluripotent stem cells (iPSCs) and *ex-vivo* T cells including engineered CAR-T cells.

The nucleic acid may preferably be a vector, e.g. a viral vector such as a recombinant adeno-associated viral (rAAV) vector, lentivirus , retrovirus ,adenovirus or Sendai virus vector capable of expressing both at least one modular polypeptide component ,
e.g. including an artificial nickase described herein, and at least one targeting nucleic acid component. More than one targeting nucleic acid component may be provided to a host cell to target more than one DNA site. In this case, each targeting nucleic acid component may interact with a single modular polypeptide component, i.e. more than one targeting nucleic acid, e.g. a pair, will be combined in action with a single DBD which sees a single PDS. However, each targeting nucleic acid component of a pair may interact with a different modular polypeptide component each possessing a DBD which binds a different PDS. Whichever of these modes of delivery is chosen, the DNA target sites for the targeting nucleic acid components may be external to the pair of PDS sites (referred to as the 'PDS-in' configuration) or the DNA target sites for the targeting nucleic acid components may be internal to the pair of PDS sites (referred to as the 'PDS-out' configuration'). Such configurations are illustrated in Figures 18 and 19 with each targeting nucleic acid targeting a different DNA strand of a dsDNA.

It will be recognised that a pair of nucleoprotein complexes of the invention for use as above may include as the effector component a Fok1 nuclease domain joined to the DBD by a suitable linker sequence, i.e. will represent an ApGet-i construct joined by a linker to the catalytic domain of a Fok1 endonuclease (referred to herein as a ApGet-Fok1 modular polypeptide). Such a pair of complexes may operate in the PDS-in or PDS-out configuration to produce a double-stranded break in a dsDNA, e.g. a genomic DNA for gene knock out. See Figure 18 which illustrates a pair of ApGet-Fok1 modular polypeptides operating in the PDS-in configuration for this purpose.

Especially preferred is one or more vectors, preferably a single vector, for vector delivery of a pair of nucleoprotein complexes of the invention providing an artificial nickase to a cell to cut both strands of a dsDNA. Such delivery may also be able to achieve gene knock out by non-homologous end joining. It will be recognised however that such nucleoprotein complex delivery may be accompanied with provision of a sequence template for homologous recombination. This sequence template may be provided by an entirely separate expression construct or by expression from a separate expression cassette on the same vector. Figure 16 illustrates single vector constructs able to express all of a modular polypeptide including an artificial nuclease, a targeting nucleic acid component for interacting with the modular polypeptide and a sequence for homology repair of an enzyme-coding sequence upon cutting of a pair of target sites.

As a further aspect of the invention, there is provided a method for modifying one or more target nucleic acid sequences employing one or more nucleoprotein complexes of the invention or one or more nucleic acids for provision thereof in a host cell with the proviso that such methods as claimed do not extend to methods of modifying the germ line identity of a human being or methods of medical treatment practised on the human or animal body as such. As indicated above, desirably, a single polypeptide component for provision of the one or more nucleoprotein complexes may be provided in cells by expression from a vector. The same vector may preferably also express the required one or more targeting nucleic acids, preferably one or more targeting RNAs.

As a still further aspect of the invention there is provided a combination of (i) at least one polypeptide component for a genome modification tool of the invention, or a polynucleotide capable of expressing the same and (ii) one or more targeting nucleic acids which can interact or link with said polypeptide component(s), or one or more polynucleotides capable of expressing the same, for use in a method as above, or for use in therapeutic treatment. Such a combination may be provided in the form of a kit for one or more specific applications, e.g. a single polypeptide component or polynucleotide encoding the same may be provided with one or more targeting nucleic acids, or one or more polynucleotides designed to target one or more desired nucleic acid locations. However, as indicated above both the polypeptide component and the one or more targeting nucleic acids, preferably targeting RNAs, may preferably be expressed from a single vector.

It will be appreciated that by way of example the invention can be utilised to edit cells utilised in bioproduction, vaccine production, research tools and reagents, production of genetically-modified animals and plants and cell therapy. Components of the invention can be utilised for gene therapy applications.

Nucleoprotein complexes of the invention may, for example be utilized in the provision of cells for use in therapy. One such application is generating therapeutic cells such as T cells engineered to express a Chimeric Antigen Receptor (CAR-T) or T Cell Receptor (TCR). The CAR-T/TCR cells may be derived from primary T cells or differentiated from stem cells. Suitable stem cells include, but are not limited to, mammalian stem cells such as human stem cells, including, but not limited to, hematopoietic, neural, embryonic, induced pluripotent stem cells (iPSC), mesenchymal, mesodermal, liver, pancreatic, muscle, and retinal stem cells. Other stems cells include, but are not limited to, mammalian stem cells such as mouse stem cells, e.g., mouse embryonic stem cells.

Nucleoprotein complexes of the invention may be used to knockout, modify or increase the expression of a single gene or multiple genes in various types of cells or cell lines, including but not limited to cells from mammals. Nucleoprotein complexes of the invention may be applicable to multiplex genetic modification, which, as in known in the art, involves genetically modifying multiple genes or multiple targets within the same gene. The technology may be used for many applications, including but not limited to knock out of genes to prevent graft versus host disease by making non-host cells non-immunogenic to the host or prevent host vs graft disease by making non-host cells resistant to attack by the host. These approaches are also relevant to generating allogenic (off-the-shelf) or autologous (patient specific) cell-based therapeutics. Target genes may include, but are not limited to, the T Cell Receptor, the major histocompatibility complex (MHC class I and class II) genes, including B2M, and genes involved in the innate immune response.

As indicated above and by way of summary, it will be recognised that a DNA modification system as now taught may be delivered into cells in various ways well-known to those skilled in cell transformation including:
1. Introduction of ribonucleoprotein particles
2. mRNA
3. Plasmids expressing a targeting RNA component and the modular polypeptide component
4. Virus-like particles containing any as noted in 1, 2 or 3 above
5. Lipid nanoparticles containing as noted in 1, 2 or 3 above.
6. Exosomes containing any as noted in 1, 2 or 3 above.
7. Liposomes containing any as noted in 1, 2 or 3
8. Viral vectors, e.g. rAAV vectors, expressing a targeting RNA component and the modular polypeptide component.

As noted above, it is envisaged that a synthetic genome-editing tool as now taught including an artificial nuclease formed from linked synthetic peptide units may advantageously permit genome-editing for gene knock-out, or gene modification by homologous recombination, employing just a single vector for delivery of the required expression cassettes, e.g. a vector capable of expressing all of an ApGet modular polypeptide and at least one targeting RNA and possibly additionally a template sequence for homologous recombination between a pair of cut sites.

The following non-limiting examples are provided to illustrate the invention and further describe the derivation and testing of the nickase in the modular polypeptide of SEQ. ID. No. 1 by way of proof of concept of an artificial nickase which may be preferably employed as an element of a synthetic genome editing system of the invention .

### Examples

### Example 1: Selection of a DNA-binding domain

As noted above, a commercially available M13 phage display library was employed (New England Biolabs, catalogue #E8111L) which contains around 1 x 10⁹ 12mer peptides fused to the N-terminus of the phage minor coat protein III. Two different 6 nt dsDNA baits were employed with a biotin tag immobilized on a streptavidin-coated 96 well plate as specified in Table 1 below:

**Table 1. Oligonucleotides that anneal to form biotinylated double-stranded DNA baits of PDS1 (GAGGTC) and PDS2 (ACGGGT).**

| Designation of oligo | Oligo sequence (5' to 3' direction)+modifications |
|---|---|
| 1 | GAGGTC - 3' TEG biotin |
| 2 | GACCTC |
| 3 | ACGGGT - 3' TEG biotin |
| 4 | ACCCGT |

ELISA assays were used to assess binding of peptides of the phage display library to the PDS1 and PDS2 dsDNA baits using an anti-M13 phage antibody labelled with horseradish peroxidase (HRP). After three rounds of bio-panning and sequence analysis of the bound peptides in the library, two 12 mer peptide sequences were selected, chemically synthesised, and modified as set out in Table 2 below.

Further details of the biopanning and selection of a DBD are set out below.

### Biopanning and selection of a DBD

1. **Blocking step** of the streptavidin coated 96 well plate (Fisher Scientific, UK) with the Blocking Buffer (0.1 M NaHCO₃ (pH 8.6), 5 mg/ml BSA, filter sterilised and stored at 4° C).
2. 6 rounds of wash with 1x TBST after discarding the blocking solution.
3. **Precomplexing step** (this step can be done at the same time as the blocking step, mentioned above), where the amplified phage library was incubated with each of the dsDNA baits in separate reactions. Each bait includes annealed double stranded DNA oligos (dsDNA), containing one of the PDS sites. In the example now provided, the forward strand in each of the dsDNA oligos, mentioned above was 3' end labelled with TEG-biotin (Sigma/Merck). The single strand DNA oligonucleotide sequences used to generate the dsDNA PDS containing baits at this step are represented in the Table 1 above. The length of PDS should be kept as short as possible (so as to allow reliable oligonucleotide synthesis and stable base-pairing to form dsDNA). Conditions of the precomplexing step: 5micL phage library (10¹¹ pfu) incubated with 50nM dsDNA bait (final concentration) in 200µl TBST (total volume of the binding reaction).
**4. Immobilization step.** Precomplexed library was immobilized on streptavidin-coated 96 well plate (Fisher Scientific UK).
**5.** 10 washes with TBST after discarding the unbound phage.
**6. Elution step.** 10-20 mins with 0.2M Glycine-HCI (pH2.2), with the subsequent neutralization (15µM Tris-HCI, pH 9.1 to 100µM of 0.2M Glycine-HCI, pH2.2).
7. **Amplification and titering steps** (for the non-amplified and amplified eluates) were performed before each selection (panning) round, according to the manufacturer's instructions (NEB). In total, 2-3 rounds were done.
8. **Sequence analysis step.** A clone PCR with the subsequent analysis of the Sanger sequences (20 clones for each bait in each tested round) was performed on the "blue" (with functional b-gal) clones grown on the LB-agar plates from the titering analysis (NEB).

Additional modifications of the selection procedure, described above included the following:
1. Introduction of the separate counter-selection round (either before second round or before first round), where the unbound phage library (after step 1) was collected, amplified and tested by titering, before the actual bio-panning round.
2. Introduction of the pre-screening round after the counter-selection round. This was done in the experiments with the baits, that contain PDS followed by an "extended region" sequence, thus allowing for the bio-panning procedure to be performed at 37° C. Pre-screening step was performed with the oligos containing "extended regions only", therefore allowing removal of the phage clones that bind to these extended regions.

Peptide 2 in the phage library had high binding ability for the PDS1 dsDNA bait. Peptide 4 was selected on the basis of binding to the PDS2 dsDNA bait. These peptides are joined in the library to the phage coat protein via a GGS linker. Hence, Peptide 1 and Peptide 3 with this linker at the C-terminus were also provided for further testing.

**Table 2. List of the chemically synthesized peptides used in the test for peptide binding and unwinding of dsDNA targets. The peptides were synthesized with the indicated modifications.**

| **Designation of peptide** | **Amino acid sequence** | **Modification** |
|---|---|---|
| Peptide 1 | SLETWLKHREKDGGS (SEQ. ID. No. 37) | C-terminal amidation, C-terminal GGS linker |
| Peptide 2 | SLETWLKHREKD (SEQ. ID. NO:38) | N-terminal acetylation, C-terminal amidation |
| Peptide 3 | AGDWESWTNGSWGGS (SEQ. ID. NO:39) | C-terminal amidation, C-terminal GGS linker |
| Peptide 4 | AGDWESWTNGSW (SEQ. ID. No: 40) | N-terminal acetylation, C-terminal amidation |

It was reasoned that the phage clones with the selected DNA binding peptides will alter a DNA duplex in a region presenting the cognate PDS, which may result in recognition by DNA nucleases, such as T7E1, commonly used to detect insertions or deletions (indels), which result from the activity of genome editing enzymes, such as Cas9, Cas12a etc. Results of such cleavage assays for destabilized DNA duplex using the extended dsDNA baits as set out in Table 3 below and T7E1 and Surveyor enzymes support this hypothesis (see Figures 3a-c), i.e. that binding of the clones presenting the selected fused peptides to dsDNA destabilizes the structure of the DNA duplex in a manner facilitating accessibility for RNA hybridization. Resultant transient unwinding and formation of a heteroduplex structure can be envisaged as shown in Figure 2.

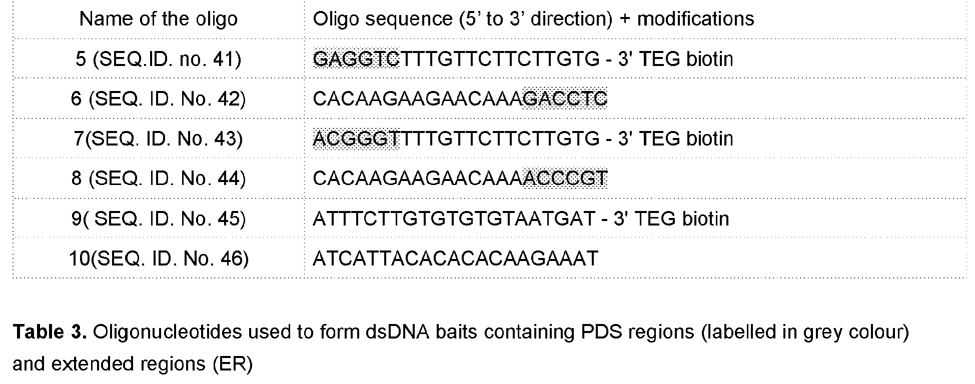

The modified peptides as set out in Table 2 were used to test the sequences for use as DNA-binding domains without M13 phage connection. The modifications included amidation and acetylation at the N- and C termini respectively to reflect natural peptide modifications to improve peptide stability and prevent degradation. In the case of Peptides 1 and 3, an additional C-terminal linker (GGS) was provided as noted above. This also permitted assessment of the binding characteristic to the cognate PDS with presence of a linker permitting linkage to a different non-phage protein.

Results of a cleavage assay using T7E1 enzyme for binding and non-duplex structure formation when presented with an extended dsDNA containing the cognate PDS clearly pointed to the Peptide 1 sequence as the best candidate to be a DNA-binding domain when PDS1 is the chosen predetermined cognate PDS. See Figure 4.

These results were further corroborated by performing fluorescent DNA binding assays. A FRET experiment was carried out to assess the binding and unwinding activity of Peptide 1 with ability to facilitate RNA hybridization when presented with PDS1 in an extended dsDNA target as shown schematically in Figure 5. It was hypothesized that, upon binding, Peptide 1 will cause some transient unwinding of the dsDNA which will facilitate binding of a targeting RNA. The fluorescent donor, 6-carboxyfluorescein (FAM), was used to tag an appropriate targeting RNA with a 15bp complementary sequence. Target DNA was tagged with the fluorescent acceptor Cy5 at a 3' end or Texas Red at a 5'-end. RNA-DNA hybridisation was shown to be facilitated by binding of Peptide 1 to its cognate PDS1 sequence as reflected by the FRET signal - a Cy5 or Texas red fluorescence signal upon FAM excitation (see Figure 5).

In summary, the results from the cleavage and FRET experiments are consistent with the binding of Peptide 1 to its respective PDS in dsDNA causing partial and transient unwinding of the dsDNA and facilitating hybridisation of a complementary RNA. Hence, the Peptide 1 amino acid sequence (SEQ. ID. No. 4) incorporating a C-terminal GGS linker is one example of a favoured polypeptide sequence for use as the DNA-binding domain in a genome editing tool of the invention, e.g. ApGet system. It will be recognized that by similar studies short polypeptide sequences may be found for binding to other desired predetermined dsDNA target sequences of 6 or less than 6 nucleotides. Site-directed mutagenesis may be utilized to further improve binding capability for any desired PDS.

It will be appreciated that Peptide 1 will commonly be provided with at least a second linker for incorporation into a complete required modular polypeptide component including additionally the targeting nucleic acid recognition domain and the effector component. Moreover, it may be desired to extend the incorporated GGS linker. However, this can be achieved with maintenance of the desired binding characteristic for the PDS and confirmed by appropriate further testing. Thus, Peptide 1 as the DBD may be linked to an effector component, e.g. an artificial nuclease, by extension of the C-terminal by a further sequence of G and S residues, e.g. the linker of SEQ. ID. No. 5 [GGGGSGGGGSGGGGGS] as employed in the ApGet of SEQ. ID. No.1.

### Example 2: Provision of an artificial nickase

The first step in the design of an artificial nickase was selection of an ordered polypeptide capable of forming a supra-molecular self-assembled nanostructure. For this purpose, beta sheet forming peptides were chosen of length 7 or 9 amino acids with an alternating pattern of hydrophobic leucine and hydrophilic lysine residues. Such a pattern is known to form a self-assembled nanostructure with propensity for amyloidal structural formation. This pattern was further modified by the substitution of amino acids at hydrophilic positions based on the known catalytic amino acids of naturally occurring nucleases, e.g. an RuvC1 nuclease domain as present in Cas9.

It was reasoned that multiple such beta sheet forming peptides can be joined by linkers or loops to control number as shown below for 7mers where H refers to a hydrophobic amino acid, C refers to a catalytic amino acid and X refers to a connecting linker of length n amino acids. Each of the peptide units may be identical in which case each C may be a different amino acid of a catalytic trio - either a recognised catalytic triad or a trio of different amino acid types providing catalytic function in a known naturally-occurring nuclease such as a RuvC1 nuclease domain. Alternate linked peptide units may be linked N- terminal to C-terminal or with reversion.
(H-C₁-H-C₂-H-C₃-H-(X)ₙ-H-C₁-H-C₂-H-C₃-H)ₙ
   or
(H-C₁-H-C₂-H-C₃-H-(X)ₙ-H-C₃-H-C₂-H-C₁-H)ₙ

Table 4 below sets out the precise peptide sequences of 7 or 9 amino acids which were used in first developing an efficient artificial nickase. The starting sequence (bA) as indicated above was an inactive 7mer of alternating leucine residues and lysine residues. The naturally-occurring nuclease domain for provision at hydrophilic positions of amino acids of a catalytic trio are listed. For example all of peptides bB, bD, iBD, bE, bF had all lysines substituted by the three amino acids corresponding with the catalytic amino acids of a RuvC1 nuclease domain [Glutamic acid (E) Aspartic acid (D) and Histidine (H)] separated by hydrophobic residues.

As noted above, a RuvC1 nuclease domain has been reported to have 4 catalytic amino acids - His983, Asp986, Asp10 and Glu762. Mutation of any of these catalytic amino acids has been shown to result in loss of function (Nishimasu et al. (2014) Cell 156, 935-949). It was reasoned however that it may suffice in the context of a multimer as above to employ for simplicity identical peptide units with alternating hydrophobic residues and, for example, just a single His, Asp and Glu whereby the hydrophobic residues maintain propensity to form a stable β-sheet like supramolecular structure while all required catalytic amino acid residues are brought into proximity for a functioning nuclease. The 9mer Peptide bE was however provided with two Asp residues plus a glutamate and histidine.

In some instances, leucine was also substituted by isoleucine. Thus, bD is a 7mer starting with a leucine and having leucine residues alternating with E, D and H in that order. IbD has the same amino acid pattern except for substitution of leucine by isoleucine (I) as the hydrophobic residues. It is possible that isoleucine residues could aid the formation of β-sheet structure more efficiently than leucine residues as the former are more hydrophobic than the later.

**Table 4**

| | **Amino acid sequence** | | | | | | | | | **Nuclease domain** |
|---|---|---|---|---|---|---|---|---|---|---|
| **Peptide** | | | | | | | | | | |
| bA | L | K | L | K | L | K | L | | | Inactive amyloid |
| bB | L | D | L | E | L | H | L | | | RuvC1 |
| bC | L | D | L | D | L | H | L | | | RuvC1 |
| bD | L | E | L | D | L | H | L | | | RuvC1 |
| ibD | I | E | I | D | I | H | I | | | RuvC1 |
| bE | L | D | L | D | L | E | L | H | L | RuvC1 |
| bF | I | H | I | D | I | E | L | | | RuvC1 |
| bG | I | D | I | E | I | R | I | | | RuvC1 |
| bh | I | H | I | H | I | Q | I | | | Active (NPA) amyloid |
| bI | L | H | L | E | L | N | L | | | HnH and endo VII |
| bJ | L | H | L | E | L | H | L | | | S1/P1 nuclease |
| bK | L | N | L | Y | L | E | L | | | UvrC-N |
| bL | L | D | | D | L | K | L | H | L | Res endonuclease (mut H) |
| bM | I | N | L I | D | I | H | L | | | HnH and endo VII |
| bN | I | H | I | D | I | S | I | | | resolvases |

Peptide IbD and Peptide bD are previously represented above as SEQ. ID. No. 7 and SEQ. ID. No. 8 respectively. The remaining peptides are SEQ. ID. Nos. 47-59.

Such peptides were tested for ability to self-assemble to provide a β-sheet supramolecular structure and nickase activity. Ability to provide a β- sheet supramolecular structure was determined by thioflavin T fluorescence binding (Xue et al. (2017) Royal Soc. Open Sci. 4: 160696). Nickase activity was investigated by means of a molecular beacon assay combined with a gel plasmid cleavage assay.

### Thioflavin T assay

Thioflavin T (ThT) was mixed with the peptides in reaction buffer solution. Peptides were diluted to 200µM (from a stock solution of 4.4mM in DMSO) using a reaction buffer solution (the same buffer as used for the initial molecular beacon assays as set out below). ThT was added to the peptide in the reaction buffer solution at 25µM with pulse vortexing and centrifugation. Plates containing 200µl per cell of the peptide/Tht solution were incubated at 37°C. The emission signal at 485nm was measured every 5 minutes using an excitation wavelength of 450 nm. An increase of emission signal was taken to be indicative of ThT binding to amyloid-like fibrils. For peptide bD, a high emission signal was measured instantly indicating ready self-assembly of peptides to form such fibrils (see Figure 7).

### Nickase activity investigation

A molecular beacon assay was firstly used to screen each of the peptides for DNA binding and cleavage. For the purpose of these studies, the following DNA oligonucleotide containing 33 nucleotides was employed
[6-FAM]CCTCTCCGTGTCTTGTACTTCCCGTCAGAGAGG[BHQ1] (SEQ. ID. No. 60) where the first six nucleotides at each terminus complement one another forming a 6bp double stranded DNA stem. This results in a 23-nucleotide single stranded DNA loop region. The hairpin structure formed contains a fluorescein molecule as a 5' modification (6-FAM) and a fluorescent quencher (BHQ1) as a 3' modification. In the hairpin conformation the fluorescent signal of the molecular beacon is reduced. If a peptide binds or causes cleavage, the hairpin structure will disassemble releasing the fluorophore from the quencher.

The reaction conditions were chosen to reflect conditions that might be expected to strengthen the probability of DNA cleavage by each peptide. As noted above, the peptide designs were inspired by the catalytic active sites of known nucleases. Often the divalent metal of choice for such sites is Mg²⁺. However producing a reaction intermediate with Mg²⁺ ions require stringent coordination geometry and charge requirements and can be substrate specific and highly selective. In contrast Mn²⁺ ions require less coordination sites to facilitate catalysis. Mn²⁺ ions have been used to screen mutated nucleases *in vitro* as such ions tend to exhibit relaxed substrate specificity and can rescue defective enzymes compared to Mg²⁺ ions. Hence, Mn²⁺ ions were used for the initial activity screen of peptides in this instance.

Each reaction was carried out in a reaction buffer of 25mM Tris-HCl (pH 7.5), 130mM NaCl, 27µM KCI and 5mM MnCl₂. The molecular beacon was provided at a concentration of 400nM.

The emission signal of the fluorophore after a 15-minute incubation was measured for each peptide mixed with the molecular beacon at a 500:1 concentration ratio.

Peptides bL and bN gave a relatively high emission signal with such conditions. These peptides were therefore used to further assess variance of the assay conditions as regards Mn²⁺ concentration, metal ion dependency and peptide concentration.

### Metal concentration

The concentration of manganese chloride used in the initial screen was in excess. This was to enhance the probability of forming a reaction intermediate to push the reaction to completion. An excess of metal ions is often employed in *in vitro* reactions. However too much metal can produce an inhibitory effect on the reaction. By employing a range of manganese chloride concentrations from 0-5mM, the optimal manganese chloride concentration for bN peptide was found to be 0.25mM.

### Metal dependency

The reaction with different metal ions showed differences in the signal emitted by the molecular beacon with either bL or bN peptides. The disassembly of the molecular beacon was more extensive in the presence of zinc chloride ions compared to manganese, calcium and magnesium chloride for bL peptide. In contrast the bN peptide showed a high increase in the signal regardless of the metal in the reaction. A high signal was also observed without any metal ions. However, the signal was greater with Mn²⁺ ions. This suggests that by using different peptide designs that contain different active site regions a potentially different mechanism of DNA cleavage could be generated.

### Effect of peptide concentration

The molecular beacon at 400nM was added to increasing concentrations of bN peptide from 0-800µM in the presence of either Mn²⁺ or Zn²⁺ ions. The bN peptide elicited a signal change with Mn²⁺ and Zn²⁺ ions. However, at a concentration of 400µM the signal from the bN peptide with Mn²⁺ ions was greater than the bN peptide with Mg²⁺ ions. A general increase in the signal measured for the disassembly of the molecular beacon was observed with increasing peptide bN concentration. The signal of the bN peptide in the presence of Mn²⁺ ions does saturate after 400µM whereas the signal was found to still increase up to 800µM of bN peptide with Zn²⁺ ions.

### Gel plasmid cleavage assay

The signal emitted from the molecular beacon could result from cleavage of the DNA by the peptide, resulting in the dissociation of the double-stranded region of the hairpin structure due to a low Tₘ range (melting temperature) for the resulting oligos generated. However, the signal could also be produced by the peptide binding to the DNA and distorting the shape of the hairpin structure. This could change the proximity of the fluorophore and the quencher which could also produce a signal release. Therefore, the molecular beacon assay cannot differentiate between binding and cleavage.

For this reason a further gel plasmid cleavage assay was employed to look for cleavage. Such a gel cleavage assay can differentiate between plasmid that is intact (no cleavage), nicked (ssDNA cleavage) and linearised (dsDNA cleavage) as the three plasmid species will migrate differently on an agarose gel. Intact plasmids have a supercoiled structure and therefore can migrate easily through an agarose gel whereas a ssDNA cleavage on the plasmid will relax this supercoiled structure. This produces a relaxed circle/nicked structure that migrates more slowly through the pores. The dsDNA cleavage of a plasmid produces a linearised plasmid. The linear structure migrates faster than a relaxed circular structure although not as fast as the supercoiled structure.

To optimize the assay, plasmid DNA (pBR322) was mixed with increasing concentrations of the peptide bL or bN, in the presence of Zn²⁺ ions. This was found to result in an increase in the percentage of nicked plasmid after 12 hours. The percentage of nicked species increases with greater peptide concentration for both bL and bN. The percentage of nicked plasmid decreases for the bN peptide with a peptide concentration greater than 600µM whereas the increase in the amount of nicked plasmid continues to increase at such higher peptide concentrations for the bL peptide. An increasing proportion of the plasmid DNA is also observed in the wells of the gel. This DNA can be binding to the peptide causing a complex that is unable to migrate into the gel.

The peptides bL, bN and bD all demonstrated some cleavage. However, the peptide bD was chosen for further study because of its higher propensity to produce a highly stable amyloid beta sheet structure as assessed by ThT assay.

### Protein design

The percentage of cleavage produced by peptide bD was found to increase with peptide concentration suggesting assembly of the peptide into a supramolecular structure enhances its functionality as a cleavage enzyme. However, the peptide assembly without control can produce a large aggregate. Hence, constructs were designed in which several identical peptides were linked by a short amino acid linker or loop that could be expected to produce a beta turn-like structure. This not only connects the monomer units but allows close proximity of the monomer units encouraging the peptides to produce the desired beta sheet structure.

The proof of concept design took account of the following principles:
1. A beta strand mimicking peptide of length 7 or 9 amino acids (hereinafter referred to as a beta peptide) with an alternating pattern of hydrophobic and hydrophilic residues to be used. The hydrophobic leucine residue is interchangeable with isoleucine.
2. Provision of linkers of 4 amino acid to connect the individual beta strand mimicking monomers designed to provide a beta turn and encourage a beta sheet fold between the connecting peptides units.
3. Provision of flanking units with negative design, i.e. to discourage intermolecular binding and aggregation between each beta sheet protein molecule.

For the final proof of concept construct, the leucine residues of peptide bD were replaced with isoleucine to give the 7mer peptide ibD since ibD was found to produce no observable aggregates even at the highest tested concentration. It is possible that isoleucine could aid the formation of β-sheet structure more efficiently than leucine, as the former is more hydrophobic than the later. The bD peptide did not exhibit the greatest signal from the peptide functionality studies *in vitro.* However, the peptide showed the least propensity to aggregate and to precipitate, therefore it was expected that the bD peptide would be a more stable peptide as the monomer unit of a larger multimer construct such as the decamer designed. To enhance the potential for folding into a stable secondary beta structure, the leucine's were substituted with isoleucine. This has been previously demonstrated to enhance the enzymatic activity of amyloid-forming peptides (Rufo et al. Short peptides self-assemble to produce catalytic amyloids. Nature Chem. (2014) 6, 303-309) and was applied to potentially produce a more stable fold for the multimeric construct.

### Beta turn designs

To produce beta turns that can connect the beta peptide units together, the appropriate length of and the amino acid composition of each beta turn needs to be designed. Consideration is given to both the design of beta turns in native proteins and the type of amino acid side chains that are required to orientate the beta strand sequences (the beta peptide units) to hydrogen bond and produce a beta sheet structure. Studies show that the optimal beta turn length in native proteins is often 2, 4 or 5 residues in length. In native proteins a beta turn that consists of either 2 or 5 amino acids often folds in favor of a specific chirality whereas beta turns with a length of 4 amino acids do not fold in favor of any specific chirality. Hence, for the proof of concept, an amino acid length of 4 was chosen for provision of the linkers between the selected self-assembling peptides.

The role of the beta turns is to encourage this self-assembly by bringing the peptide units close to each other. Therefore, it is not necessary to design a rigid beta turn with amino acids residues that might force a specific-turn conformation and restrict the potential structural fold of the beta peptides. Instead, the design of the beta turns is more flexible. In native proteins polar amino acid residues (serine, asparagine, glutamine and threonine) are often present in beta turns. The hydrophilic side chains of these amino acids can readily hydrogen bond, stabilizing the potential beta turn conformation. In addition, the side chains of these amino acids have no charge so are less likely to contribute to structural rearrangement or interact with the active site residues. To stabilise the predominantly hydrophobic beta peptide units, charged amino acids (glutamic acid and aspartic acid) are also incorporated into the beta turn designs potentially increasing the solubility of the complete protein. The selection of the fourth amino acid is more restricted, preferentially to glycine. The small and flexible side chain of this amino acid prevents steric repulsion, allowing for the β-β connection.

### Negative design of flanking units

The beta peptide's ability to readily self-assemble into a beta-sheet structure is advantageous for producing a beta sheet protein structure but the self-assembly of the beta peptides is uncontrollable, producing potentially large fibrillar structures. The introduction of the beta turns can control the number of beta peptides per molecule. However, this does not prevent intermolecular assembly of these beta sheet protein molecules. To prevent this, "negative design" is incorporated into the beta sheet protein design as seen in natural β-sheet proteins (see Richardson and Richardson (2002 PNAS 99, 2754-2759). For this a single amino acid with a charged side chain, a lysine amino acid residue, was introduced into each flanking beta peptide unit thereby providing charged residues at the intermolecular interface and inhibiting the aggregation of the beta sheet protein molecules.

For the proof of concept construct, the second and third hydrophobic residues (positions 3 and 5) were substituted for lysine amino acids residues in the respective N- and C- terminal beta peptide units. See Figure 8.

### Beta sheet peptide orientation

The FTIR experiments suggested that the beta peptide units predominantly adopt an anti-parallel beta sheet structure, but the same also suggested that the self-assembly is dynamic and evolves over time including parallel beta sheets and some unstructured loops regions. It was recognized that an artificial construct could have two different types of secondary structure formation with monomers binding with each other in the same N to C terminal direction or opposite direction. To produce a non-inverted beta sheet like assembly, each peptide unit was connected in the N- to C-terminal direction using a beta turn. This leads to the catalytic residue in position 2 of the first peptide unit potentially hydrogen bonding to the catalytic residue in position 6 of the second peptide unit (Figure 9). Thus, for example, consecutive ibD peptides may be linked by a N₄ linker providing a beta turn as follows:

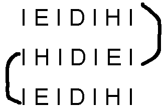
I E I D I H I
I H I D I E I
I E I D I H I

See also again Figures 8 and 9.

To produce an inverted beta sheet like structure the sequence of every other peptide unit is reversed. This results in the catalytic residue in position 2 of peptide unit 1 (in the N to C terminal direction) potentially hydrogen bonding to the catalytic residue in position 2 of peptide unit 2 (Figure 9).

### Example 3: Fusion of artificial nickase into an ApGET modular polypeptide

Taking the design aspects above the beta peptides were assembled initially into a pentamer, consisting of 5 beta peptides, and decamer consisting of 10 beta peptides. The pentamers and decamers (Figure 8) were flanked on their N- and C- terminus by a beta peptide unit that incorporates negative design to reduce aggregation as explained above. In addition, inverted and non-inverted designs were produced (See Figure 9). Each peptide unit represents a potential beta strand and is separated by a beta turn of four amino acids. The N-terminal was fused directly onto the C-terminus of the DBD as shown by SEQ. ID. No. 1 through a linker (Linker 1). A flexible, soluble region was provided at the terminus of the C-terminal flanking region comprising of asparagine, glutamine, glycine and serine.

For testing of nickase activity as part of a longer fusion construct additionally providing both a RSBD and DBD, each protein construct was fused onto the C- terminal region of the construct ApGet-i (SEQ. ID. No. 1 minus the final linker and nickase) by provision of a linker sequence. The linker chosen was
GGGGSGGGGSGGGGGS (SEQ. ID. No. 5).

This final construct is referred to as an ApGet1.0 nuclease protein.

Such modular polypeptides were expressed using two different systems to use various mechanism of action or *in vitro* characterisation studies: (i) *in vitro* transcription translation (IVTT) and 2) bacterial recombinant protein production *(E. coli* protein production). To enhance the solubility and to ease downstream protein purification steps a series of protein tags were attached onto the ApGet1.0 sequence (see Figure 10). The first protein design contained an N-terminal hexa-histidine tag (6His-tag) preceding a protease specific cleavage site (TEV cleavage site). The cleavage site can be cleaved by the TEV protease allowing the removal of the 6His-tag after purification of the ApGet1.0 protein. The second protein design was conceived to potentially enhance the solubility of the ApGet1.0 construct by fusing a maltose binding protein (MBP) to the N-terminus of ApGet1.0. The MBP tag was provided with an N-terminal 6His-tag. The C terminus was fused to a second 6His Tag followed by a spacer region consisting of glycine and serine followed by a TEV protease cleavage site. The spacer reduces steric interference of the MBP on the folding of the ApGet1.0 and allows adequate access to the TEV protease for successful cleavage of the His-MBP-His tag from ApGet1.0.

### In vitro transcription and translation (IVTT)

An IVTT expression system was used to initially screen the ApGet 1.0 proteins for cleavage activity with enhancement of expression as discussed above. A cell free *in vitro* transcription and translation system translates a protein without the constraints of a cellular environment. Such a system is often used to express insoluble and toxic proteins that are not stable in the cell and would otherwise aggregate or cause cell death reducing protein yield. The PURExpress^{®} IVTT system (New England BioLabs) was used which can express the proteins free from proteases, RNases and DNases. This was desirable as the presence of proteases can reduce the overall protein yield and RNases/DNases can interfere with *in vitro* cleavage assays.

The MBP-fused ApGet1.0 was cleaved prior to any cleavage assays to remove the large MBP protein as it might sterically interfere with the action of the ApGet1.0 proteins. The cleaved protein was semi-purified before cleavage assays by carrying out a crude (batch) purification of the protein mix with Ni-NTA resin. The resin captures the 6His tag extracting the His-MBP-His fusion tag and His-TEV protease from the protein solution.

### Initial non-specific cleavage assays with ApGet1.0 proteins

Initial cleavage assays were carried out with the ApGet1.0 decamer proteins expressed with IVTT and incubated with plasmid DNA substrate. To act as negative controls the assays with ApGet1.0 decamers were carried out alongside IVTT expressed ApGet-i (expressed with additional inclusion of the DBD-enzyme linker of ApGet1.0 at the C-terminus) to control for miscellaneous activity from the IVTT mixture and a no protein control to control for different buffer compositions. Without an associated targeting nucleic acid, any cleavage of DNA substrate by an ApGet1.0 protein was expected to be non-specific. As the design principles used for the artificial nuclease anticipate that divalent metal cations will be essential for activity, buffers containing either no metal, Mn²⁺ or Mg²⁺ were tested. Mn²⁺ and Mg²⁺ were used at 5mM. The remaining buffer components reflected physiological conditions: 25mM Tris-HCI pH8.0, 150mM NaCl.

By way of example, the ApGet1.0 protein of SEQ. ID. No. 1 was found to nick plasmid DNA (representing ssDNA cleavage) in the presence of Mg²⁺ or Mn ²⁺ ions. As noted above, this is the ApGET employing the artificial nuclease of SEQ.ID. no. 6:

IEKDIHINDGGIEIDIHIDSGGIEIDIHISSGSIEIDIHINNGNIEIDIHIGSDGIEIDIHIGNSGIEIDIHID
NGGIEIDIHIDSDGIEIDIHISSSGIEIDIHIGSEGIEIDIHIGDSGIEIDKHINQGS (SEQ. ID. No. 6).

In this sequence, all the 7mer monomer units of IbD (IEIDIHI) are linked without reversal in the N-terminal to C- terminal direction by 4mer linkers to provide beta turns. The flanking N-terminal and C- terminal peptide units are shown in bold immediately above and have the 2nd and 3rd hydrophobic residue positions respectively substituted by a positively charged lysine residue. As also noted above, the C-terminal unit is provided with a C -terminus sequence of NQGS to aid solubility.

No cleavage was detected without a divalent metal present. The percentage of nicked species was greater when Mn²⁺was present compared to Mg²⁺. The no protein control and ApGet-i are not able to cleave the DNA substrate in the presence or absence of metals indicating a specific cleavage activity associated with the ibD peptide decamer (see Figure 11).

### Recombinant protein expression in E. Coli

The expression of an ApGet1.0 10mer protein using the IVTT system is useful to be able to screen for initial cleavage activity. However, an increase in protein yield, purity, and the ability to quantify the proteins can assist in revealing the efficiency of the enzymes and understanding their mechanism of action. *E. coli* recombinant technology can produce a greater yield of protein and purification can be assisted using a series of columns for chromatography.

### Expression and Purification

For increased high level expression, a DNA sequence encoding the ApGet1.0 protein with an MBP tag as discussed above, was placed within a high-copy plasmid, pET24a which ensures that upon transformation each cell contains many copies of the ApGet1.0 protein thereby increasing protein yield. This was enhanced further by using the T7 promoter. The plasmid was used to transform DE3 lysogenic bacterial cells that contain the T7 RNA polymerase gene and expression of the RNA polymerase was induced by IPTG.

The host cells chosen were BL21 (DE3) RIPL codon plus *E*. *coli* cells (Agilent Technologies). The MBP-fused ApGet1.0 protein was over expressed with an induction temperature of 18°C for 12-18 hours. The ApGet-i protein (again including a C-terminal linker) was similarly expressed at 37°C for 4 hours.

To purify the ApGet1.0 protein, cell lysate containing the overexpressed ApGet1.0 protein was passed through a Ni-NTA affinity-based chromatography column, using the 6His-tag to purify the MBP fused ApGet1.0 protein from the lysate. A second cation exchange column was used to further purify the MBP-ApGet1.0 protein, followed by cleavage reaction with the TEV protease. This cleaves the target protein from the whole His-MBP-His protein tag. Final separation of the His-MBP-His tag and the TEV protease from the target protein was carried out by batch purification using Ni-NTA resin. The target protein remains in solution while the tag and protease bind to the resin.

### Example 4: Proof of concept testing in bacterial cells

We devised a testing platform to firstly explore in *E.coli* cells the on-target recruitment of the ApGet polypeptide. *E.coli* were co-transformed with a first plasmid, termed the "Editor" encoding an ApGet configuration to be tested, and a second plasmid, the "Detector" encoding factors that generate a differential signal upon gene-editing mediated by the ApGet system. The two plasmids have distinct and compatible origins of replication and contain different antibiotic resistance genes allowing co-transformation and stable maintenance of both plasmids in the *E.coli* population.

### Delivery of an ApGet-i system into bacterial cells and proof of transcription inhibition

The Editor plasmid is a multi-copy plasmid, which encodes the ApGet polypeptide under the control of a native SpCas9 promoter and the nucleic acid component (NAC) under the control of the J23119 promoter. This is a commonly used strong synthetic promoter to drive gRNA expression in *E*. *coli* (see, for example, in Qi et al. Repurposing CRISPR as an RNA-guided platform for sequence specific control of gene expression. Cell (2013) 152, 1173-1183). The Detector plasmid comprises a Lac promoter driven eYFP or LacZa protein coding sequence with its 5' upstream region containing four identical targets complementary to the target element deployed in the ApGet system. See Figure 12.

The target element sequence (TE) in the Editor plasmid is:
GAACTTTCAGTTTAGCGGTCT (SEQ. ID.no. 61)

The targeting element in the NAC was fused using the connector AATTT to a BoxB RNA scaffold sequence which binds the RSBD of the ApGet polypeptide.

The targets in the two Detector plasmids are as follows (SEQ. ID. No. 62):
**Target sequence in PC1 and PC2 in 5'-3' direction (sequences in grey boxes are PDS and sequences in white boxes are targets, separated by other sequences in between):**

GAACTTTCAGTTTAGCGGTCTGAGGTCCCATAGCTGTTTCCTGTGTGATATCATGCAGCGGAGTG
CGAACTTTCAGTTTAGCGGTCTGAGGTCCCATAGCTGTTTCCTGTGTGAATTTCGCGCAACGCAT
AAGAACTTTCAGTTTAGCGGTCTGAGGTCCCATAGCTGTTTCCTGTGTGATGCGCCACATCCACA
TCGGAACTTTCAGTTTAGCGGTCTGAGGTCCCATAGCTGTTTCCTGTGTGA

In preliminary experiments, an "ApGet-i "system was generated where the protein component encoded by the Editor plasmid excluded any nuclease. This was identical to the linked RSBD and DBD components of the ApGet polypeptide of SEQ. ID. No.1 but minus the artificial nickase. Nevertheless, it was anticipated that the successful recruitment of the ApGet modular polypeptide-NAC complex to the PDS containing targets would inhibit transcription of eYFP reducing the fluorescent signal or inhibit expression of LacZa. This proved to be the case; see Figures 13a and b. After the induction, the reduction in the eYFP fluorescence or in the activity of the LacZa gene (in hydrolysis of o-nitrophenyl-beta-D-galactosidase) was observed only when either of the detector plasmids were delivered with the ApGet-i expressing plasmid indicative of transcription inhibition activity of the ApGet-i.

Table 5 below summarises the construct testing with detection of eYFP expression. Table 6 below summarises the construct testing with detection of LacZa expression. Construct 1 = plasmid for expression of ApGet-i. Construct 2= the eYFP detector plasmid. Construct 3 = the LacZa detector plasmid. Construct 4= plasmid having similar backbone to construct 1 but without an ApGet-i expression cassette.

**Table 5**

| Reaction no. | 1 | 2 | 3 | 4 | 5 | 6 | 7 |
|---|---|---|---|---|---|---|---|
| Construct1 | + | - | + | - | + | + | - |
| Construct2 | + | + | - | - | + | - | + |
| Construct3 | - | - | + | + | - | + | - |
| Construct4 | - | - | - | - | - | - | + |
| Culture | LB | LB | LB | LB | LB | LB | LB |
| Copy number of detector | multiple | multiple | multiple | multiple | multiple | multiple | multiple |
| Induction | IPTG | IPTG | IPTG | IPTG | no IPTG | no IPTG | IPTG |

**Table 6**

| Reaction no. | 1 | 2 | 3 | 4 |
|---|---|---|---|---|
| Construct1 | + | - | - | - |
| Construct2 | - | - | + | - |
| Construct3 | + | + | - | + |
| Construct4 | - | - | + | + |
| Culture | LB | LB | LB | LB |
| Copy number of detector | multiple | multiple | multiple | multiple |
| Induction | IPTG | IPTG | IPTG | IPTG |

### Testing with a Fok1 nuclease domain

The testing of the ApGet concept was then extended by fusing the catalytic unit of the Fokl nuclease through a linker (the linker of SEQ. ID. No 5 as shown above in SEQ. ID.no.1) to the DBD domain of the ApGet-i so as to create a fully functional RNA-directed genome editing enzyme. The nuclease linker was chosen in part to optimize the nucleotide sequence for synthesis of synthetic DNA fragments. In this case, the Editor plasmid for the ApGet-Fokl system consisted of the nucleic acid component (NAC), expressed under the control of the constitutive J23119 promoter and the ApGet-Fokl protein under the control of an inducible Tet-On promoter (the Tet repressor (TetR) regulates expression of the Tet promoter (TetP) by binding to the Tet Operator (TetO) sequence). The backbone of the Editor plasmid is derived from pET28a and includes the ColE1 origin of replication and kanamycin resistance expression unit (see Figure 14). The Detector plasmid contained a target cassette comprising two identical target sequences on opposite DNA strands (recognized by a single ApGet NAC component expressed from the Editor plasmid used in the experiment), for example with PDS sequences located internal to their respective target sequences (we term this the "inward", or "PDS-in" configuration).

The target element sequence and target sequences were as follows:
**Target Element sequence:** CACACAGGAAACAGTATTCAT (SEQ. ID. NO. 63)
**Target Sequences (grey boxes indicate PDS, white boxes indicate target sequences):** "PDSout" configuration:

5' GACCTCATGAATACTGTTTCCTGTGTGCACACAGGAAACAGTATTCAT[?][?][?][?][?][?] 5'
3' [?][?][?][?][?][?]TACTTATGACAAAGGACACACGTGTGTCCTTTGTCATAAGTACTCCAG 3'

"PDSin" configuration with 5 nt spacer:
5' CACACAGGAAACAGTATTCAT[?][?][?][?][?][?]AATTGGACCTCATGAATACTGTTTCCTGTGTG 3'
3' GTGTGTCCTTTGTCATAAGTACTCCAGTTAAC[?][?][?][?][?][?]TACTTATGACAAAGGACACAC 5'

"PDSin" configuration with 8 nt spacer:
5' CACACAGGAAACAGTATTCAT[?][?][?][?][?][?]AATTGGTCGACCTCATGAATACTGTTTCCTGTGTG 3'
3' GTGTGTCCTTTGTCATAAGTACTCCAGTTAACCAG[?][?][?][?][?][?]TACTTATGACAAAGGACACAC 5'

"PDSin" configuration with 14 nt spacer:
5' CACACAGGAAACAGTATTCATGAGCTCGTTTAAACGTCGACGACCTCATGAATACTGTTTCCTGTGTG 3'
3' GTGTGTCCTTTGTCATAAGTACTCCAGCAAATTTGCAGCTG[?][?][?][?][?][?]TACTTATGACAAAGGACACAC

(SEQ. ID. Nos 64-67)

The detector plasmid also contained elements derived from pCC1 which contain the ori2 and oriV origins of replication and an antibiotic resistance gene, distinct from that on the Editor plasmid, in this specific example the chloramphenicol resistance gene (see again Figure 14).

Successful recruitment of ApGet-Fokl would be expected to result in site-specific double-strand breaks resulting in the loss of the detector plasmid, which as it contains a resistance gene to an antibiotic present in the culture medium should result in reduced bacterial growth (Figure 15). To facilitate detection of the editing activity of ApGet, these tests were done by co-transformation of the Editor and Detector plasmids into EPI300 cells (EpiCentre), which had been engineered to encode oriV activator trfa under tight regulation of the Para BAD promoter, inducible by L- Arabinose. Growth of EPI300 cells co-transformed with Editor and Detector plasmids in growth medium which lacks L-Arabinose will result in very low copy number of the Detector plasmid (as the multi-copy ori-V origin of replication is not being activated). This was expected to make cells more sensitive to the chloramphenicol upon loss of the Detector plasmid as a result of DNA editing activity of the ApGet-Fokl. Detection of the editing activity was by measuring OD600 a day after induction of ApGet with Anhydrotetracycline (ATC). Control experiments included co-transformation of either ApGet-i expressing editor (ApGet construct, lacking a nuclease domain) or non-ApGet expressing plasmid and detector plasmids into EPI300 cells. Successful testing is illustrated by the reduced OD600 observed with expression of the ApGet- FoK1 in cells also containing detector plasmids having the 'PDS-in' format for the PDS-target sequence pairs and a spacer between these pairs of 5, 8 or 14 nts (See bars 1-3 in Figure 15).

### Testing with an artificial nickase module

Following successful testing of the ApGet platform fused to Fokl nuclease, we expanded our testing to the ApGet fused to an artificial nuclease (AN) including the preferred selected artificial nickase as noted above (SEQ. ID. No. 6) .

In summary, an ApGet system was delivered to *E. coli* bacterial cells with the aim of correcting a disrupted gene. The cells were co-transformed with a detector plasmid carrying a disrupted nanoluciferase gene and an editor plasmid expressing the ApGet system where the polypeptide component of the system ( e.g. a modular polypeptide having the amino acid sequence of SEQ. ID. No. 1) is under the control of a T7 promoter and Lac operator and the RNA component of the system is expressed under the control of a synthetic promoter (J23119) A repair template is also embedded with approx. 200bp right and left homology arms in the editor plasmid.

The sequence of the donor or repair template element in the 5'-3' direction was as follows: (sequence of the left homology arm is underlined and sequence of the right homology arm highlighted in grey):

GTACACGGCCGCATAATCGAAATTAATACGACTCACTATAGGGGAATTGTGAGCGGATA
ACAATTCCCCATCTTAGTATATTAGTTAAGTATAAGAAGGAGATATACATATGGTGTTCA
CACTCGAAGATTTCGTTGGGGACTGGCGACAGACAGCCGGCTACAACCTGGACCAAGT
CCTTGAACAGGGAGGTGTGTCCAGTTTGTTTCAGAATCTCGGGGTGTCCGTAACTCCG
ATCCAAAGGATTGTCCTGAGCGGTGAAAATGGGCTGAAGATCGACATCCATGTCATCAT
CCCGTATGAAGGTCTGAGCGGCGACCAAATGGGCCAGATCGAAAAAATTTTTAAGGTG

GTGTACCCTGTGGATGATCATCACTTTAAGGTGATCCTGCACTATGGCACACTGGTAAT
CGACGGGGTTACGCCG (SEQ. ID. No. 68)

Various ApGet polypeptides with different lengths and orientations of the nuclease peptide units were tested (See Figure 16). The expression cassettes were modified compared to those for expression of ApGet polypeptide production for purification and testing as discussed above. The expression trials showed that the addition of the solubility tag MBP enhanced the expression of the protein in *E. coli.* However, such a tag was not required to obtain editing activity. A cleavable 6His tag was however retained.

Specifically, we tested ApGet1.0 with the nuclease domain consisting of up to 10 peptide units (decamer) in "non-inverted" and "inverted" protein translation directions. To maximize the detection sensitivity of the ApGet editing activity, the detector plasmid provided a "gain of function" detection system, where generation of nicks in the double-stranded DNA sequence of the detector element can facilitate Homology Directed Repair (HDR), to restore detector activity in the presence of the donor template. To further increase the detection activity, the detector was designed in the form of a nanoluciferase gene, expressed under the control of a T7 promoter and disrupted by the target cassette, which is recognized by ApGet1.0. The target cassette is composed of two identical target sequences in tandem, in either "PDS-out" or "PDS-in" configurations. (See constructs f and g respectively in Figure 17). The PDSin and PDSout detectors employed the same target sequences as employed previously and noted above and were located exactly between the homology arms.

We reasoned that the target cassette, consisting of two target sequences located in close proximity to each other, may increase the clustered nicking activity of the AN of ApGet1.0 thus increasing the chance for the double-stranded break, which in turn may trigger more efficient HDR which will result in the expression of the functional nanoluciferase. As noted above, the Homology-directed repair (HDR) template for the nanoluciferase was constructed on the same plasmids that express ApGet as a separate unit.

Figures 18 and 19 further illustrate the use of PDS-in and PDS-out configurations of pairs of PDS-target sequences as targeted by a synthetic genome editing system of the invention.

Various configurations of the ApGet1.0 as tested are listed in Table 7 below.

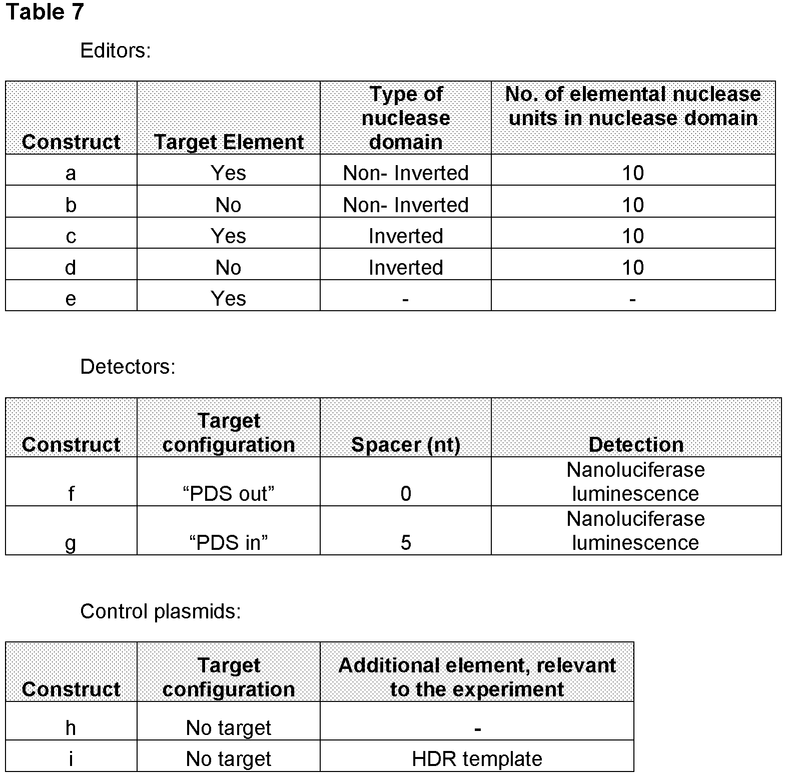

Summary of different configurations of the ApGet (a-e), detectors (f-g) and control plasmid (h-i) tested in the experiment for ability to restore nanoluciferase activity. Control plasmids used were either "h" (devoid of any functionally relevant elements for the current experiments) or "i" (plasmid with HDR unit only).

BL21 (DE3) cells were co-transformed with each of these configurations and either of the two types of Detectors with the disrupted Nanoluciferase open reading frame (Nanoluc ORF) (PDS-in or PDS-out configurations). The Nanoluc signal/OD600 ratio was analysed for each sample, reflecting the efficiency of HDR which leads to the restoration of functional nanoluciferase (Figure 19). HDR events were confirmed by Sanger Sequencing analysis as well as deep amplicon sequencing of the repaired Nanoluciferase coding sequence of the detector unit.

These results clearly showed discernible activity of ApGet1.0 systems in promoting HDR in *E. coli,* apparently by generating nicks at the integration site. Preferred for this purpose is the ApGet modular polypeptide of SEQ. ID.NO.1 with a suitable targeting nucleic acid.

### Example 5: Binding ability of the selected DBD to variant PDSs

As noted above the DBD of sequence SLETWLKHREKDGGS (SEQ. ID. No. 4) was selected on the basis of binding affinity for the dsDNA sequence represented by the 5' to 3' sequence 5'GAGGTC3' (the initially chosen predetermined sequence (PDS) for use in targeting). A further study was undertaken to determine the importance of various nucleotides in this PDS for binding to the same DBD using a nanoluciferase detection assay (Promega). This was on the basis of the prior observation as noted above that expression of ApGet-i in bacteria can suppress transcription of sequences containing target sites for the NAC plus the PDS used for DBD selection.

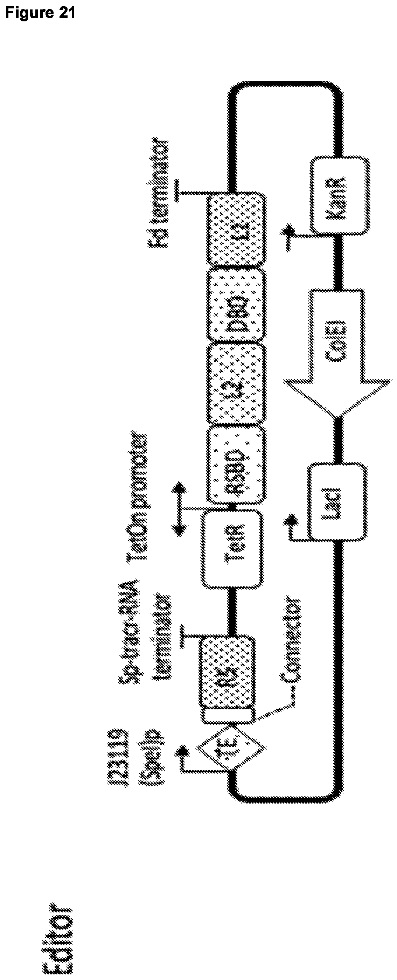

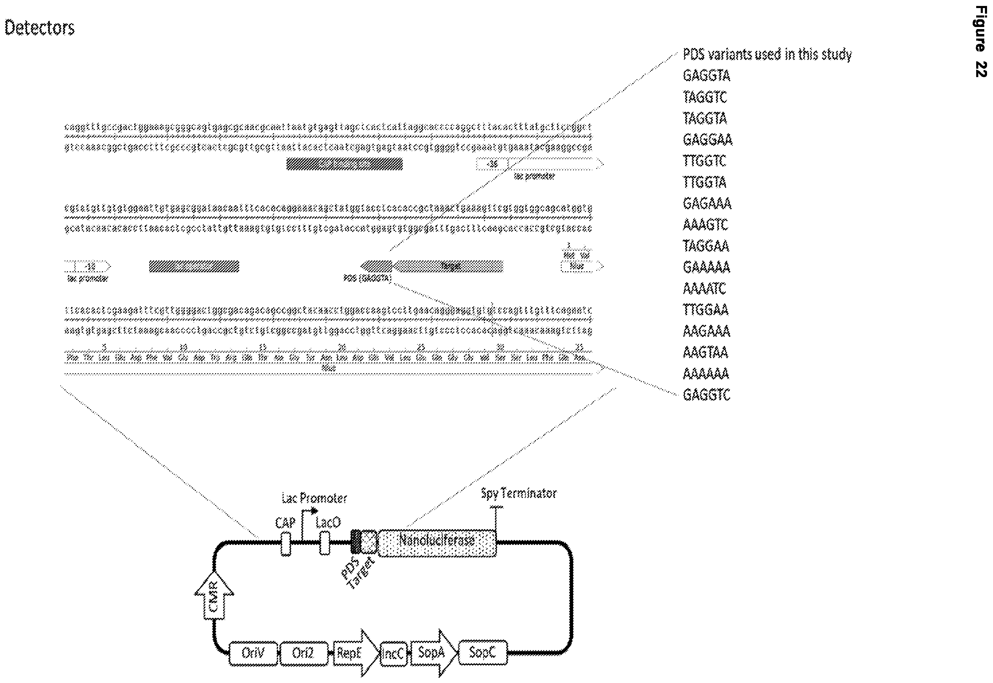

An ApGet-i expressing plasmid (the Editor plasmid) was provided as shown in Figure 21. A nanoluciferase expression cassette was cloned in a second plasmid vector (the Detector plasmid) with the PDS as originally used for DBD selection and variants of that PDS in the target region as shown in Figure 22.

Bacterial cells were transformed with the Editor plasmid in which ApGet-i expression was under the control of a TetR promoter. The NAC was also expressed by the same plasmid under the control of a constitutive J23119 promoter. The Detector and Editor plasmids were provided with different resistance genes and compatible origins of replication. The ApGet-i expression plasmid has a ColE1 high copy origin of replication. The Detector plasmids have Ori2 and OriV origins of replication. Transformation of the Detector and Editor plasmids was carried out in EPI300 cells (Lucigen), which allow for the copy control of the Detector plasmids. The transformed cultures were induced the next day with Anhydrotetracycline (ATC) to induce the expression of ApGet-i and the nanoluciferase expression was induced with IPTG (isopropyl β-D-1-thiogalactopyranoside). The signal of the nanoluciferase was monitored on the microplate reader and normalized to take account of the degree of bacterial growth in the culture.

A plasmid expressing LacZ protein with a backbone similar to the Editor plasmid and without the ApGet-i ORF was used as a control (Figure 23a). This control allowed a comparison of the reduction in the expression level of the luciferase gene under the influence of various PDSs.

The results of the experiment are summarized in Figure 23b.

It was demonstrated that the DBD directed to different PDSs of the DNA is effective in inhibiting expression of the luciferase. This confirms that the DBD described herein is flexible in terms of its ability to bind to the DNA. For example, the DBD of SEQ. ID. No. 4 can be expected to be effective in targeting not only 5'GAGGTC3' sequences in a dsDNA but also, for example, 5'TTGGGTC3' and 5'AAAAAA3' with good if not better affinity.

### Example 6: Gene editing studies in mammalian cells

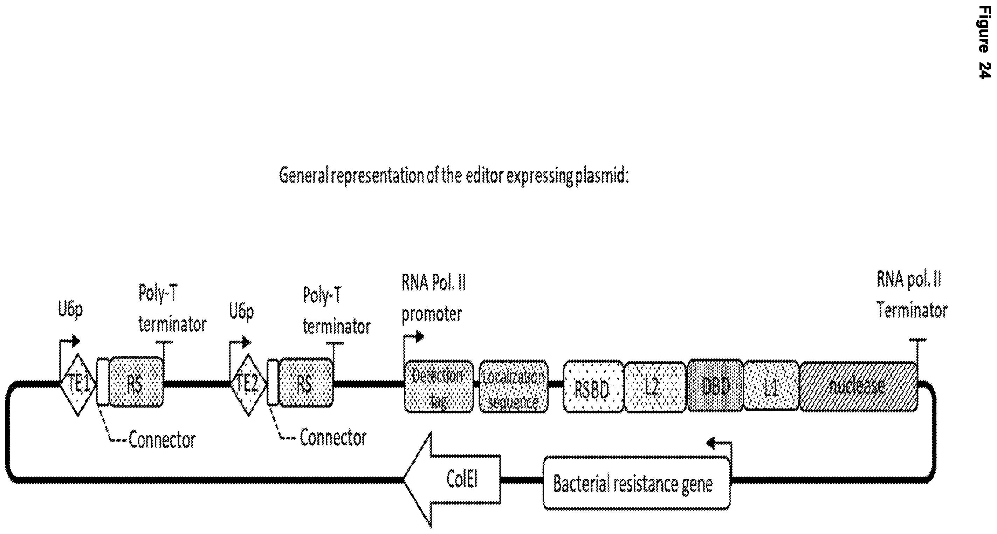

The editing efficiency of ApGet in mammalian cells was tested in HEK293T and HAP1 cells. For this purpose, optimized configurations of ApGet in plasmid or RNP forms were created. For the transient expression of ApGet in mammalian cell cultures, ApGet and NAC expression units were constructed in a circular vector of minimal necessary length, only containing a high copy origin of replication and an antibiotic resistance gene to allow for the efficient production of this vector (Figure 24). ApGet and NAC units were expressed under their respective RNA pol II and RNA pol III promoters: CMV, Cbh/CAG or Ef1A promoters for expression of ApGet and a U6 promoter for expression of each of two NAC units were used. The variants of the ApGet expression cassettes employed are illustrated more fully in Figure 25. A nuclear localization signal was provided preceded at the N-terminus by a FLAG^{®} epitope tag.

### (i) Detection of PD-L1/CD274 editing by ApGet using immunofluorescence microscopy.

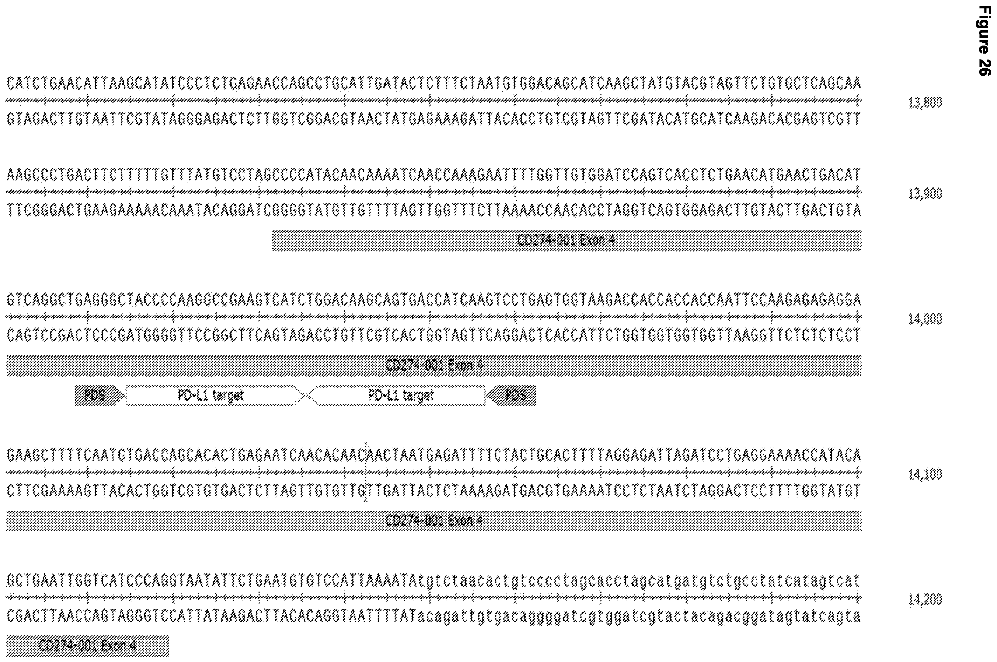

In this example, the target region of the ApGet was chosen to affect the expression of all known alternative splicing transcripts with open reading frames of the PD-L1 gene (also known as CD274) (Q9NZQ7-1, Q9NZQ7-2, Q9NZQ7-3, Uniprot). Layout of the ApGet targeting region in exon 4 of the PD-L1 gene target, including binding sites of ApGet target elements and PDS sites is shown in Figure 26.

ApGet RNP was delivered to haploid HAP1 cells by electroporation (NEON, Thermofisher). Haploid cells were selected so that there could be anticipated a direct phenotypic consequence of any reading frame changing or significant deletion mutation as the cells only contain a single copy of the target gene. Two hours after transfection, interferon gamma was added to the transfected cell cultures to induce PD-L1 expression. 24 hours after transfection, the cells were fixed, and PD-L1 was detected using immunofluorescence labeled antibodies.

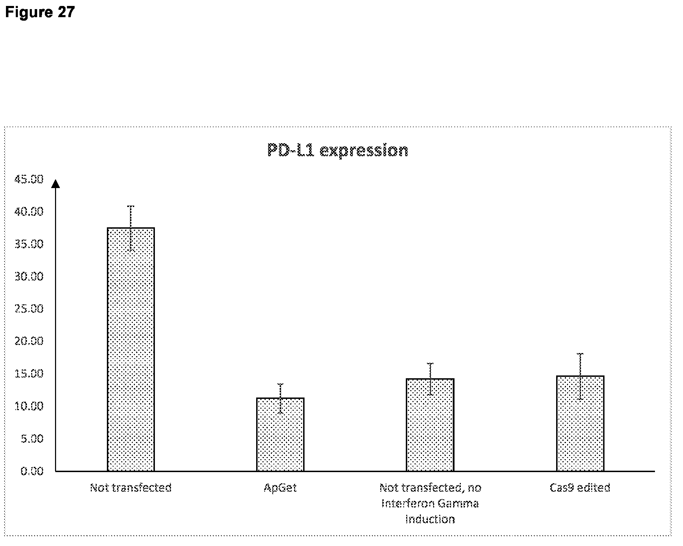

Quantification of the PD-L1 expressing cells was done by ImageJ and values were plotted for a graph using MS Excel; See Figure 27.

This experiment confirmed that expression of the PD-L1 protein in response to interferon gamma-induction was much reduced, which is consistent with gene editing at the PD-L1 locus targeted by the ApGet system.

### (ii) ApGet activity facilitates genomic integration of a donor template sequence through Homology-Directed Repair (HDR)

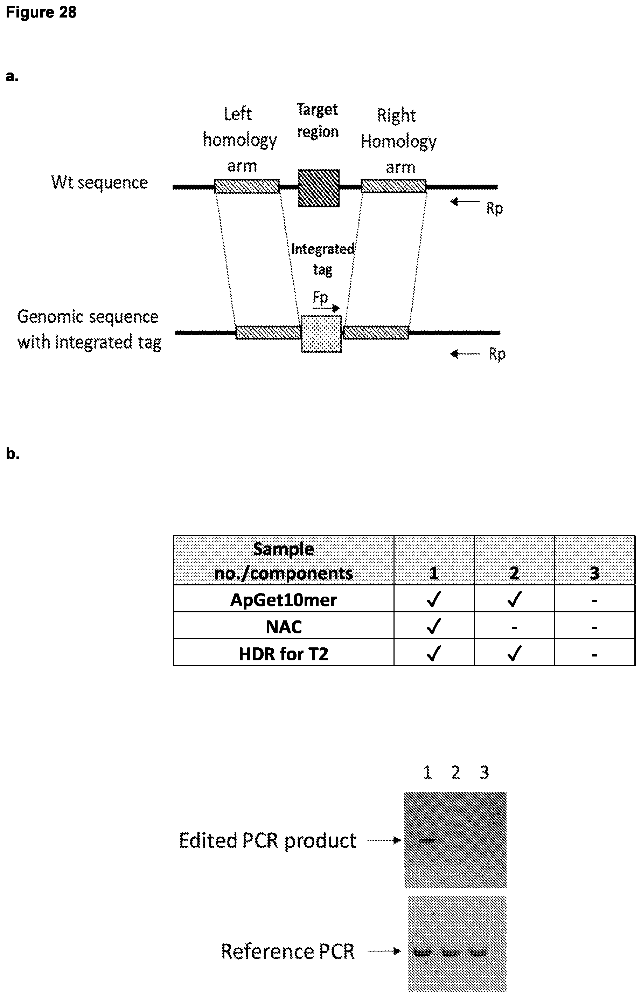

HAP1 cells were co-transfected with a vector expressing ApGet (Fig. 25, Construct I), NAC for two adjacent regions of the target region in the PD-L1 gene in the form of RNA oligos and donor template in the form of single stranded DNA oligo (ssODN, Alt-R, IDT). The donor template was designed to have a HA tag sequence with the stop codons at the 3' end, flanked by 50bp homology arms from both sides. The homology arms of the donor template were designed to bind to sequences 30bp upstream and downstream of the target region so as to minimize the effect of potential ApGet activity on and close to the target region on already integrated HDR template (Fig. 28a). Cells were harvested 24 hours post transfection and genomic DNA was extracted and analyzed by HDR specific PCR, where one of the primers binds to the integrated HA tag and another primer binds to the genomic sequence outside of the homology arms. Layout and results of the experiment described in this example are presented in Fig. 28b.

The experiment provides evidence that ApGet-mediated editing triggered the integration of the HA tag into chromosomal DNA via an HDR mechanism.

### (iii) ApGet evokes chromosomal editing at a target site in human cells

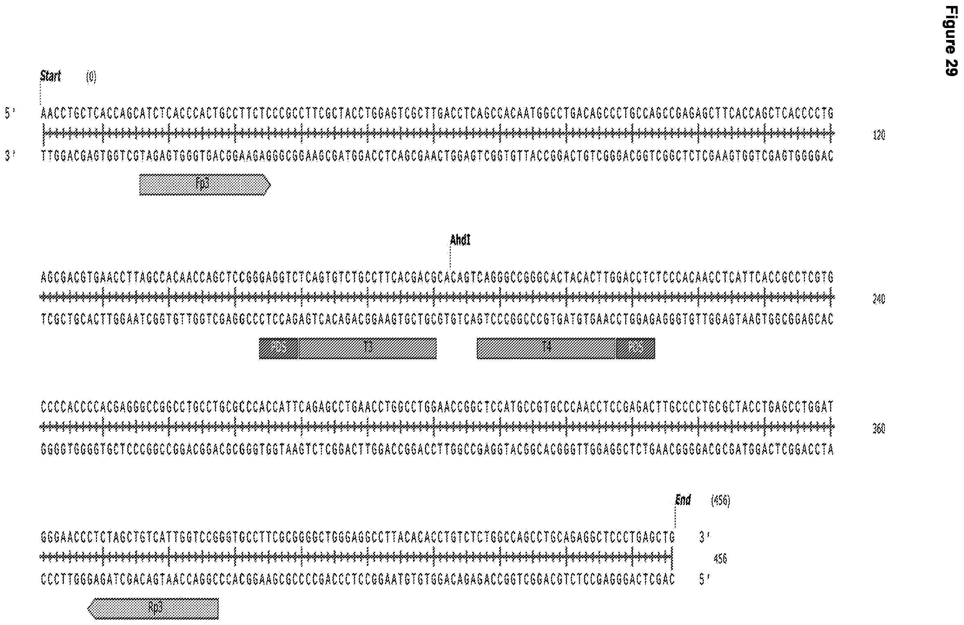

HEK293T cells were transfected with ApGet expressing constructs (j and k as shown in Figure 25), with targeting to the *TSKU* gene, that includes juxtaposed PDS sequences on either side of the target site; See Figure 29 Genomic DNA was collected 48 hours after transfection and amplified by PCR using primers flanking the target site. DNA sequence traces from Sanger sequencing with fluorescent chain terminating residues were obtained from the PCR products. These were analyzed using the cloud-based software package, TIDER, which calculates the degree of gene editing by assessing the fraction of wild-type sequence at each position. A non-transfected sample was used as a control (Brinkman et al, Nucleic Acids Res. (2018)).

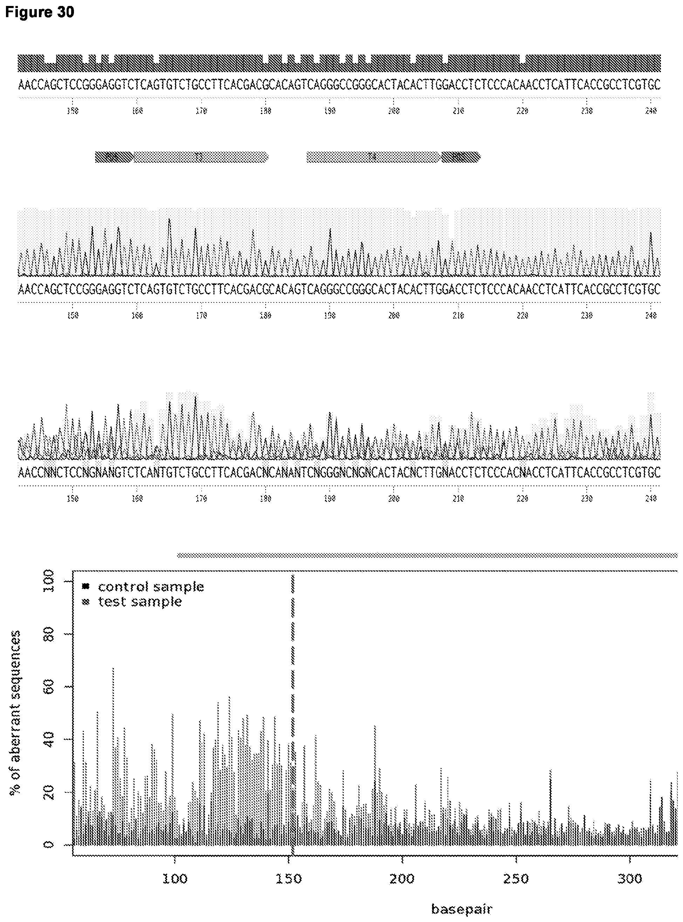

Results shown in Figure 30 indicate high rates of substitution from the wild-type sequence at sequence positions 5' to base pair 150 in the test samples compared to the control sample. This provides strong support for the contention that the ApGet system is editing chromosomal DNA at the target site in the human HEK-293 cells.

### Example 7: Recombinant production of ApGet proteins

Further studies were undertaken on ApGET protein generation using recombinant protein expression and purification techniques. These studies were undertaken with a view to optimizing ApGET-i and ApGET production (referred to below as the 'proteins of interest' or POI) by expression in *E.coli.* The POI were modified to enable soluble expression and ease of purification by producing fusions with various tags. The starting configurations which were employed for these fusion proteins are detailed in Figure 31 and consisted of the following: His-tag (6x histidine) for Immobilized metal affinity chromatography (IMAC)-based purification, a solubility tag of either maltose binding protein (MBP) or small ubiquitin-like modifier (SUMO), spacer followed by a TEV protease cleavage site, a FLAG^{®} epitope tag, a nuclear localization sequence (NLS), the POI sequence and finally a Strep-tag^{®}II sequence at the C-terminal end. This tag may be used for both ApGeti and ApGet production. However, ApGeti can be purified without the Strep II tag and thus may be expressed simply with inclusion of Linker 1 (Seq. ID. No. 5) at the C-terminus.

Between the His-tag and the solubility tag, a number of glycine or serine residues were incorporated. As the spacer between the solubility tag and the TEV protease site was originally a glycine serine rich linker, this was replaced with an asparagine rich linker in order to reduce the number of glycine and serine residues in the protein construct.

TEV protease-mediated cleavage in these constructs generated an NH2-terminal leader consisting of amino acids Gly-Trp-Gly-Ser (GWGS), thus introducing an additional aromatic amino acid residue to improve the sensitivity of absorbance measurements at 280 nm for protein quantification.

The Strep II tag when incorporated as a COOH-terminal tag enables use of affinity chromatography on a specifically engineered version of streptavidin to select full-length proteins. This allowed the removal of prematurely terminated translation products from the final protein preparations. Through this method production of full length ApGet protein was successfully achieved with purification, detection and solubility tags.

### Example 8: Transcriptional activation using an ApGeti linked to VP64

Experiments were undertaken to prove the ability of the ApGeti platform linked to a non-nuclease effector to provide modification of gene regulation as well as the modularity of the individual components of the system. The VP64 transcriptional activator protein was substituted for the artificial nuclease in the ApGet modular polypeptide corresponding to SEQ. ID. No. 1 to provide a construct designated ApGeti-VP64 and the ability of this construct to transcriptionally activate the ASCL1 gene in HEK293T cells was determined. Two variants of ApGeti-VP64 with linker variation and a dCas9-VP64 construct were also tested.

The ASCL-1 gene was selected as a known target gene for such a CRISPR/ Cas based transcriptional activator with low background transcription level. Four locations in the gene promoter region were chosen to be targeted using 4 different NACs. Each NAC consisted of a targeting element joined via a short connector to the same RNA scaffold (the Box B lambda phage sequence). As indicated above, three different modular ApGet polypeptides were employed with the same VP64 effector and the same RSBD (the lambda N22 peptide). Either linker 1 between the VP64 component and the DBD or linker 2 between the RSBD and DBD was varied as shown in table 8 below.

**Table 8**

| **Name** | **Components** | **Notes** |
|---|---|---|
| ApGet | RSBD (λN peptide) + L2 + DBD + L1 + Artificial Nuclease | Modular construct of SEQ. ID. No. 1 |
| ApGet-i_VP64 | RSBD (λN peptide) + L2 + DBD + L1 + VP64 | Artificial Nuclease is replaced with VP64 |
| ApGet-i_VP64-L1a | RSBD (λN peptide) + L2 + DBD + L1a + VP64 | Linker 1 is replaced with a different Linker |
| ApGet-i_VP64-L2b | RSBD (λN peptide) + L2b + DBD + L1 + VP64 | Linker 2 is replaced with a different Linker |
| ApGet-i | RSBD (λN peptide) + L2 + DBD + L1 + No effector protein | Control |

The amino acid sequences of the various components of the APGeti-VP64 modular polypeptides listed above are provided in table 9.

**Table 9**

| **Type of the element** | **Peptide sequence** |
|---|---|
| Lambda N22 | MDAQTRRRERRAEKQAQWKAAN (SEQ. ID. No 2) |
| DBD | SLETWLKHREKDGGS (SEQ. ID. No. 4) |
| L2 | GGGGSGGGGSGGGGSGGGGSGGGGSGGGGSGGGGS (SEQ.ID no. 3) |
| L1 | GGGGSGGGGSGGGGGS (SEQ. ID. No. 5) |
| L1a | EGKSSGSGSESKST (SEQ. ID. No. 69) |
| L2b | GGGGSGGGGSGGGGSGGGGSGGGGSGGGGS (SEQ. ID. No. 70) |
| VP64 | GRADALDDFDLDMLGSDALDDFDLDMLGSDALDDFDLDMLGSDALDD FDLDMLIN (SEQ. ID. No. 71) |

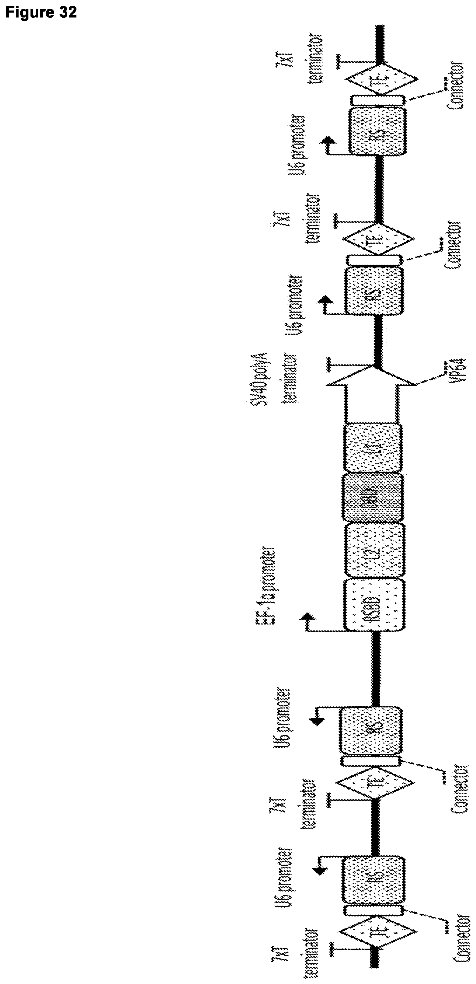

Each ApGeti-VP64 variant was expressed together with four separate NAC transcriptional units from the same plasmid. The plasmid constructs all had a general structure comprising a minimalistic vector backbone (containing a ColE1 origin of replication and a bacterial resistance gene) and an insert, comprising 5 separate transcription units: two U6 promoter driven NAC units followed by and in reverse direction of transcription to an Ef1-α promoter driven ApGeti-VP64 expression unit, followed by another two separate U6 promoter driven NAC units, as shown in Figure 32. The sequences of the different NAC expression units as well as ApGeti-VP64 variant expression units are set out in following table 10. Table 11 summarises the expression units of each plasmid construct.

NAC numbering (1 to 4) Is according to the position of the NAC in the construct (from 5' to 3'). NAC1 and NAC2 sequences are given on reverse complementary strand, as they are in reverse transcription orientation to ApGeti-VP64 and NAC3 and NAC4. Each NAC expression unit comprises a U6 promoter followed by RS (bold letters), TE (underlined sequence) and terminator sequence comprising of 7x t. Each ApGeti-VP64 expression sequence comprises an Ef1-α promoter and 5' untranslated region, followed by ApGeti-VP64 encoding sequence (underlined), comprising the RSBD (capital Italic letters), followed by Linker2 (bold letters), DBD (bold, capital letters), Linker1 (capital letters) and VP64 module (italic letters),

**Table 10**

| |
|---|
| NAC1 (Seq. ID. No. 72): |
| 5’- aaaaaaatttattcagccgggagtccggaaattgggcccttcttcagggcccttacggtgtttcgtcctttccacaagatatataa agccaagaaatcgaaatactttcaagttacggtaagcatatgatagtccattttaaaacataattttaaaactgcaaactacccaa gaaattattactttctacgtcacgtattttgtactaatatctttgtgtttacagtcaaattaattctaattatctctctaacagccttgtatcgtat atgcaaatatgaaggaatcatgggaaataggccctc-3’ |
| NAC2 (Seq. ID. No. 73): |
| 5'- aaaaaaaaagcgctttgcttcaagttcttaaaattgggcccttcttcagggcccttacggtgtttcgtcctttccacaagatatataa agccaagaaatcgaaatactttcaagttacggtaagcatatgatagtccattttaaaacataattttaaaactgcaaactacccaa gaaattattactttctacgtcacgtattttgtactaatatctttgtgtttacagtcaaattaattctaattatctctctaacagccttgtatcgtat atgcaaatatgaaggaatcatgggaaataggccctc-3' |
| NAC3 (Seq. ID. No. 74): |
| 5'- gagggcctatttcccatgattccttcatatttgcatatacgatacaaggctgttagagagataattagaattaatttgactgtaaacac aaagatattagtacaaaatacgtgacgtagaaagtaataatttcttgggtagtttgcagttttaaaattatgttttaaaatggactatca tatgcttaccgtaacttgaaagtatttcgatttcttggctttatatatcttgtggaaaggacgaaacaccgtaagggccctgaagaa gggcccaatttgctccaatttctagggtcacctttttt-3' |
| NAC4 (Seq. ID. No.75): |
| 5’- gagggcctatttcccatgattccttcatatttgcatatacgatacaaggctgttagagagataattagaattaatttgactgtaaacac aaagatattagtacaaaatacgtgacgtagaaagtaataatttcttgggtagtttgcagttttaaaattatgttttaaaatggactatca tatgcttaccgtaacttgaaagtatttcgatttcttggctttatatatcttgtggaaaggacgaaacaccgtaagggccctgaagaa gggcccaatttggggctgggtgtcccattgaatttttt-3’ |
| ApGeti-VP64 (Seq. ID. No.76 ): |
| 5'- ggctccggtgcccgtcagtgggcagagcgcacatcgcccacagtccccgagaagttggggggaggggtcggcaattgaacc ggtgcctagagaaggtggcgcggggtaaactgggaaagtgatgtcgtgtactggctccgcctttttcccgagggtgggggagaa ccgtatataagtgcagtagtcgccgtgaacgttctttttcgcaacgggtttgccgccagaacacaggtaagtgccgtgtgtggttccc gcgggcctggcctctttacgggttatggcccttgcgtgccttgaattacttccacctggctgcagtacgtgattcttgatcccgagcttc gggttggaagtgggtgggagagttcgaggccttgcgcttaaggagccccttcgcctcgtgcttgagttgaggcctggcctgggcg ctggggccgccgcgtgcgaatctggtggcaccttcgcgcctgtctcgctgctttcgataagtctctagccatttaaaatttttgatgac ctgctgcgacgctttttttctggcaagatagtcttgtaaatgcgggccaagatctgcacactggtatttcggtttttggggccgcgggc ggcgacggggcccgtgcgtcccagcgcacatgttcggcgaggcggggcctgcgagcgcggccaccgagaatcggacggg ggtagtctcaagctggccggcctgctctggtgcctggcctcgcgccgccgtgtatcgccccgccctgggcggcaaggctggccc ggtcggcaccagttgcgtgagcggaaagatggccgcttcccggccctgctgcagggagctcaaaatggaggacgcggcgctc gggagagcgggcgggtgagtcacccacacaaaggaaaagggcctttccgtcctcagccgtcgcttcatgtgactccacggagt accgggcgccgtccaggcacctcgattagttctcgagcttttggagtacgtcgtctttaggttggggggaggggttttatgcgatgga gtttccccacactgagtgggtggagactgaagttaggccagcttggcacttgatgtaattctccttggaatttgccctttttgagtttgga tcttggttcattctcaagcctcagacagtggttcaaagtttttttcttccatttcaggtgtcgtgacaagtttgtacaaaaaagcaggctg |
| ccaccatggactataaggaccacgacggagactacaaggatcatgatattgattacaaagacgatgacgataagatggcccc aaagaagaagcggaaggtcggtatccacggagtcccagcagctATGGACGCTCAGACAAGAAGAGA GCGTCGGGCCGAAAAGCAAGCTCAGTGGAAGGCGGCGAATggaggtggcggatcgggcggc ggcgggagcggtggtggcagcggcggtggcggaagcggaggaggggggagcggcggaggtagcgg agggggggggtccTCCCTGGAAACCTGGCTGAAACACAGAGAGAAGGACGGTGGGAGTG GTGGTGGAGGGAGCGGCGGAGGTGGCAGCGGCGGCGGGGGCGGGTCAggacgggctga cgcattggacgattttgatctggatatgctgggaagtgacgccctcgatgattttgaccttgacatgcttggttcggatgcccttgatga ctttgacctcgacatgctcggcagtgacgcccttgatgatttcgacctggacatgctgattaactgacagcacccagctttcttgtac aaagtggtgatggccggccgcttcgagcagacatgataagatacattgatgagtttggacaaaccacaactagaatgcagtga aaaaaatgctttatttgtgaaatttgtgatgctattgctttatttgtaaccattataagctgcaataaacaagttaacaacaattgc attcattttatgtttcaggttcagggggaggtgtgggaggttttttaaagcaagtaaaacctctacaaatgtggta -3' |
| RSBD (Lambda N22) DNA sequence (Seq. ID. No.77 ): |
| ATGGACGCTCAGACAAGAAGAAGAGAGCGTCGGGCCGAAAAGCAAGCTCAGTGGAAG GCGGCGAAT |
| Linker 1 (L1) DNA sequence (Seq. ID No. 78): |
| 5'-GGTGGTGGAGGGAGCGGCGGAGGTGGCAGCGGCGGCGGGGGCGGGTCA-3' |
| Modified linker 1 (L1a) DNA sequence (Seq. ID No. 79 ): |
| 5'-GAGGGAAAATCTTCCGGGAGTGGTTCAGAATCTAAGAGCACC-3' |
| Linker 2 (L2) DNA sequence (Seq. ID. No. 80): |
| 5’- ggaggtggcggatcgggcggcggcgggagcggtggtggcagcggcggtggcggaagcggaggaggggg gagcggcggcggaggtagcggaggggggggtcc-3’ |
| Modified linker 2 (L2b) DNA sequence (Seq. ID. No. 81 ): |
| 5’- Ggcggcggcgggagcggtggtggtggcagcggcggtggcggaagcggaggaggggggagcggcggcggag gtagcggagggggggggtcc-3’ |
| VP64 module DNA sequence (Seq. ID No. 82) |
| ggacgggctgacgcattggacgattttgatctggatatgctgggaagtgacgccctcgatgattttgaccttgacatgcttggttcgg atgcccttgatgactttgacctcgacatgctcggcagtgacgcccttgatgatttcgacctggacatgctgattaactga |
| DBD DNA sequence (Seq. ID No. 83): |
| **TCCCTGGAAACCTGGCTGAAACACAGAGAGAAGGACGGTGGGAGT** |

**Table 11**

| Name of construct | Construct outline (5' to 3') | | | | |
|---|---|---|---|---|---|
| ApGet-i_VP64 | NAC1 | NAC2 | ApGeti-VP64 | NAC3 | NAC4 |
| ApGet-i_VP64-L2b | NAC1 | NAC2 | Same sequence as ApGet-i_VP64, except for the modified linker2 (L2b) | NAC3 | NAC4 |
| ApGet-i_VP64-L1a | NAC1 | NAC2 | Same sequence as ApGet-i_VP64, except for the modified linker1 (L1a) | NAC3 | NAC4 |
| ApGet-i_VP64 | NAC1 | NAC2 | Same sequence as ApGet-i_VP64, but without the VP64 module | NAC3 | NAC4 |

### Growth of cells and transfection

HEK293T cells were transfected with a plasmid expressing an ApGet-i protein fused to VP64 plus the 4 NACs using Lipofectamine 3000 reagent in accordance with the manufacturer's instructions. 2.5.mg of the ApGeti-VP64 expressing plasmid was employed to 0.7-1 x 10⁶ HEK293T cells in wells in a 6- well plate format (ThermoFisher Scientific)

### RNA and cDNA preparation

Total RNA was extracted 48 hours post transfection using a commercially available RNA preparation kit (Monarch Total RNA Miniprep Kit, NEB). The harvested and purified RNA was transcribed also using a commercially available kit, Improm-II^{™} Reverse Transcriprion System. The level of expression of mRNA was quantified using pre-designed Taqman qPCR assays (IDT). Target Cq values (FAM dye) were normalized to ActinB Cq values (HEX dye). The fold changes in the target gene (ASCL1) expression were determined by comparing to mock transfected controls.

### Results

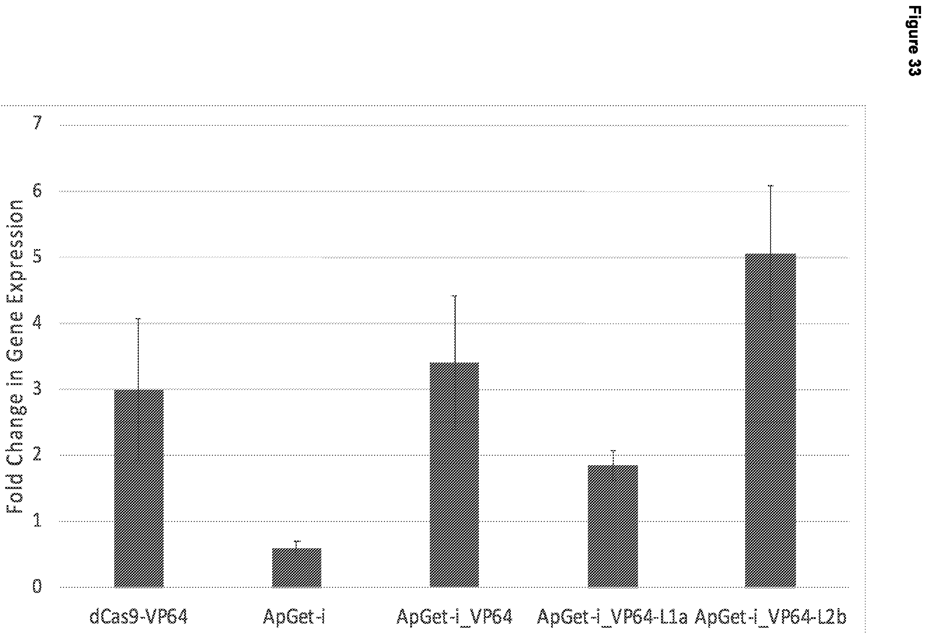

Results of the Taqman qPCR assays to assess transcriptional activation by the various ApGet-VP64 polypeptides are shown in Figure 33. For each sample, ASC1 specific signal was normalized to ActB signal. Relative fold change of the transcription level of the ASCL-1 gene normalized to the levels of mock transfected cells (vector backbone without the ApGet-VP64) are presented In the graph.

All of the ApGeti-VP64 polypeptides tested showed transcriptional activation.

## Claims

1. A nucleoprotein complex suitable for use in modifying a target nucleic acid sequence comprising (A) a targeting nucleic acid and (B) a modular polypeptide component,
wherein said targeting nucleic acid comprises:
(i) a targeting nucleic acid element (TE) complementary to a region of the target;
(ii) a recognition element which specifically interacts with a nucleic acid recognition module of said modular polypeptide component and
(iii) a connecting sequence which links said targeting element and said recognition element
and said modular polypeptide component comprises as linked, separate functional modules:
(a) said nucleic acid recognition module which is devoid of enzymic activity;
(b) a DNA-binding domain (DBD) for assisting in melting and/or unwinding of a double helix of the target, wherein the DBD recognises a predetermined sequence in the target, module (a) and said DBD not being naturally found in linkage,
(c) an effector component for use in modifying the target whereby site-specific modification directed by said TE and DBD will occur,
wherein said DBD is a polypeptide sequence of no more than 70-75 amino acid residues; wherein the length of the modular polypeptide component is no more than 300 amino acids in length;
and wherein module (b) is joined to module (a) and module (c) respectively; optionally wherein modules (a), (b) and (c) are joined by a first and/or a second linker.

2. A nucleoprotein complex as claimed in claim 1, wherein said targeting nucleic acid is an RNA.

3. A nucleoprotein complex as claimed in claim 1 or claim 2, wherein the length of the modular peptide component is no more than 220-250 amino acids in length.

4. A nucleoprotein complex as claimed in any one of claims 1 to 3, wherein said recognition element of the targeting nucleic acid is an RNA scaffold (RS) which binds an RNA scaffold binding domain (RSBD) providing said module (a) of the modular polypeptide component, for example said RS is the 19nt Box B RNA motif and said RSBD is the Lambda N22 peptide as set forth in SEQ. ID. No.2.

5. A nucleoprotein complex as claimed in claim 4, comprising as said component (A) a wholly nucleic acid component (NAC) and (B) a modular polypeptide component, wherein the NAC comprises:
(i) a targeting element (TE) complementary to a region of the target;
(ii) an RNA scaffold (RS) which specifically binds to an RNA scaffold binding domain (RSBD) of said modular polypeptide component and
(iii) a connecting sequence which links said TE and the RS
and said modular polypeptide component comprises as linked, separate functional modules:
(a) said RSBD;
(b) said DNA-binding domain linked to (a) which recognises a pre-determined sequence in the target, module (a) and said DBD not being naturally found in linkage;
(c) an effector component linked to (b) for use in modifying the target, whereby site specific modification directed by said TE and said DBD will occur and
(d) optionally, or if required, a first linker and/or a second linker linking said DBD to said effector component and the RSBD respectively.

6. A nucleoprotein complex as claimed in claim 4 or claim 5, wherein said targeting nucleic acid or said NAC provides two or more RNA scaffolds, either the same or different.

7. A nucleoprotein complex as claimed in any one of the preceding claims, wherein said DBD is no more than a 30 mer, no more than a 25 mer, no more than a 20mer, preferably no more than a 15mer; optionally wherein said DNA-binding domain binds a predetermined sequence of 3-6 nucleotides; optionally wherein said DBD is a peptide of no more than 15 amino acids, e.g. the 15 mer of sequence SLETWLKHREKDGGS (SEQ. ID. No. 4) which binds the dsDNA sequence represented by the 5' to 3' sequence 5'GAGGTC3'.

8. A nucleoprotein complex as claimed in any one of the preceding claims, wherein said DBD is capable of binding to more than one predetermined sequence.

9. A nucleoprotein complex as claimed in any one of the preceding claims, wherein said modular polypeptide component additionally provides a nuclear localization signal (NLS) and /or organelle localization signal, wherein said NLS and /or organelle localization signal is provided at the N- or C-terminus optionally connected to an additional sequence to aid detection.

10. A nucleoprotein complex as claimed in any one of the preceding claims, wherein said effector component is selected from (i) an endonuclease for producing double-stranded breaks or a Fok 1 nuclease domain (ii) a nickase (iii) a transcription activator (iv) a transcriptional repressor (v) an epigenetic modulator enzyme (vii) a recombinase (viii) a transposase (ix) an integrase and (x) a nucleobase modifying enzyme construct.

11. A nucleoprotein complex as claimed in claim 10 wherein said effector component is an artificial nuclease or nickase comprising a multimer of linked, self-assembling peptides forming a beta sheet structure wherein:
(i) the self-assembling peptides of said multimer each present an alternating pattern of hydrophobic and hydrophilic residues with, all or at least a proportion, of the hydrophilic residues of each such peptide representing a catalytic triad of amino acids whereby said multimer is capable of cleaving a single-strand or both strands of a dsDNA;
(ii) the self-assembling peptides are joined by linkers to limit the multimer number, e.g. to 4-15, preferably 5-10 and
(iii) N- terminal and C-terminal flanking sequences are provided linked to the multimer to prevent or limit aggregation between multimers of the nuclease protein with increased positive charge compared to the peptide units of the multimer due to inclusion of one or more positively charged residues, e.g. by inclusion of one or more lysine or arginine residues.

12. A nucleoprotein complex as claimed in claim 11, wherein:
(i) the modular polypeptide component is no more than 220-300 amino acids excluding any cleavable tag or localization signal or any other sequence other than components (a) - (d);
(ii) said peptides forming said multimer are identical 7mers or comprise two or more non-identical 7mers wherein the alternating hydrophobic residues are leucine and/ or isoleucine starting with the N-terminal residue;
(iii) said peptides forming said multimer are identical peptides all continuously linked in the N-terminal to C-terminal direction so as to provide an anti-parallel beta sheet structure;
(iv) the linkers joining the peptides of said multimer are amino acid linkers capable of providing a beta turn or beta turn like loops, e.g. consisting of a combination of amino acids selected from glycine (G), serine (S), asparagine (N), glutamine (Q), threonine (T), glutamic acid (E) and aspartic acid (D); and/or
(v) the C-terminus of said C-terminal flanking peptide provides a flexible tail sequence at its C-terminus to aid solubility.

13. A nucleoprotein complex as claimed in claim 11 or claim 12, wherein said peptides of said multimer are 7 mers of sequence IEIDIHI;
optionally wherein an artificial nickase is provided comprising the sequence 
IEKDIHINDGGIEIDIHIDSGGIEIDIHISSGSIEIDIHINNGNIEIDIHIGSDGIEIDIHIGNSGI
EIDIHIDNGGIEIDIHIDSDGIEIDIHISSSGIEIDIHIGSEGIEIDIHIGDSGIEIDKHINQGS
(SEQ. ID. No. 6)
or variant thereof with one or more variant beta turns and /or variation of the NQGS tail sequence;
optionally wherein the modular polypeptide component comprises the sequence of SEQ. ID. NO. 1 consisting of the following components fused in the N- terminal to C-terminal direction:
(i) the Lambda N22 protein, MDAQTRRRERRAEKQAQWKAAN (SEQ. ID. No. 2)
(ii) the linker GGGGSGGGGSGGGGSGGGGSGGGGSGGGGSGGGGS (SEQ. ID. No. 3)
(iii) the DBD SLETWLKHREKDGGS (SEQ. ID. No. 4) which binds the dsDNA sequence represented by the 5' to 3' sequence 5'GAGGTC3'
(iv) the linker GGGGSGGGGSGGGGGS (SEQ. ID. No. 5) and
(v) the nickase module: 
IEKDIHINDGGIEIDIHIDSGGIEIDIHISSGSIEIDIHINNGNIEIDIHIGSDGIEIDIHI
GNSGIEIDIHIDNGGIEIDIHIDSDGIEIDIHISSSGIEIDIHIGSEGIEIDIHIGDSGI
EIDKHINQGS (SEQ. ID. No. 6)
or the same modular polypeptide sequence with one or both of said linkers substituted by an alternative linker.

14. A nucleoprotein complex as claimed in claim 11 or claim 12, wherein the modular polypeptide component comprises the following components fused in the N- terminal to C-terminal direction:
(i) the Lambda N22 protein, MDAQTRRRERRAEKQAQWKAAN (SEQ. ID. No. 2)
(ii) the linker GGGGSGGGGSGGGGSGGGGSGGGGSGGGGSGGGGS (SEQ. ID. No. 3)
(iii) the DBD SLETWLKHREKDGGS (SEQ. ID. No. 4) which binds the dsDNA sequence represented by the 5' to 3' sequence 5'GAGGTC3'
(iv) the linker GGGGSGGGGSGGGGGS (SEQ. ID. No. 5) and
(v) an alternative effector component other than said artificial nickase module, e.g. a transcription activator such as VP64
or the same modular polypeptide sequence with one or both of said linkers substituted by an alternative linker.

15. A nucleic acid or combination of nucleic acids for provision of one or more nucleoprotein complexes as claimed in any of the preceding claims in a host cell; optionally wherein the nucleic acid is a vector capable of expressing both the polypeptide component of a nucleoprotein complex according to any one of claims 10 to 12 providing an artificial nickase and at least one targeting nucleic acid, e.g. targeting RNA.

16. A method for modifying one or more target nucleic acid sequences employing one or more nucleoprotein complexes as claimed in any one of claims 1 to 14 or one or more nucleic acids for provision thereof in a host cell with the proviso that the method is not a method of modifying the germ line identity of a human being or method of treatment practised on the human or animal body.

17. A combination of (i) at least one modular polypeptide component of a nucleoprotein complex according to any one of claims 1 to 14, or a polynucleotide encoding the same and (ii) one or more targeting nucleic acids as defined in claim 1 which can link with said polypeptide component(s), or one or more polynucleotides encoding the same, for use in a method for therapeutic treatment.

## Patentansprüche

1. Ein Nukleoproteinkomplex, der zur Verwendung bei der Modifikation einer Ziel-Nukleinsäuresequenz geeignet ist, der (A) eine targetierende Nukleinsäure und (B) eine modulare Polypeptidkomponente umfasst,
wobei die genannte targetierende Nukleinsäure Folgendes umfasst:
(i) ein targetierendes Nukleinsäureelement (TE), das zu einer Region des Ziels komplementär ist;
(ii) ein Erkennungselement, das spezifisch mit einem Nukleinsäure-Erkennungsmodul der genannten modularen Polypeptidkomponente interagiert, und
(iii) eine verbindende Sequenz, die das genannte targetierende Element und das genannte Erkennungselement verknüpft,
und wobei die genannte modulare Polypeptidkomponente als verknüpfte, separate funktionelle Module Folgendes umfasst:
(a) das genannte Nukleinsäure-Erkennungsmodul, das keine Enzymaktivität besitzt;
(b) eine DNA bindende Domäne (DBD) zur Hilfe beim Schmelzen und/oder Entwinden einer Doppelhelix des Ziels, wobei die DBD eine vorbestimmte Sequenz in dem Ziel erkennt, wobei Modul (a) und die genannte DBD nicht natürlich in Verknüpfung vorkommen,
(c) eine Effektorkomponente zur Verwendung bei der Modifikation des Ziels, wodurch eine durch das genannte TE und die genannte DBD gelenkte ortsspezifische Modifikation stattfindet,
wobei die genannte DBD eine Polypeptidsequenz von nicht mehr als 70-75 Aminosäureresten ist;
wobei die Länge der modularen Polypeptidkomponente nicht mehr als 300 Aminosäuren lang ist;
und wobei Modul (b) mit Modul (a) bzw. Modul (c) verbunden ist; wobei Module (a), (b) und (c) optional durch einen ersten und/oder einen zweiten Linker verbunden sind.

2. Nukleoproteinkomplex nach Anspruch 1, wobei die genannte targetierende Nukleinsäure eine RNA ist.

3. Nukleoproteinkomplex nach Anspruch 1 oder Anspruch 2, wobei die Länge der modularen Peptidkomponente nicht mehr als 220-250 Aminosäuren lang ist.

4. Nukleoproteinkomplex nach einem der Ansprüche 1 bis 3, wobei das genannte Erkennungselement der targetierenden Nukleinsäure ein RNA-Gerüst (RS) ist, das eine RNA-gerüstbindende Domäne (RSBD) bindet, die das genannte Modul (a) der modularen Polypeptidkomponente bereitstellt, zum Beispiel ist das genannte RS das 19nt-Box-B-RNA-Motiv und die genannte RSBD ist das Lambda-N22-Peptid wie in SEQ. ID. No. 2 dargelegt.

5. Nukleoproteinkomplex nach Anspruch 4, der als genannte Komponente (A) eine vollständige Nukleinsäurekomponente (NAC) und (B) eine modulare Polypeptidkomponente umfasst, wobei die NAC Folgendes umfasst:
(i) ein targetierendes Element (TE), das zu einer Region des Ziels komplementär ist;
(ii) ein RNA-Gerüst (RS), das spezifisch an eine RNA-gerüstbindende Domäne (RSBD) der genannten modularen Polypeptidkomponente bindet, und
(iii) eine verbindende Sequenz, die das genannte TE und das RS verknüpft,
und wobei die genannte modulare Polypeptidkomponente als verknüpfte, separate funktionelle Module Folgendes umfasst:
(a) die genannte RSBD;
(b) die genannte, mit (a) verknüpfte DNA-bindende Domäne, die eine vorbestimmte Sequenz in dem Ziel erkennt, wobei Modul (a) und die genannte DBD nicht natürlich in Verknüpfung vorkommen;
(c) eine mit (b) verknüpfte Effektorkomponente zur Verwendung bei der Modifikation des Ziels, wodurch eine durch das genannte TE und die genannte DBD gelenkte ortsspezifische Modifikation stattfindet, und
(d) optional, oder falls erforderlich, ein erster Linker und/oder ein zweiter Linker, der die genannte DBD mit der genannten Effektorkomponente bzw. der RSBD verknüpft.

6. Nukleoproteinkomplex nach Anspruch 4 oder Anspruch 5, wobei die genannte targetierende Nukleinsäure oder genannte NAC zwei oder mehrere RNA-Gerüste, die entweder gleich oder verschieden sind, bereitstellt.

7. Nukleoproteinkomplex nach einem der vorhergehenden Ansprüche, wobei die genannte DBD nicht mehr als ein 30-Mer, nicht mehr als ein 25-Mer, nicht mehr als ein 20-Mer, bevorzugt nicht mehr als ein 15-Mer ist; wobei die genannte DNA-bindende Domäne optional eine vorbestimmte Sequenz von 3-6 Nukleotiden bindet; wobei die genannte DBD optional ein Peptid von nicht mehr als 15 Aminosäuren ist, z. B. das 15-Mer von Sequenz SLETWLKHREKDGGS (SEQ. ID. No. 4), die die dsDNA-Sequenz bindet, die durch die 5'-zu-3'-Sequenz 5'GAGGTC3' repräsentiert wird.

8. Nukleoproteinkomplex nach einem der vorhergehenden Ansprüche, wobei die genannte DBD in der Lage ist, an mehr als eine vorbestimmte Sequenz zu binden.

9. Nukleoproteinkomplex nach einem der vorhergehenden Ansprüche, wobei die genannte modulare Polypeptidkomponente zusätzlich ein nukleäres Lokalisationssignal (NLS) und /oder Organellen-Lokalisationssignal bereitstellt, wobei das genannte NLS und/oder Organellen-Lokalisationssignal am N- oder C-Terminus bereitgestellt wird, optional mit einer zusätzlichen Sequenz verbunden, um die Detektion zu unterstützen.

10. Nukleoproteinkomplex nach einem der vorhergehenden Ansprüche, wobei die genannte Effektorkomponente aus Folgenden ausgewählt ist: (i) einer Endonuklease zum Herstellen von Doppelstrangbrüchen oder einer Fok-1-Nukleasedomäne, (ii) einer Nickase, (iii) einem Transkriptionsaktivator, (iv) einem Transkriptionsrepressor, (v) einem epigenetischen Modulatorenzym, (vii) einer Rekombinase, (viii) einer Transposase, (ix) einer Integrase und (x) einem Nukleobasen modifizierenden Enzymkonstrukt.

11. Nukleoproteinkomplex nach Anspruch 10, wobei die genannte Effektorkomponente eine künstliche Nuklease oder Nickase ist, die ein Multimer von verknüpften, selbstassemblierenden Peptiden, die eine Beta-Sheet-Struktur bilden, umfasst, wobei:
(i) die selbstassemblierenden Peptide des genannten Multimers jeweils ein alternierendes Muster von hydrophoben und hydrophilen Resten präsentieren, wobei alle oder mindestens ein Anteil der hydrophilen Reste jedes solchen Peptids eine katalytische Triade von Aminosäuren repräsentieren, wodurch das genannte Multimer in der Lage ist, einen einzelnen Strang oder beide Stränge einer dsDNA zu spalten;
(ii) die selbstassemblierenden Peptide durch Linker verbunden sind, um die Multimerzahl, z. B. auf 4-15, bevorzugt 5-10, zu beschränken, und
(iii) N-terminale und C-terminale flankierende Sequenzen, die mit dem Multimer verknüpft sind, bereitgestellt werden, um eine Aggregation zwischen Multimeren des Nukleaseproteins mit erhöhter positiver Ladung verglichen mit den Peptideinheiten des Multimers, bedingt durch Einschluss von einem oder mehreren positiv geladenen Resten zu verhindern oder beschränken, z. B. durch Einschluss eines oder mehrerer Lysin- oder Argininreste.

12. Nukleoproteinkomplex nach Anspruch 11, wobei:
(i) die modulare Polypeptidkomponente nicht mehr als 220-300 Aminosäuren ist, ausschließlich eines spaltbaren Tags oder Lokalisationssignals oder einer anderen Sequenz außer den Komponenten (a) - (d);
(ii) die genannten Peptide, die das genannte Multimer bilden, identische 7-Mere sind oder zwei oder mehrere nicht identische 7-Mere umfassen, wobei die alternierenden hydrophoben Reste, angefangen mit dem N-terminalen Rest, Leucin und/ oder Isoleucin sind;
(iii) die genannten Peptide, die das genannte Multimer bilden, identische Peptide sind, die alle kontinuierlich in der N-terminalen zu C-terminalen Richtung verknüpft sind, um somit eine antiparallele Beta-Sheet-Struktur bereitzustellen;
(iv) die Linker, die die Peptide des genannten Multimers verbinden, Aminosäure-Linker sind, die in der Lage sind, eine beta-Drehung oder beta-drehungsartige Schleifen bereitzustellen, z. B. bestehend aus einer Kombination von Aminosäuren, ausgewählt aus Glycin (G), Serin (S), Asparagin (N), Glutamin (Q), Threonin (T), Glutaminsäure (E) und Asparaginsäure (D); und/oder
(v) der C-Terminus des genannten C-terminalen flankierenden Peptids eine flexible Schwanzsequenz an seinem C-Terminus bereitstellt, um die Löslichkeit zu unterstützen.

13. Nukleoproteinkomplex nach Anspruch 11 oder Anspruch 12, wobei die genannten Peptide des genannten Multimers 7-Mere der Sequenz IEIDIHI sind;
wobei optional eine künstliche Nickase bereitgestellt wird, die die Sequenz 
IEKDIHINDGGIEIDIHIDSGGIEIDIHISSGSIEIDIHINNGNIEIDIHIGSDGIEID
IHIGNSGIEIDIHIDNGGIEIDIHIDSDGIEIDIHISSSGIEIDIHIGSEGIEIDIHIG
DSGIEIDKHINQGS (SEQ. ID. No. 6)
oder eine Variante davon mit einer oder mehreren varianten beta-Drehungen und/oder einer Variation der NQGS-Schwanzsequenz umfasst;
wobei optional die modulare Polypeptidkomponente Folgendes umfasst: die Sequenz von SEQ. ID. NO. 1, die aus den folgenden Komponenten besteht, die in der N-terminalen zu C-terminalen Richtung kondensiert sind:
(i) dem Lambda-N22-Protein, MDAQTRRRERRAEKQAQWKAAN (SEQ. ID. No. 2)
(ii) dem Linker GGGGSGGGGSGGGGSGGGGSGGGGSGGGGSGGGGS (SEQ ID. No. 3)
(iii) der DBD SLETWLKHREKDGGS (SEQ. ID. No. 4), die die dsDNA-Sequenz bindet, die durch die 5'-zu-3'-Sequenz 5'GAGGTC3' repräsentiert wird,
(iv) dem Linker GGGGSGGGGSGGGGGS (SEQ. ID. No. 5) und
(v) dem Nickase-Modul: 
IEKDIHINDGGIEIDIHIDSGGIEIDIHISSGSIEIDIHINNGNIEIDIHIGSDGIEID
IHIGNSGIEIDIHIDNGGIEIDIHIDSDGIEIDIHISSSGIEIDIHIGSEGIEIDIHIG
DSGIEIDKHINQGS (SEQ. ID. No. 6)
oder die gleiche modulare Polypeptidsequenz, in der einer oder beide der genannten Linker durch einen alternativen Linker substituiert sind.

14. Ein Nukleoproteinkomplex nach Anspruch 11 oder Anspruch 12, wobei die modulare Polypeptidkomponente Folgendes umfasst: die folgenden Komponenten, die in der N-terminalen zu C-terminalen Richtung kondensiert sind:
(i) das Lambda-N22-Protein, MDAQTRRRERRAEKQAQWKAAN (SEQ. ID. No. 2)
(ii) der Linker GGGGSGGGGSGGGGSGGGGSGGGGSGGGGSGGGGS (SEQ. ID. No. 3)
(iii) die DBD SLETWLKHREKDGGS (SEQ. ID. No. 4), die die dsDNA-Sequenz bindet, die durch die 5'-zu-3'-Sequenz 5'GAGGTC3' repräsentiert wird,
(iv) den Linker GGGGSGGGGSGGGGGS (SEQ. ID. No. 5) und
(v) eine alternative Effektorkomponente außer dem genannten künstlichen Nickase-Modul, z. B. einen Transkriptionsaktivator wie etwa VP64
oder die gleiche modulare Polypeptidsequenz, in der einer oder beide der genannten Linker durch einen alternativen Linker substituiert sind.

15. Eine Nukleinsäure oder Kombination von Nukleinsäuren zur Bereitstellung eines oder mehrerer Nukleoproteinkomplexe nach einem der vorhergehenden Ansprüche in einer Wirtszelle; wobei die Nukleinsäure optional ein Vektor ist, der in der Lage ist, sowohl die Polypeptidkomponente eines Nukleoproteinkomplexes nach einem der Ansprüche 10 bis 12 zu exprimieren, wobei eine künstliche Nickase und mindestens eine targetierende Nukleinsäure, z. B. targetierende RNA, bereitgestellt werden.

16. Verfahren zur Modifikation einer oder mehrerer Ziel-Nukleinsäuresequenzen, die einen oder mehrere Nukleoproteinkomplexe nach einem der Ansprüche 1 bis 14 einsetzen, oder einer oder mehrerer Nukleinsäuren zur Bereitstellung davon in einer Wirtszelle, mit der Maßgabe, dass das Verfahren kein Verfahren zur Modifikation der Keimbahnidentität eines menschlichen Wesens oder Verfahren zur Behandlung, das an dem menschlichen oder tierischen Körper ausgeführt wird, ist.

17. Eine Kombination von (i) mindestens einer modularen Polypeptidkomponente eines Nukleoproteinkomplexes nach einem der Ansprüche 1 bis 14 oder eines für diese kodierenden Polynukleotids, und (ii) einer oder mehreren targetierenden Nukleinsäuren wie in Anspruch 1 definiert, die mit der (den) genannten Polypeptidkomponente(n) verknüpft werden können, oder ein oder mehrere für diese kodierende Polynukleotide, zur Verwendung in einem Verfahren zur therapeutischen Behandlung.

## Revendications

1. Complexe nucléoprotéique convenant à une utilisation dans la modification d'une séquence d'acide nucléique cible comprenant (A) un acide nucléique de ciblage et (B) un composant polypeptidique modulaire, dans lequel ledit acide nucléique de ciblage comprend :
(i) un élément d'acide nucléique de ciblage (TE) complémentaire à une région de la cible ;
(ii) un élément de reconnaissance qui interagit spécifiquement avec un module de reconnaissance d'acide nucléique dudit composant polypeptidique modulaire et
(iii) une séquence de connexion qui lie ledit élément de ciblage et ledit élément de reconnaissance
et ledit composant polypeptidique modulaire comprend, sous forme de modules fonctionnels distincts qui sont liés :
(a) ledit module de reconnaissance d'acide nucléique qui est dépourvu d'activité enzymatique ;
(b) un domaine de liaison à l'ADN (DBD) pour aider à la fusion et/ou au déroulement d'une double hélice de la cible, le DBD reconnaissant une séquence prédéterminée dans la cible, le module (a) et ledit DBD n'étant pas liés à l'état naturel,
(c) un composant effecteur destiné à une utilisation dans la modification de la cible, une modification spécifique du site dirigée par lesdits TE et DBD ayant ainsi lieu,
dans lequel ledit DBD est une séquence polypeptidique ne comportant pas plus de 70 à 75 résidus acides aminés ;
dans lequel la longueur du composant polypeptidique modulaire n'est pas supérieure à 300 acides aminés ;
et dans lequel le module (b) est joint au module (a) et au module (c) respectivement ; optionnellement
dans lequel les modules (a), (b) et (c) sont joints par un premier et/ou un deuxième lieur.

2. Complexe nucléoprotéique selon la revendication 1, dans lequel ledit acide nucléique de ciblage est un ARN.

3. Complexe nucléoprotéique selon la revendication 1 ou la revendication 2, dans lequel la longueur du composant peptidique modulaire n'est pas supérieure à 220 à 250 acides aminés.

4. Complexe nucléoprotéique selon l'une quelconque des revendications 1 à 3, dans lequel ledit élément de reconnaissance de l'acide nucléique de ciblage est un échafaudage d'ARN (RS) qui se lie à un domaine de liaison d'échafaudage d'ARN (RSBD) en produisant ledit module (a) du composant polypeptidique modulaire, par exemple ledit RS est le motif d'ARN 19nt Box B et ledit RSBD est le peptide Lambda N22 tel qu'énoncé dans la SÉQ. ID n° 2.

5. Complexe nucléoprotéique selon la revendication 4, comprenant comme ledit composant (A) un composant entièrement acide nucléique (NAC) et (B) un composant polypeptidique modulaire, dans lequel le NAC comprend :
(i) un élément de ciblage (TE) complémentaire à une région de la cible ;
(ii) un échafaudage d'ARN (RS) qui se lie spécifiquement à un domaine de liaison d'échafaudage d'ARN (RSBD) dudit composant polypeptidique modulaire et
(iii) une séquence de connexion qui lie ledit TE et le RS
et ledit composant polypeptidique modulaire comprend, sous forme de modules fonctionnels distincts qui sont liés :
(a) ledit RSBD ;
(b) ledit domaine de liaison à l'ADN lié à (a) qui reconnaît une séquence prédéterminée dans la cible, le module (a) et ledit DBD n'étant pas liés à l'état naturel ;
(c) un composant effecteur lié à (b) destiné à une utilisation dans la modification de la cible, une modification spécifique du site dirigée par ledit TE et ledit DBD ayant ainsi lieu, et
(d) optionnellement, ou si nécessaire, un premier lieur et/ou un deuxième lieur liant ledit DBD respectivement audit composant effecteur et au RSBD.

6. Complexe nucléoprotéique selon la revendication 4 ou la revendication 5, dans lequel ledit acide nucléique de ciblage ou ledit NAC produit deux échafaudages d'ARN ou plus, soit identiques soit différents.

7. Complexe nucléoprotéique selon l'une quelconque des revendications précédentes, dans lequel ledit DBD n'est pas plus d'un 30-mer, pas plus d'un 25-mer, pas plus d'un 20-mer, de préférence pas plus d'un 15-mer ; optionnellement dans lequel ledit domaine de liaison à l'ADN se lie à une séquence prédéterminée de 3 à 6 nucléotides ; optionnellement dans lequel ledit DBD est un peptide ne contenant pas plus de 15 acides aminés, par exemple le 15-mer ayant la séquence SLETWLKHREKDGGS (SÉQ. ID n° 4) qui se lie à la séquence d'ADNdb représentée par la séquence 5' à 3' 5'GAGGTC3'.

8. Complexe nucléoprotéique selon l'une quelconque des revendications précédentes, dans lequel ledit DBD est capable de se lier à plus d'une séquence prédéterminée.

9. Complexe nucléoprotéique selon l'une quelconque des revendications précédentes, dans lequel ledit composant polypeptidique modulaire produit en outre un signal de localisation nucléaire (NLS) et/ou un signal de localisation d'organite, dans lequel ledit signal de localisation NLS et/ou d'organite est produit à l'extrémité N- ou C-terminale optionnellement connectée à une séquence supplémentaire pour aider à la détection.

10. Complexe nucléoprotéique selon l'une quelconque des revendications précédentes, dans lequel ledit composant effecteur est sélectionné parmi (i) une endonucléase pour produire des cassures double brin ou un domaine de nucléase Fok 1, (ii) une nickase, ((iii) un activateur de transcription, (iv) un répresseur transcriptionnel, (v) une enzyme modulatrice épigénétique, (vii) une recombinase, (viii) une transposase, (ix) une intégrase, et (x) une construction enzymatique de modification de nucléobase.

11. Complexe nucléoprotéique selon la revendication 10, dans lequel ledit composant effecteur est une nucléase ou une nickase artificielle comprenant un multimère de peptides liés et à auto-assemblage formant une structure en feuillet bêta, dans lequel :
(i) les peptides à auto-assemblage dudit multimère présentent chacun un motif alterné de résidus hydrophobes et hydrophiles, la totalité ou au moins une proportion des résidus hydrophiles de chacun de ces peptides représentant une triade catalytique d'acides aminés, ledit multimère étant capable de cliver un seul brin ou les deux brins d'un ADNdb ;
(ii) les peptides à auto-assemblage sont joints par des lieurs pour limiter le nombre de multimères, par exemple à 4-15, de préférence à 5-10 et
(iii) des séquences flanquantes N-terminale et C-terminale sont présentes, liées au multimère pour empêcher ou limiter l'agrégation entre multimères de la protéine de nucléase avec une charge positive accrue par rapport aux unités peptidiques du multimère en raison de l'inclusion d'un ou plusieurs résidus à charge positive, par exemple par inclusion d'un ou plusieurs résidus lysine ou arginine.

12. Complexe nucléoprotéique selon la revendication 11, dans lequel :
(i) le composant polypeptidique modulaire ne comprend pas plus de 220 à 300 acides aminés, à l'exclusion de toute étiquette clivable ou de tout signal de localisation ou de toute autre séquence autre que les composants (a) à (d) ;
(ii) lesdits peptides formant ledit multimère sont des 7-mers identiques ou comprennent deux 7-mers non identiques ou plus, les résidus hydrophobes alternés étant la leucine et/ou l'isoleucine, en commençant par le résidu N-terminal ;
(iii) lesdits peptides formant ledit multimère sont des peptides identiques, tous liés en continu dans la direction N-terminale à C-terminale de manière à produire une structure en feuillet bêta anti-parallèle ;
(iv) les lieurs qui joignent les peptides dudit multimère sont des lieurs acides aminés capables de produire un tour bêta ou des boucles semblables à des tours bêta, par exemple constitués d'une combinaison d'acides aminés sélectionnés parmi la glycine (G), la sérine (S), l'asparagine (N), la glutamine (Q), la thréonine (T), l'acide glutamique (E) et l'acide aspartique (D) ; et/ou
(v) l'extrémité C-terminale dudit peptide flanquant C-terminal produit une séquence de queue flexible à son extrémité C-terminale pour favoriser la solubilité.

13. Complexe nucléoprotéique selon la revendication 11 ou la revendication 12, dans lequel lesdits peptides dudit multimère sont des 7-mers ayant la séquence IEIDIHI ;
optionnellement dans lequel une nickase artificielle est produite comprenant la séquence 
IEKDIHINDGGIEIDIHIDSGGIEIDIHISSGSIEIDIHINNGNIEIDIHIGSDGIEID
IHIGNSGIEIDIHIDNGGIEIDIHIDSDGIEIDIHISSSGIEIDIHIGSEGIEIDIHIG
DSGIEIDKHINQGS (SÉQ. ID n° 6)
ou une variante de celle-ci comportant un ou plusieurs tours bêta variants et/ou une variation de la séquence de queue NQGS ;
optionnellement dans lequel le composant polypeptidique modulaire comprend la séquence SÉQ. ID n° 1 constituée des composants suivants fusionnés dans la direction N-terminale à C-terminale :
(i) la protéine Lambda N22, MDAQTRRRERRAEKQAQWKAAN (SÉQ. ID n° 2)
(ii) le lieur GGGGSGGGGSGGGGSGGGGSGGGGSGGGGSGGGGS (SÉQ. ID n° 3)
(iii)le DBD SLETWLKHREKDGGS (SÉQ. ID n° 4) qui se lie à la séquence d'ADNdb représentée par la séquence 5' à 3' 5'GAGGTC3'
(iv) le lieur GGGGSGGGGSGGGGGS (SÉQ. ID n° 5) et
(v) le module de nickase : IEKDIHINDGGIEIDIHIDSGGIEIDIHISSGSIEIDIHINNGNIEIDIHIGSDGIEID 
IHIGNSGIEIDIHIDNGGIEIDIHIDSDGIEIDIHISSSGIEIDIHIGSEGIEIDIHIG
DSGI EIDKHINQGS (SÉQ. ID n° 6)
ou la même séquence polypeptidique modulaire dans laquelle un desdits lieurs ou les deux sont substitués par un lieur alternatif.

14. Complexe nucléoprotéique selon la revendication 11 ou la revendication 12, dans lequel le composant polypeptidique modulaire comprend les composants suivants fusionnés dans la direction N-terminale à C-terminale :
(i) la protéine Lambda N22, MDAQTRRRERRAEKQAQWKAAN (SÉQ. ID n° 2)
(ii) le lieur GGGGSGGGGSGGGGSGGGGSGGGGSGGGGSGGGGS (SÉQ. ID n° 3)
(iii) le DBD SLETWLKHREKDGGS (SÉQ. ID n° 4) qui se lie à la séquence d'ADNdb représentée par la séquence 5' à 3' 5'GAGGTC3'
(iv) le lieur GGGGSGGGGSGGGGGS (SÉQ. ID n° 5) et
(v) un composant effecteur alternatif autre que ledit module de nickase artificielle, par exemple un activateur de transcription tel que VP64
ou la même séquence polypeptidique modulaire dans laquelle un desdits lieurs ou les deux sont substitués par un lieur alternatif.

15. Acide nucléique ou combinaison d'acides nucléiques destiné(e) à l'apport d'un ou plusieurs complexes nucléoprotéiques selon l'une quelconque des revendications précédentes dans une cellule hôte ;
l'acide nucléique étant optionnellement un vecteur capable d'exprimer à la fois le composant polypeptidique d'un complexe nucléoprotéique selon l'une quelconque des revendications 10 à 12 produisant une nickase artificielle et au moins un acide nucléique de ciblage, par exemple un ARN de ciblage.

16. Procédé de modification d'une ou plusieurs séquences d'acide nucléique cibles à l'aide d'un ou plusieurs complexes nucléoprotéiques selon l'une quelconque des revendications 1 à 14 ou d'un ou plusieurs acides nucléiques pour leur apport dans une cellule hôte, à condition que le procédé ne soit pas un procédé de modification de l'identité de lignée germinale d'un être humain ou une méthode de traitement pratiquée sur le corps humain ou animal.

17. Combinaison de (i) au moins un composant polypeptidique modulaire d'un complexe nucléoprotéique selon l'une quelconque des revendications 1 à 14, ou un polynucléotide le(s) codant et (ii) un ou plusieurs acides nucléiques de ciblage tels que définis dans la revendication 1 qui peuvent se lier audit/auxdits composant(s) polypeptidique(s), ou un ou plusieurs polynucléotides le(s) codant, destinée à une utilisation dans une méthode de traitement thérapeutique.
